(19) Europäisches Patentamt
European Patent Office
Office européen des brevets

(11) Publication number: **0 017 970**
**B1**

(12) **EUROPEAN PATENT SPECIFICATION**

(45) Date of publication of patent specification: **07.07.82**

(21) Application number: **80102034.8**

(22) Date of filing: **16.04.80**

(51) Int. Cl.³: **C 07 D 487/04** //A61K31/40, C12P17/18, C12R1/465, (C07D487/04, 209/00, 205/00).

# (54) Novel beta-lactam derivatives and process for production thereof.

(30) Priority: **17.04.79 JP 45990/79**

(43) Date of publication of application: **29.10.80 Bulletin 80/22**

(45) Publication of the grant of the patent: **07.07.82 Bulletin 82/27**

(84) Designated Contracting States: **DE FR GB IT**

(56) References cited: **EP - A - 0 007 973**

(73) Proprietor: **SANRAKU-OCEAN CO., LTD.**
**15-1, Kyobashi 1-chome Chuo-ku**
**Tokyo (JP)**

(72) Inventor: **Yoshioka, Takeo**
**3-6-36 Numama**
**Zushi (JP)**
Inventor: **Yamamoto, Kenichi**
**Shonan Laifutaun G-2-2, 1120 Endo**
**Fujisawa (JP)**
Inventor: **Yamada, Kaoru**
**2-26-405, Kitagawara-cho**
**Kameoka (JP)**
Inventor: **Kato, Yasuyuki**
**Takeso 6, 1970 Arima Takatsu-ku**
**Kawasaki (JP)**
Inventor: **Shimauchi, Yasutaka**
**1-101-13 Yurigaoka Ninomiya-machi**
**Naka (JP)**
Inventor: **Ishikura, Tomoyuki**
**640 Kowada**
**Chigasaki (JP)**

(74) Representative: **Kraus, Walter, Dr. et al,**
**Patentanwälte Dres. Kraus & Weisert**
**Irmgardstrasse 15**
**D-8000 München 71 (DE)**

**EP 0 017 970 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Novel beta-lactam derivatives and process for production thereof

This invention relates to novel compounds of $\beta$-lactam type. More specifically, it relates to compounds of the formula

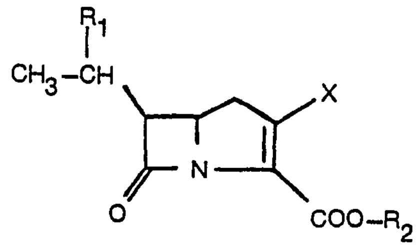

(I)

wherein $R_1$ represents a hydrogen atom, a hydroxyl group or a methyl group, $R_2$ represents a $C_1$—$C_6$ alkyl group or an aralkyl group having 7 to 20 carbon atoms which may be substituted by a halogen atom, a $C_1$—$C_6$ alkyl group, a $C_1$—$C_6$ alkoxy group, a halo $C_1$—$C_6$ alkyl group, a nitro group or a $C_1$—$C_6$ alkylsulfonyl group, and X represents a bromine or iodine atom with the proviso that *o*- and *p*-nitrobenzyl 3-bromo-6-(1-hydroxyethyl)-7-oxo-1-azabicyclo[3.2.0]hept-2-ene-2-carboxylate are excluded.

This invention relates also to a process for producing compounds of the general formula (I')

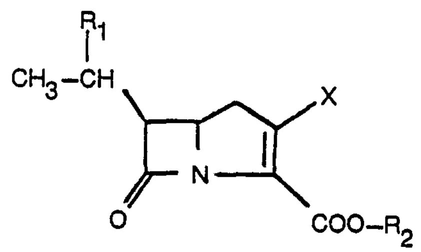

(I')

wherein $R_1$ represents a hydrogen atom, a hydroxyl group or a methyl group, $R_2$ represents a $C_1$—$C_6$ alkyl group or an aralkyl group having 7 to 20 carbon atoms which may be substituted by a halogen atom, a $C_1$—$C_6$ alkyl group, a $C_1$—$C_6$ alkoxy group, a halo $C_1$—$C_6$ alkyl group, a nitro group or a $C_1$—$C_6$ alkylsulfonyl group, and X represents a bromine or iodine atom, which comprises reacting a compound of the formula II

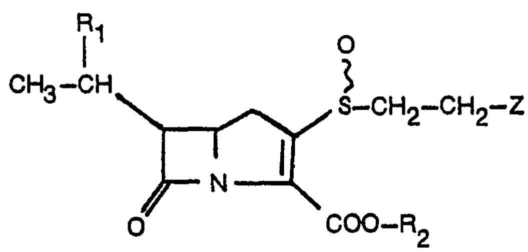

(II)

wherein $R_1$ and $R_2$ are as defined above, and Z represents an amino group or a blocked amino group, with a brominating or iodinating agent selected from N-bromimides, N-bromamides, N-iodimides, N-iodamides and alkali metal iodides.

EP - A - 0 007 973 contains an implicit disclosure of the compounds of formula I as mentioned above in which $R_1$ represents OH, $R_2$ represents *o*- or *p*-nitrobenzyl and X represents Br (formula 19 and the description on page 11, lines 2 to 4 and page 12, lines 23 to 26 of the cited document). Accordingly, *o*- and *p*- nitrobenzyl 3-bromo-6-(1-hydroxyethyl)-7-oxo-1-azabicyclo [3.2.0] hept-2-ene-2-carboxylates are known from this citation and, therefore, are not covered by this invention.

Otherwise the compounds of formula (I) are novel compounds not described in the prior literature. By utilizing the fact that these compounds contain an active bromine or iodine atom at the 3-position, they can be converted to useful compounds having excellent pharmacological activity in which the 3-position is substituted by other groups.

The compounds of formula (I) exhibit superior antimicrobial activities in *in vitro* tests in the presence of horse serum.

In the present specification and the appended claims, the term "$C_1$—$C_6$" used to qualify groups or compounds means that the groups or compounds so qualified have not more than 6, preferably not more than 4, carbon atoms.

The "$C_1$—$C_6$ alkyl group" in formulas (I) and (I') linear or branched, and includes, for example, methyl, ethyl, *n*- or *iso*-propyl, *n*-, *iso*-, *sec*- or *tert*-butyl, and *n*-pentyl.

The "substituted or unsubstituted aralkyl group containing 7 to 20 carbon atoms" in formula (I) and (I') is preferably a $C_1$—$C_6$ alkyl group (especially methyl or ethyl) substituted by 1 to 3 substituted or unsubstituted phenyl groups. Examples of the substituent on the benzene ring in the substituted phenyl group are halogen atoms such as chlorine and bromine, $C_1$—$C_6$ alkyl groups such as methyl, $C_1$—$C_6$ alkoxy groups such as methoxy, $C_1$—$C_6$ haloalkyl groups such as trifluoromethyl, nitro groups, and $C_1$—$C_6$ alkyl sulfonyl groups such as methylsulfonyl. Preferably, the benzene ring is mono-, di or tri-, especially mono-substituted with such a substituent. Thus, specific examples of the aralkyl group for $R_2$ are benzyl, phenethyl, benzhydryl, trityl, *p*-nitrobenzyl, *o*-nitrobenzyl, *p*-chlorobenzyl, *o*-chlorobenzyl, *p*-fluorobenzyl, *p*-bromobenzyl, *p*-methoxybenzyl, *p*-methylsulfonylbenzyl, and *p*-trifluoromethylbenzyl. Of these, benzyl and mono-substituted benzyl groups, particularly the *p*-nitrobenzyl group, are advantageous because they can be easily split off by hydrogenolysis.

A preferred group of compounds in this invention is a group of compounds of formulas (I) and (I') in which $R_1$ represents a hydrogen atom or a methyl group. Another preferred group is a group of compounds of formulas (I) and (I') in which $R_2$ represents an alkyl group having 1 to 4 carbon atoms, or a methyl or ethyl group substituted by 1 to 3 phenyl groups which may be substituted by 1 to 3 substituents selected from halogen atoms, $C_1$—$C_6$ alkyl groups, $C_1$—$C_6$ alkoxy groups, $C_1$—$C_6$ haloalkyl groups, nitro groups and $C_1$—$C_6$ alkyl sulfonyl groups. In the latter group, more preferred are compounds of formulas (I) and (I') in which $R_2$ represents an alkyl group having 1 to 4 carbon atoms, a benzyl group, a mono-substituted benzyl group, or a benzhydryl group.

Typical examples of the compounds of formulas (I) and (I') are listed below.

Methyl 3-bromo-6-ethyl-7-oxo-1-azabicyclo[3.2.0]hept-2-ene-2-carboxylate,
benzyl 3-bromo-6-ethyl-7-oxo-1-azabicyclo[3.2.0]hept-2-ene-2-carboxylate,
*p*-nitrobenzyl 3-bromo-6-ethyl-7-oxo-1-azabicyclo[3.2.0]hept-2-ene-2-carboxylate,
*o*-nitrobenzyl 3-iodo-6-ethyl-7-oxo-1-azabicyclo[3.2.0]hept-2-ene-2-carboxylate,
benzhydryl 3-bromo-6-ethyl-7-oxo-1-azabicyclo[3.2.0]hept-2-ene-2-carboxylate,
trityl 3-bromo-6-ethyl-7-oxo-1-azabicyclo[3.2.0]hept-2-ene-2-carboxylate.
phenethyl 3-iodo-6-ethyl-7-oxo-1-azabicyclo[3.2.0]hept-2-ene-2-carboxylate,
*p*-methylsulfonylbenzyl 3-bromo-6-ethyl-7-oxo-1-azabicyclo[3.2.0]hept-2-ene-2-carboxylate,
methyl 3-iodo-6-ethyl-7-oxo-1-azabicyclo[3.2.0]hept-2-ene-2-carboxylate,
methyl 3-bromo-6-(1-hydroxyethyl)-7-oxo-1-azabicyclo[3.2.0]hept-2-ene-2-carboxylate,
*n*-propyl 3-iodo-6-(1-hydroxyethyl)-7-oxo-1-azabicyclo[3.2.0]hept-2-ene-2-carboxylate,
benzyl 3-bromo-6-(1-hydroxyethyl)-7-oxo-1-azabicyclo[3.2.0]hept-2-ene-2-carboxylate,
benzyl 3-iodo-6-ethyl-7-oxo-1-azabicyclo[3.2.0]hept-2-ene-2-carboxylate,
*p*-nitrobenzyl 3-iodo-6-(1-hydroxyethyl)-7-oxo-1-azabicyclo[3.2.0]hept-2-ene-carboxylate,
trityl 3-bromo-6-(1-hydroxyethyl)-7-oxo-1-azabicyclo[3.2.0]hept-2-ene-2-carboxylate,
methyl 3-bromo-6-isopropyl-7-oxo-1-azabicyclo[3.2.0]hept-2-ene-2-carboxylate,
*tert.*-butyl 3-iodo-6-isopropyl-7-oxo-1-azabicyclo[3.2.0]hept-2-ene-2-carboxylate,
benzyl 3-bromo-6-isopropyl-7-oxo-1-azabicyclo[3.2.0]hept-2-ene-2-carboxylate,
*p*-nitrobenzyl 3-bromo-6-isopropyl-7-oxo-1-aza-bicyclo[3.2.0]hept-2-ene-2-carboxylate, and
benzhydryl 3-iodo-6-isopropyl-7-oxo-1-azabicyclo[3.2.0]hept-2-ene-2-carboxylate.

In the preparation of the compounds of formula I', the reaction of the compounds of formula (II) with the halogenating agents can be carried out generally in an organic solvent inert to the reaction, for example halogenated hydrocarbons such as methylene chloride, chloroform, carbon tetrachloride or chlorobenzene, under relatively mild conditions, i.e. at a temperature at which the solvent used is maintained liquid, preferably at 0 to 70°C.

By this reaction, the N-substituted or unsubstituted aminoethylsulfinyl group at the 3-position of the starting compound of formula (II) is split off, and instead, a bromine or iodine atom is introduced thereinto.

Suitable N-bromimides and N-iodimides used in the aforesaid halogenation reaction are expressed by the following formula

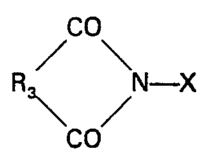

(III-a)

wherein $R_3$ represents a lower alkylene group, a lower alkenylene group or a phenylene group, and X represents a bromine or iodine atom.

Specific examples include N-bromosuccinimide, N-iodosuccinimide, N-bromomaleimide, N-iodomaleimide, N-bromophthalimide, and N-iodophthalimide. Of these, N-bromosuccinimide and N-iodosuccinimide are preferred.

Suitable N-bromamides and N-iodamides that can be equally used as the halogenating agent are represented by the following formula

$$R'_3\text{—CONHX} \qquad \text{(III-b)}$$

wherein $R'_3$ represents a lower alkyl group or a lower alkenyl group, and X represents a bromine or iodine atom.

Specific examples include N-bromoacetamide, N-iodoacetamide, N-bromopropionamide, N-bromoacrylamide, and N-bromo-n-butyramide. Of these, N-bromoacetamide is preferred.

Suitable alkali metal iodides are sodium iodide and potassium iodide.

The amount of such a halogenating agent is not particularly limited, and can be varied over a wide range depending upon the type of the halogenating agent, and/or the type of the starting compound of formula (II). Advantageously, it is generally used in an amount of at least 1 mole, preferably 1.2 to 2 moles, per mole of the compound of formula (II).

The halogenation reaction proceeds rapidly, and can usually be completed in 0.5 to 2 hours under the aforesaid conditions.

After the reaction, the resulting compound of formula (I') can be isolated and purified by various methods known *per se.* For example, the resulting reaction mixture is diluted with a water-immiscible solvent, for example a halogenated hydrocarbon such as methylene chloride, an aromatic hydrocarbon such as benzene or toluene, or an ester such as ethyl acetate, and washed with a phosphate buffer having a pH of 6.8. The solvent layer is dried, and distilled under reduced pressure. The residue is then subjected to gel filtration on a column of Biobeads S-series (a product of Bio-Rad Company), and if desired, further to column chromatography on a silica gel column. Alternatively, the resulting reaction mixture is directly subjected to chromatography on a silica gel column. Thus, the compound of formula (I') can be isolated.

The compounds of formula (II) used as the starting material in the aforesaid halogenation reaction are novel compounds. These compounds can be produced by methods similar to methods for producing known compounds corresponding to formula (II) in which $R_1$ is a trimethylsilyloxy group, $R_2$ is a trimethylsilyl group and Z is a p-nitrobenzyloxycarbonylamino group, and those in which $R_1$ is a hydroxyl group, $R_2$ is a *p*-nitrobenzyl group and Z is a *p*-nitrobenzyloxycarbonylamino group (see Japanese Laid-Open Patent Publication No. 111591/77 and U.S. Patent No. 4,123,547).

For example, the compounds of formula (II) can be easily prepared by the S-oxidation of compounds of the formula

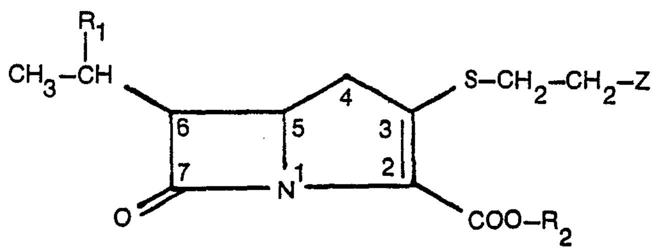

(IV)

wherein $R_1$, $R_2$ and Z are as defined hereinabove.

The "blocked amino group" in formulae (II) and (IV) above includes, for example, mono- or dialkyl or aralkyl amino groups such as methylamino, ethylamino, butylamino, benzylamino, tritylamino, dimethylamino, diethylamino, dibutylamino and dibenzylamino; mono- or di-(trialkyl)silylamino groups such as trimethylsilylamino and ditrimethylsilylamino; acylamide groups such as formamide, acetamide, propionamide, butyrylamide, phenylacetamide, chloroacetamide, trifluoroacetamide, *o*-nitrophenoxyacetamide, methoxyacetamide, mesylamide, and tosylamide; and alkoxycarbonylamino groups such as methoxycarbonylamino, ethoxycarbonylamino, butoxycarbonylamino, benzyloxycarbonylamino, nitrobenzyloxycarbonylamino and methoxybenzylcarbonylamino.

The S-oxidation of the compounds of formula (IV) can be carried out by methods known *per se,* for example methods frequently used in the S-oxidation of sulfur-containing $\beta$-lactam antibiotics of the penicillin type or the cephalosporin type. For example, the compound of formula (IV) is reacted with a mild oxidizing agent which does not substantially act on the $\beta$-lactam skeleton, for example perbenzoic acid, phenyldichloroiodide, or sodium metaperiodate. Suitable mild oxidizing agents are organic peracids, especially perbenzoic acid and m-chloro-perbenzoic acid. Substituted perbenzoic acids, such as m-chloro-perbenzoic acid, are preferred.

Advantageously, the reaction of the compound of formula (IV) with the oxidizing agent is carried out usually in an inert solvent such as methylene chloride, chloroform or carbon tetrachloride under mild temperature conditions, for example at room temperature or below, perferably at −30°C to 20°C. The reaction can be completed usually in 3 minutes to 3 hours under these conditions.

The amount of the oxidizing agent used in the S-oxidation of the compound of formula (IV) can be varied widely according to the type of the oxidizing agent and the reaction conditions. Generally, the suitable amount of the oxidizing agent is 0.3 to 1.8 molar equivalents, preferably 0.7 to 1.3 molar equivalents, per mole of the compound of formula (IV).

The resulting compound of formula (II) can be isolated and purified by methods known *per se.* Or it can be submitted to the aforesaid halogenation reaction immediately after the excess unreacted oxidizing agent remaining in the reaction mixture is decomposed or removed.

Most of the compounds of formula (IV) are known. Compounds of formula (IV) in which $R_1$ is a hydrogen atom and Z is an acetylamino group (antibiotic PS-5 and its esters) and a method for the production thereof are disclosed, for example, in J. Antibiotics, *31,* 480—482 (1978), and German Offenlegungsschrift (DE—OS) No. 2813922. Compounds of formula (IV) in which $R_1$ is a hydroxyl group, and a method for production thereof are disclosed, for example, in DE—OS No. 2652680 and DE—OS No. 2652674. Furthermore, compounds of formula (IV) in which $R_1$ is a methyl group and Z is an acetylamino group and a method for production thereof are disclosed, for example, in European Patent Application Published Specification No. 1567.

The compounds of formula (IV) in which $R_1$ is a methyl group are antibiotics which the present inventors discovered previously and named "antibiotics PS-6". These substances are produced by using an antibiotic PS-6-producing microorganism separated, for example, from the soil. *Streptomyces* sp. A271 (ATCC 31358) is a typical example of such a microorganism. The microbiological properties of *Streptomyces* sp. A271, the fermentative method for producing antibiotic PS-6, the method for its purification, the method for its esterification, etc. are disclosed in the aforesaid European Patent Application, Published Specification No. 1567 which the present inventors previously filed.

The specification of the aforesaid European Patent is hereby cited in lieu of describing its contents in the present application, but specific examples are given hereinbelow.

The compounds of formula (I') have been found to have a unique property in that when a highly active bromine or iodine atom at their 3-position makes contact with a nucleophilic reagent such as a thiol compound, a group exchange reaction very easily takes place to produce derivatives in which the 3-position is replaced by another group.

The compounds of formula (I') are industrially very useful compounds as intermediates for conversion to valuable derivatives corresponding to formula (I') in which the 3-position is replaced by another group.

For example, the compounds of formula (I') can be converted to compounds of the formula

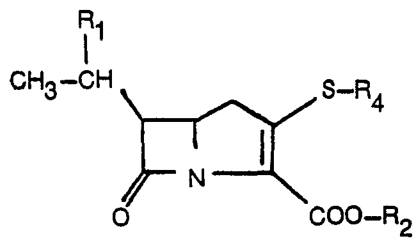

(VI-a)

wherein $R_1$ and $R_2$ are as defined hereinabove, and $R_4$ is a $C_1$—$C_6$ alkyl group, a hydroxy $C_1$—$C_6$ alkyl group, a $C_1$—$C_6$ alkoxy-$C_1$—$C_6$ alkyl group, a di($C_1$—$C_6$ alkyl)amino-$C_1$—$C_6$ alkyl group, a $C_1$—$C_6$ alkanoyloxy-$C_1$—$C_6$ alkyl group, a phenylacetyl amino-$C_1$—$C_6$ alkyl group, a cyclohexyl group, a phenyl group, *p*-nitrophenyl group, or a 5- or 6-membered aromatic heterocyclic group having 1 or 2 nitrogen atoms, by reacting them with thiol compounds of the formula

$$R_4—SH \qquad (V)$$

wherein $R_4$ is as defined above, or the reactive derivatives thereof. Saponification or hydrogenolysis of the resulting compounds gives compounds of the following formula

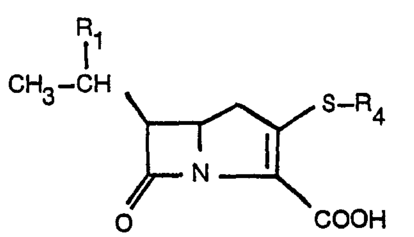

(VI-b)

wherein $R_1$ and $R_4$ are as defined above, or the salts thereof, which have antimicrobial activities.

Advantageously, the reaction of the compound of formula (I') with the thiol compound of formula (V) is carried out usually in a solvent such as N,N-dimethylformamide, tetrahydrofuran, dioxane, hexamethylphosphoric triamide or glyme, particularly in dimethylformamide, at low temperatures, for example −60°C to 30°C, preferably −50°C to −30°C. Under these conditions, the reaction can usually be completed in 20 minutes to 40 hours.

When the thiol compound of formula (V) is used, the reaction sometimes proceeds even in the absence of a base as a catalyst. Generally, it is desirable to perform the reaction in the presence of bases. Usable bases include, for example, amines such as triethylamine, alkali metal hydrides such as sodium hydride and potassium hydride, alkali metal amides such as sodium amide and potassium amide, alkali

metal hydroxides such as sodium hydroxide and potassium hydroxide, and alkali metal alkoxides such as sodium ethoxide, sodium methoxide and potassium butoxide. Advantageously, the bases are used in a proportion of at least 0.9 equivalent, preferably 1.0 to 1.2 equivalents, per mole of the thiol compound.

Instead of performing the aforesaid reaction in the presence of bases, a reaction derivative of the thiol compound of formula (V) may be used. Typical examples of such reactive derivatives include sodium ethanethiolate, sodium butanethiolate, potassium ethanethiolate, potassium butanethiolate, sodium cyclohexanethiolate, sodium phenylthiolate, sodium dimethylaminoethanethiolate, sodium hydroxyethanethiolate, sodium phenylacetamidoethanethiolate, sodium 4-pyridinethiolate, sodium 2-pyridinethiolate and sodium 2-pyrimidinethiolate.

The amount of the thiol compound of formula (V) or its reactive derivative is not critical. Generally, it is advantageously used in an amount of at least 1.0 mole, preferably 1.1 to 1.5 moles, per mole of the compound of formula (I').

Thus, the compound of formula (VI-a) is obtained. As required, this compound is purified, and then saponified or hydrogenolyzed to split off the group $R_2$. As a result, it can be converted to the compound of formula (VI-b) or its salt.

Hydrogenolysis of the compound of formula (VI-a) can be performed by methods known *per se.* For example, it can be performed by treating the compound (VI-a) with hydrogen in a solvent such as water or an ether (e.g., dioxane or tetrahydrofuran) in the presence of a catalyst such as platinum oxide, palladium-carbon or palladium black in combination with a buffer having a pH of at least 7.

The resulting compound of formula (VI-b) can be isolated and purified in accordance with various methods known *per se.* Usually, the compound can be isolated from the reaction mixture by methods frequently used in isolating and purifying carboxylic acid-type antibiotics. For example, solids are removed by filtration from the reaction mixture after the reaction. A buffer such as a phosphate buffer is added to the filtrate. While the pH of the filtrate is maintained so that it is not shifted to an acidic side, the filtrate is subjected to the following procedures either singly or in combination with each other, and if required, repeatedly. These procedures include, for example, a combination of adsorption on activated carbon. Amberlite XAD—2® (a product of Rohm & Haas Co.), Diaion HP—20® (a product of Mitsubishi Chemical Co., Ltd.), and elution with methanol/water, acetone/water, adsorption and elution by ion exchange resins such as Dowex 1X2® (a product of Dow Chemical Co.), Dowex 50X2® (a product of Dow Chemical Co.) and QAE-Sephadex A—25® (a product of Pharmacia Fine Chemicals, Co.); gel filtraction on Sephadex G—10® (a product of Pharmacia Fine Chemicals Co.), Biogel P—2® (a product of Bio-Rad Co.); column or thin-layer chromatography on cellulose, Avicel SF® (a product of American Viscose Corporation), DEAE-Cellulose Watman DE—32® (a product of Watman Co.), DEAE-Sephadex A—25® (a product of Pharmacia Fine Chemicals Co.), silica, alumina; forced precipitation by addition of a solvent such as acetone; and lyophilization.

According to the process of this invention, in the course of obtaining the final product from the starting compound *via* the reaction intermediate, the reaction can be allowed to proceed without affecting the stereochemistry of each of these compounds.

It has been found that when compounds of formula (IV) in which $R_1$ is a hydrogen atom or a methyl group are produced by the fermentative method described in the above-cited known references, they have a 5,6-trans configuration. Thus, compounds of formulae (I), (II), (VI-a) and (VI-b) in which $R_1$ represents a hydrogen atom or a methyl group obtained by the present invention from such starting materials can assume a 5,6-trans configuration. On the other hand, compounds of formula (IV) in which $R_1$ represents a hydroxy group can be obtained as epimers of either the 5,6-trans or 5,6-cis-configuration by the methods described in the above-cited references. They can also be obtained as isomers ascribable to the asymmetric carbon at the 8-position of these epimers. Accordingy, when $R_1$ represents a hydroxyl group, the compounds of formulae (I), (II), (VI-a) and (VI-b) may exist as epimers attributed to the assymmetric carbon at the 8-position of the compounds of the 5,6-trans or 5,6-cis configuration.

The compound of formula (VI-b) obtained by the method described hereinabove can be converted to a salt by a conventional method, for example by treating it with an inorganic base such as sodium bicarbonate, disodium phosphate, dipotassium phosphate, potassium 2-ethylhexanoate or ammonia, or an organic base such as monoethylamine, dimethylamine, triethylamine, monoethanolamine, diethanolamine, benzathine or procaine.

The compounds of formula (VI-b) of their salts have superior antibacterial activities.

Typical species of the compounds of formula (VI-b) and their salts have antibiotic activities as shown in Tables I—1 and I—8 below. Table I—9 shows the antibiotic activities of known compounds.

TABLE I–1

| | | Antibiotic Activity minimum inhibitory concentration (μg/ml) | | | |
|---|---|---|---|---|---|
| Compound of formula (VI-b) | $R_1$ | H | $-CH_3$ | –OH* | –OH** |
| | $R_4$ | 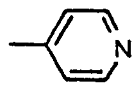 | 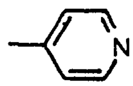 | 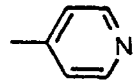 | 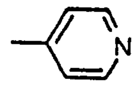 |
| Test Organism | | | | | |
| *Bacillus subtilis* ATCC 6633 | | <0.007 | 0.013 | 0.20 | <0.007 |
| *Sarcina lutea* | | <0.007 | 0.013 | 0.10 | <0.007 |
| *Staphylococcus aureus* FDA 209P | | <0.007 | 0.007 | 0.10 | <0.007 |
| *Staphylococcus aureus* Smith | | <0.007 | 0.013 | 0.20 | <0.007 |
| *Staphylococcus aureus* Russell | | <0.007 | 0.013 | 0.20 | <0.007 |
| *Staphylococcus epidermidis* | | <0.007 | 0.013 | 0.20 | <0.007 |
| *Alcaligenes faecalis* A 1 | | <0.007 | 0.013 | 0.025 | <0.007 |
| *Citrobacter freundii* GN 346 | | 29.5 | 50 | 100 | 6.25 |
| *Comamonas terrigena* B–996 | | <0.007 | 0.007 | 0.013 | <0.007 |
| *Enterobacter aerogenes* E19 | | 7.4 | 12.5 | 50 | 1.56 |
| *Enterobacter cloacae* 45 | | 29.5 | 50 | 50 | 6.25 |
| *Enterobacter* sp. E8 | | 0.46 | 1.56 | 0.78 | 0.025 |
| *Escherichia coli* K–12 | | 0.92 | 3.13 | 1.56 | 0.05 |
| *Escherichia coli* RGN 823 | | 14.8 | 12.5 | 25 | 6.25 |
| *Klebsiella pneumoniae* K–13 | | 14.8 | 25 | 25 | 25 |
| *Proteus mirabilis* P6 | | 1.8 | 3.13 | 6.25 | 0.39 |
| *Proteus rettgeri* P7 | | 1.8 | 1.56 | 6.25 | 0.78 |
| *Proteus vulgaris* GN76 | | 29.5 | 50 | 50 | 50 |
| *Proteus* sp. P22 | | 29.5 | 50 | 50 | 50 |

TABLE I–1 (Continued)

| | | Antibiotic Activity minimum inhibitory concentration ($\mu g/ml$) | | | |
|---|---|---|---|---|---|
| Compound of formula (VI-b) | $R_1$ | H | $-CH_3$ | –OH* | –OH** |
| | $R_4$ | 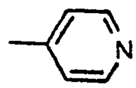 | 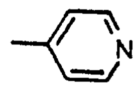 | 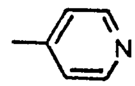 | 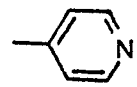 |
| Test Organism | | | | | |
| *Providencia* sp. P8 | | 0.92 | 3.13 | 1.56 | 0.20 |
| *Pseudomonas aeruginosa* IFO3445 | | 0.92 | 1.56 | 1.56 | 1.56 |
| *Pseudomonas aeruginosa* NCTC10490 | | 0.92 | 1.56 | 1.56 | 1.56 |
| *Serratia marcescens* S18 | | 29.5 | 50 | 100 | 50 |
| *Serratia marcescens* T55 | | 29.5 | 100 | 100 | 25 |

TABLE I–2

| | Antibiotic Activity minimum inhibitory concentration (μg/ml) | | | |
|---|---|---|---|---|
| Compound of formula (VI-b) $R_1$ | H | $-CH_3$ | –OH* | –OH** |
| $R_4$ | 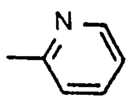 | 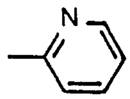 | 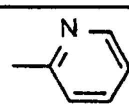 | 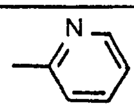 |
| Test Organism | | | | |
| *Bacillus subtilis* ATCC 6633 | 0.012 | 0.05 | 0.78 | <0.007 |
| *Sarcina lutea* | 0.0008 | 0.004 | 0.013 | <0.007 |
| *Staphylococcus aureus* FDA 209P | 0.006 | 0.007 | 0.05 | <0.007 |
| *Staphylococcus aureus* Smith | 0.012 | 0.013 | 0.39 | 0.013 |
| *Staphylococcus aureus* Russell | 0.012 | 0.013 | 0.39 | 0.013 |
| *Staphylococcus epidermidis* | 0.012 | 0.05 | 1.56 | 0.05 |
| *Alcaligenes faecalis* A 1 | 0.1 | 0.20 | 0.78 | 6.25 |
| *Citrobacter freundii* GN346 | 25 | 50 | >100 | 6.25 |
| *Comamonas terrigena* B–996 | 0.003 | 0.007 | 0.20 | <0.007 |
| *Enterobacter aerogenes* E19 | 25 | 50 | 100 | 6.25 |
| *Enterobacter cloacae* 45 | 50 | >100 | 100 | 12.5 |
| *Enterobacter* sp. E8 | 12.5 | 25 | 25 | 0.78 |
| *Escherichia coli* K–12 | 6.25 | 12.5 | 6.25 | 0.39 |
| *Escherichia coli* RGN 823 | 6.25 | 12.5 | 12.5 | 3.13 |
| *Klebsiella pneumoniae* K–13 | 25 | 50 | 50 | 25 |
| *Proteus mirabilis* P6 | 3.13 | 6.25 | 12.5 | 0.39 |
| *Proteus rettgeri* P7 | 6.25 | 6.25 | 25 | 3.13 |
| *Proteus vulgaris* GN76 | 12.5 | 12.5 | 25 | 12.5 |
| *Proteus* sp. P22 | 6.25 | 12.5 | 12.5 | 12.5 |

TABLE I–2 (Continued)

| | | Antibiotic Activity minimum inhibitory concentration ($\mu g/ml$) | | | |
|---|---|---|---|---|---|
| Compound of formula (VI-b) | $R_1$ | H | $-CH_3$ | –OH* | –OH** |
| | $R_4$ | 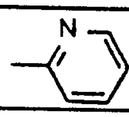 | 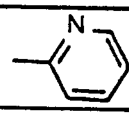 | 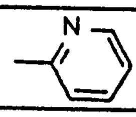 | 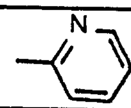 |
| Test organism | | | | | |
| *Providencia* sp. P8 | | 1.56 | 3.13 | 3.13 | 0.20 |
| *Pseudomonas aeruginosa* IFO3445 | | 12.5 | 50 | 12.5 | 12.5 |
| *Pseudomonas aeruginosa* NCTC10490 | | 25 | 50 | 25 | 50 |
| *Serratia marcescens* S18 | | 12.5 | 50 | 50 | 12.5 |
| *Serratia marcescens* T55 | | 25 | 50 | 100 | 25 |

TABLE I–3

| | | Antibiotic Activity minimum inhibitory concentration (μg/ml) | | | |
|---|---|---|---|---|---|
| Compound of formula (VI-b) | $R_1$ | H | $-CH_3$ | –OH* | –OH** |
| | $R_4$ | 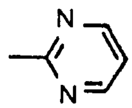 | 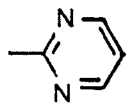 | 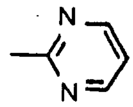 | 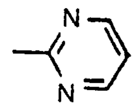 |
| Test Organism | | | | | |
| *Bacillus subtilis* ATCC 6633 | | <0.012 | 0.013 | 0.39 | <0.007 |
| *Sarcina lutea* | | <0.012 | 0.013 | 0.20 | <0.007 |
| *Staphylococcus aureus* FDA209P | | <0.012 | 0.007 | 0.20 | <0.007 |
| *Staphylococcus aureus* Smith | | 0.024 | 0.025 | 0.39 | <0.025 |
| *Staphylococcus aureus* Russell | | <0.012 | 0.013 | 0.39 | 0.013 |
| *Staphylococcus epidermidis* | | 0.024 | 0.05 | 0.39 | 0.025 |
| *Alcaligenes faecalis* A 1 | | 0.2 | 0.39 | 1.56 | 0.05 |
| *Citrobacter freundii* GN346 | | 25 | 50 | 100 | 6.25 |
| *Comamonas terrigena* B–996 | | ≤0.012 | 0.013 | 0.05 | <0.007 |
| *Enterobacter aerogenes* E19 | | 12.5 | >100 | 100 | 3.13 |
| *Enterobacter cloacae* 45 | | 25 | >100 | 50 | 12.5 |
| *Enterobacter* sp. E8 | | 3.13 | 12.5 | 6.25 | 0.013 |
| *Escherichia coli* K–12 | | 1.56 | 6.25 | 1.56 | 0.1 |
| *Escherichia coli* RGN 823 | | 3.13 | 3.13 | 3.13 | 1.56 |
| *Klebsiella pneumoniae* K–13 | | 12.5 | 25 | 25 | 25 |
| *Proteus mirabilis* P6 | | 3.13 | 3.13 | 12.5 | 0.78 |
| *Proteus rettgeri* P7 | | 3.13 | 3.13 | 12.5 | 1.56 |
| *Proteus vulgaris* GN76 | | 6.25 | 12.5 | 12.5 | 6.25 |
| *Proteus* sp. P22 | | 12.5 | 12.5 | 12.5 | 25 |

TABLE I–3 (Continued)

| | | Antibiotic Activity minimum inhibitory concentration ($\mu g/ml$) | | | |
|---|---|---|---|---|---|
| | $R_1$ | H | $-CH_3$ | –OH* | –OH** |
| Compound of formula (VI-b) | $R_4$ | 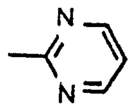 | 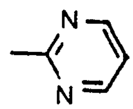 | 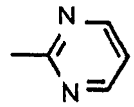 | 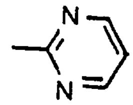 |
| Test Organism | | | | | |
| *Providencia* sp. P8 | | 0.78 | 1.56 | 0.78 | 0.20 |
| *Pseudomonas aeruginosa* IFO3445 | | 12.5 | 25 | 12.5 | 25 |
| *Pseudomonas aeruginosa* NCTC10490 | | 12.5 | 25 | 12.5 | 25 |
| *Serratia marcescens* S18 | | 12.5 | 25 | 50 | 12.5 |
| *Serratia marcescens* T55 | | 25 | >100 | 100 | 12.5 |

TABLE I–4

| | | Antibiotic Activity minimum inhibitory concentration (μg/ml) | | | |
|---|---|---|---|---|---|
| Compound of formula (VI-b) | $R_1$ | H | $-CH_3$ | –OH* | –OH** |
| | $R_4$ | 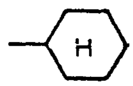 | 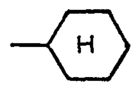 | 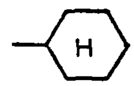 | 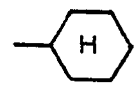 |
| Test Organism | | | | | |
| *Bacillus subtilis* ATCC 6633 | | 0.09 | 0.20 | 6.25 | 0.013 |
| *Sarcina lutea* | | <0.01 | 0.025 | 0.05 | <0.007 |
| *Staphylococcus aureus* FDA209P | | <0.01 | 0.025 | 0.05 | 0.013 |
| *Staphylococcus aureus* Smith | | 0.04 | 0.05 | 0.39 | 0.05 |
| *Staphylococcus aureus* Russell | | <0.01 | 0.025 | 0.20 | 0.013 |
| *Staphylococcus epidermidis* | | 0.04 | 0.05 | 0.39 | 0.05 |
| *Alcaligenes faecalis* A 1 | | 5.6 | 6.25 | 25 | 1.56 |
| *Citrobacter freundii* GN346 | | >180 | >100 | >100 | 100 |
| *Comamonas terrigena* B–996 | | <0.01 | 0.007 | 0.05 | <0.007 |
| *Enterobacter aerogenes* E19 | | >180 | >100 | >100 | 50 |
| *Enterobacter cloacae* 45 | | >180 | >100 | >100 | 50 |
| *Enterobacter* sp. E8 | | >180 | >100 | >100 | 12.5 |
| *Escherichia coli* K–12 | | 180 | >100 | >100 | 12.5 |
| *Escherichia coli* RGN 823 | | 180 | >100 | >100 | 100 |
| *Klebsiella pneumoniae* K–13 | | >180 | >100 | >100 | >100 |
| *Proteus mirabilis* P6 | | 45 | 100 | 100 | 6.25 |
| *Proteus rettgeri* P7 | | 180 | >100 | >100 | 100 |
| *Proteus vulgaris* GN76 | | 180 | >100 | >100 | >100 |
| *Proteus* sp. P22 | | 90 | 100 | >100 | >100 |

TABLE I–4 (Continued)

| | | Antibiotic Activity minimum inhibitory concentration ($\mu g/ml$) | | | |
|---|---|---|---|---|---|
| Compound of formula (VI-b) | $R_1$ | H | $-CH_3$ | –OH* | –OH** |
| | $R_4$ | 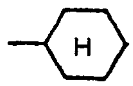 | 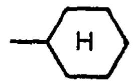 | 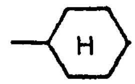 | 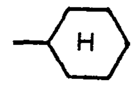 |
| Test Organism | | | | | |
| *Providencia* sp. P8 | | 180 | >100 | >100 | 25 |
| *Pseudomonas aeruginosa* IFO3445 | | 180 | >100 | >100 | >100 |
| *Pseudomonas aeruginosa* NCTC10490 | | 22.5 | 50 | 25 | 50 |
| *Serratia marcescens* S18 | | >180 | >100 | >100 | >100 |
| *Serratia marcescens* T55 | | >180 | >100 | >100 | >100 |

TABLE I–5

| | | Antibiotic Activity minimum inhibitory concentration ($\mu g/ml$) | | | |
|---|---|---|---|---|---|
| Compound of formula (VI-b) | $R_1$ | H | $-CH_3$ | –OH* | –OH** |
| | $R_4$ | 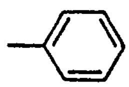 | 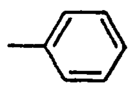 | 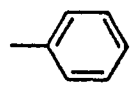 | 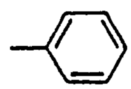 |
| Test Organism | | | | | |
| *Bacillus subtilis* ATCC 6633 | | 0.07 | 0.10 | 6.25 | 0.013 |
| *Sarcina lutea* | | <0.01 | 0.013 | 0.05 | <0.007 |
| *Staphylococcus aureus* FDA 209P | | <0.01 | 0.013 | 0.05 | <0.007 |
| *Staphylococcus aureus* Smith | | 0.1 | 0.2 | 1.56 | 0.10 |
| *Staphylococcus aureus* Russell | | <0.01 | 0.025 | 0.78 | 0.013 |
| *Staphylococcus epidermidis* | | <0.01 | 0.025 | 0.39 | 0.013 |
| *Alcaligenes faecalis* A 1 | | 4.7 | 6.25 | 25 | 1.56 |
| *Citrobacter freundii* GN346 | | 150 | >100 | >100 | 100 |
| *Comamonas terrigena* B–996 | | <0.01 | 0.007 | 0.05 | <0.007 |
| *Enterobacter aerogenes* E19 | | 150 | >100 | >100 | 12.5 |
| *Enterobacter cloacae* 45 | | 150 | >100 | >100 | 12.5 |
| *Enterobacter* sp. E8 | | 75 | >100 | 100 | 3.13 |
| *Escherichia coli* K–12 | | 37.5 | 50 | 50 | 3.13 |
| *Escherichia coli* RGN 823 | | 37.5 | 50 | 50 | 25 |
| *Klebsiella pneumoniae* K–13 | | >150 | >100 | >100 | >100 |
| *Proteus mirabilis* P6 | | 75 | >100 | 100 | 12.5 |
| *Proteus rettgeri* P7 | | 75 | 100 | >100 | 100 |
| *Proteus vulgaris* GN76 | | 150 | >100 | >100 | >100 |
| *Proteus* sp. P22 | | 75 | 100 | >100 | >100 |

TABLE I–5 (Continued)

| | | Antibiotic Activity minimum inhibitory concentration (μg/ml) | | | |
|---|---|---|---|---|---|
| Compound of formula (VI-b) | $R_1$ | H | $-CH_3$ | –OH* | –OH** |
| | $R_4$ | 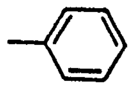 | 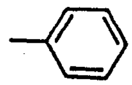 | 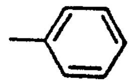 | 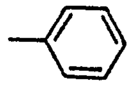 |
| Test Organism | | | | | |
| *Providencia* sp. P8 | | 18.8 | 25 | 50 | 3.13 |
| *Pseudomonas aeruginosa* IFO3445 | | 18.8 | 25 | 25 | 50 |
| *Pseudomonas aeruginosa* NCTC10490 | | 4.7 | 6.25 | 6.25 | 50 |
| *Serratia marcescens* S18 | | 75 | >100 | >100 | 100 |
| *Serratia marcescens* T55 | | 150 | >100 | >100 | 100 |

TABLE I–6

| | | Antibiotic Activity minimum inhibitory concentration (μg/ml) | | | |
|---|---|---|---|---|---|
| Compound of formula (VI-b) | $R_1$ | H | $-CH_3$ | –OH* | –OH** |
| | $R_4$ | $-CH_2CH_2OH$ | $-CH_2CH_2OH$ | $-CH_2CH_2OH$ | $-CH_2CH_2OH$ |
| Test Organism | | | | | |
| *Bacillus subtilis* ATCC 6633 | | 0.1 | 0.39 | 6.25 | 0.013 |
| *Sarcina lutea* | | 0.1 | 0.39 | 1.56 | 0.013 |
| *Staphylococcus aureus* FDA 209P | | 0.05 | 0.20 | 0.39 | 0.013 |
| *Staphylococcus aureus* Smith | | 0.1 | 0.39 | 3.13 | 0.10 |
| *Staphylococcus aureus* Russell | | 0.2 | 0.39 | 3.13 | 0.20 |
| *Staphylococcus epidermidis* | | 0.2 | 0.39 | 3.13 | 0.20 |
| *Alcaligenes faecalis* A 1 | | 1.56 | 1.56 | 6.25 | 0.39 |
| *Citrobacter freundii* GN346 | | 3.13 | 12.5 | 12.5 | 0.78 |
| *Comamonas terrigena* B–996 | | 0.025 | 0.05 | 0.10 | 0.013 |
| *Enterobacter aerogenes* E19 | | 3.13 | 12.5 | 12.5 | 0.39 |
| *Enterobacter cloacae* 45 | | 6.25 | 25 | 6.25 | 1.56 |
| *Enterobacter* sp. E8 | | 3.13 | 12.5 | 6.25 | 0.20 |
| *Escherichia coli* K–12 | | 3.13 | 12.5 | 3.13 | 0.20 |
| *Escherichia coli* RGN 823 | | 12.5 | 12.5 | 12.5 | 6.25 |
| *Klebsiella pneumoniae* K–13 | | 12.5 | 12.5 | 25 | 25 |
| *Proteus mirabilis* P6 | | 12.5 | 25 | 50 | 3.13 |
| *Proteus rettgeri* P7 | | 12.5 | 12.5 | 50 | 6.25 |
| *Proteus vulgaris* GN76 | | 25 | 25 | 50 | 25 |
| *Proteus* sp. P22 | | 50 | 50 | 100 | 100 |
| *Providencia* sp. P8 | | 3.13 | 6.25 | 6.25 | 0.78 |

TABLE I–6 (Continued)

| Compound of formula (VI-b) | | Antibiotic Activity minimum inhibitory concentration ($\mu g/ml$) | | | |
|---|---|---|---|---|---|
| | $R_1$ | H | $-CH_3$ | $-OH$* | $-OH$** |
| | $R_4$ | $-CH_2CH_2OH$ | $-CH_2CH_2OH$ | $-CH_2CH_2OH$ | $-CH_2CH_2OH$ |
| Test Organism | | | | | |
| *Pseudomonas aeruginosa* IFO3445 | | 12.5 | 12.5 | 12.5 | 25 |
| *Pseudomonas aeruginosa* NCTC10490 | | 12.5 | 25 | 12.5 | 25 |
| *Serratia marcescens* S18 | | 6.25 | 12.5 | 25 | 12.5 |
| *Serratia marcescens* T55 | | 12.5 | 50 | 50 | 12.5 |

TABLE I–7

| | | Antibiotic Activity minimum inhibitory concentration ($\mu g/ml$) | | | |
|---|---|---|---|---|---|
| Compound of formula (VI-b) | $R_1$ | H | $-CH_3$ | H | $-CH_3$ |
| | $R_4$ | $-CH_2CH_2N(CH_3)_2$ | $-CH_2CH_2N(CH_3)_2$ | $-CH_2CH_2NHCOCH_2$ 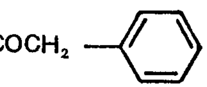 | $-CH_2CH_2NHCOCH_2$ 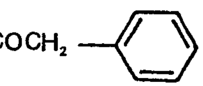 |
| **Test Organism** | | | | | |
| *Bacillus subtilis* ATCC 6633 | | 0.1 | 0.39 | 0.06 | 0.39 |
| *Sarcina lutea* | | 0.2 | 0.78 | 0.03 | 0.10 |
| *Staphylococcus aureus* FDA 209P | | <0.006 | 0.004 | 0.01 | 0.013 |
| *Staphylococcus aureus* Smith | | 0.025 | 0.05 | 0.1 | 0.20 |
| *Staphylococcus aureus* Russell | | 0.2 | 0.39 | 0.1 | 0.20 |
| *Staphylococcus epidermidis* | | 0.2 | 0.39 | 0.2 | 0.39 |
| *Alcaligenes faecalis* A 1 | | 3.13 | 6.25 | 0.86 | 1.56 |
| *Citrobacter freundii* GN346 | | 12.5 | 25 | 27.5 | 50 |
| *Comamonas terrigena* B–996 | | 0.05 | 0.10 | <0.007 | 0.013 |
| *Enterobacter aerogenes* E19 | | 25.0 | 50 | 27.5 | 50 |

TABLE I–7 (Continued)

| Compound of formula (VI-b) | | Antibiotic Activity minimum inhibitory concentration (μg/ml) | | | |
|---|---|---|---|---|---|
| | $R_1$ | H | $-CH_3$ | H | $-CH_3$ |
| | $R_4$ | $-CH_2CH_2N(CH_3)_2$ | $-CH_2CH_2N(CH_3)_2$ | $-CH_2CH_2NHCOCH_2$ 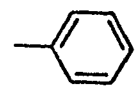 | $-CH_2CH_2NHCOCH_2$ 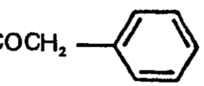 |
| **Test Organism** | | | | | |
| *Enterobacter cloacae* 45 | | 25.0 | 100 | 55.0 | >100 |
| *Enterobacter* sp. E8 | | 12.5 | 50 | 6.88 | 25 |
| *Escherichia coli* K–12 | | 12.5 | 50 | 3.43 | 12.5 |
| *Escherichia coli* RGN 823 | | 12.5 | 12.5 | 13.8 | 12.5 |
| *Klebsiella pneumoniae* K–13 | | 25.0 | 50 | 27.5 | 50 |
| *Proteus mirabilis* P6 | | 50.0 | 50 | 6.88 | 12.5 |
| *Proteus rettgeri* P7 | | 50.0 | 50 | 13.8 | 12.5 |
| *Proteus vulgaris* GN76 | | 50.0 | 50 | 55 | 50 |
| *Proteus* sp. P22 | | 50.0 | 50 | 27.5 | 50 |

TABLE I–7 (Continued)

| Compound of formula (VI-b) | | Antibiotic Activity minimum inhibitory concentration ($\mu g/ml$) | | | |
|---|---|---|---|---|---|
| | $R_1$ | H | $-CH_3$ | H | $-CH_3$ |
| | $R_4$ | $-CH_2CH_2N(CH_3)_2$ | $-CH_2CH_2N(CH_3)_2$ | $-CH_2CH_2NHCOCH_2$ 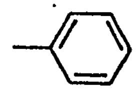 | $-CH_2CH_2NHCOCH_2$ 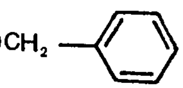 |
| **Test Organism** | | | | | |
| *Providencia* sp. P8 | | 12.5 | 25 | 3.43 | 6.25 |
| *Pseudomonas aeruginosa* IFO3445 | | 6.25 | 6.25 | 55 | 100 |
| *Pseudomonas aeruginosa* NCTC10490 | | 3.13 | 6.25 | 13.8 | 12.5 |
| *Serratia marcescens* S18 | | 50.0 | 100 | 27.5 | 100 |
| *Serratia marcescens* T55 | | 50.0 | >100 | 100 | >100 |

TABLE I–8

| Compound of formula (VI-b) | | Antibiotic Activity minimum inhibitory concentration (μg/ml) | |
|---|---|---|---|
| | $R_1$ | H | $-CH_3$ |
| | $R_4$ | $-(CH_2)_3CH_3$ | $-(CH_2)_3CH_3$ |
| Test Organism | | | |
| *Bacillus subtilis* ATCC 6633 | | 3.75 | 15 |
| *Sarcina lutea* | | 1.88 | 7.5 |
| *Staphylococcus aureus* FDA 209P | | 0.24 | 0.48 |
| *Staphylococcus aureus* Smith | | – | – |
| *Staphylococcus aureus* Russell | | – | – |
| *Staphylococcus epidermidis* | | 0.12 | 0.12 |
| *Alcaligenes faecalis* B 326 | | 15.0 | 20.0 |
| *Citrobacter freundii* GN346 | | – | – |
| *Comamonas terrigena* B–996 | | 0.24 | 0.48 |

TABLE I–9

| Known compound | Antibiotic Activity minimum inhibitory concentration (μg/ml) PS–5 | PS–6 | PS–3A* | PS–3B** | CEZ |
|---|---|---|---|---|---|
| Test Organism | | | | | |
| *Bacillus subtilis* ATCC 6633 | 0.10 | 0.78 | 1.25 | 0.004 | 0.20 |
| *Sarcina lutea* | 0.05 | 0.39 | 0.63 | 0.004 | 0.39 |
| *Staphylococcus aureus* FDA 209P | 0.025 | 0.10 | 0.31 | 0.07 | 0.10 |
| *Staphylococcus aureus* Smith | 0.10 | 0.20 | 1.25 | 0.13 | 0.20 |
| *Staphylococcus aureus* Russell | 0.10 | 0.20 | 1.25 | 0.13 | 0.20 |
| *Staphylococcus epidermidis* | 0.10 | 0.39 | 2.5 | 0.07 | 0.10 |
| *Alcaligenes faecalis* A 1 | 0.39 | 1.56 | 1.25 | 0.07 | 3.13 |
| *Citrobacter freundii* GN346 | 3.13 | 12.5 | 10.0 | 0.53 | >200 |
| *Comamonas terrigena* B–996 | 0.013 | 0.10 | 0.08 | 0.001 | 0.20 |
| *Enterobacter aerogenes* E19 | 3.13 | 25 | 20.1 | 0.27 | >200 |
| *Enterobacter cloacae* 45 | 6.25 | 25 | 10.0 | 1.06 | >200 |
| *Enterobacter* sp. E8 | 1.56 | 6.25 | 5.0 | 0.07 | 3.13 |
| *Escherichia coli* K–12 | 1.56 | 6.25 | 2.5 | 0.07 | 1.56 |
| *Escherichia coli* RGN823 | 3.13 | 6.25 | 2.5 | 0.07 | 1.56 |
| *Klebsiella pneumoniae* K–13 | 6.25 | 25.0 | 10.0 | 8.5 | 200 |
| *Proteus mirabilis* P6 | 6.25 | 12.5 | 20.1 | 0.53 | 6.25 |
| *Proteus rettgeri* P7 | 12.5 | 6.25 | 20.1 | 17.0 | >200 |
| *Proteus vulgaris* GN76 | 12.5 | 12.5 | 20.1 | 8.5 | >200 |
| *Proteus* sp. P22 | 25 | 25 | 20.1 | 17.0 | >200 |
| *Providencia* sp. P8 | 3.13 | 6.25 | 5.0 | 0.27 | 100 |
| *Pseudomonas aeruginosa* IFO3445 | 25 | 50 | 20.1 | 17.0 | >200 |

TABLE I–9 (Continued)

| | Antibiotic Activity minimum inhibitory concentration (μg/ml) | | | | |
|---|---|---|---|---|---|
| Known compound | PS–5 | PS–6 | PS–3A* | PS–3B** | CEZ |
| <u>Test Organism</u> | | | | | |
| *Pseudomonas aeruginosa* NCTC10490 | 12.5 | 50 | 20.1 | 34.0 | >200 |
| *Serratia marcescens* S18 | 6.25 | 25 | 10.0 | 2.13 | >200 |
| *Serratia marcescens* T55 | 6.25 | 50 | 20.1 | 2.13 | >200 |

*: 5,6-trans isomer
**: 5,6-cis isomer

PS–5 :

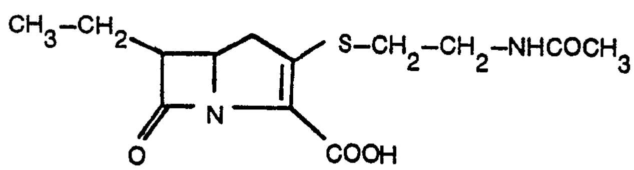

PS–6 :

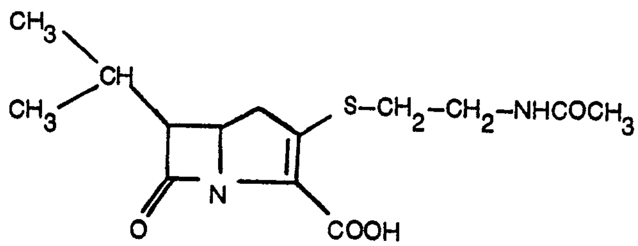

PS–3A :

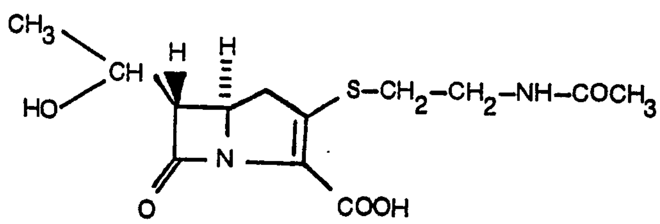

PS–3B :

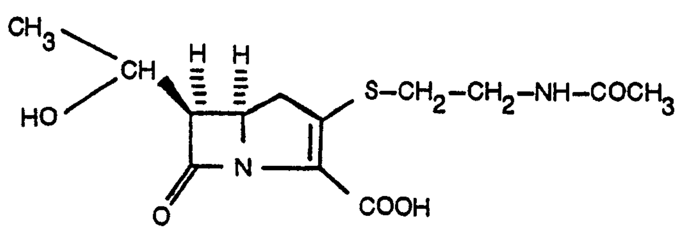

CEZ (cephazolin):

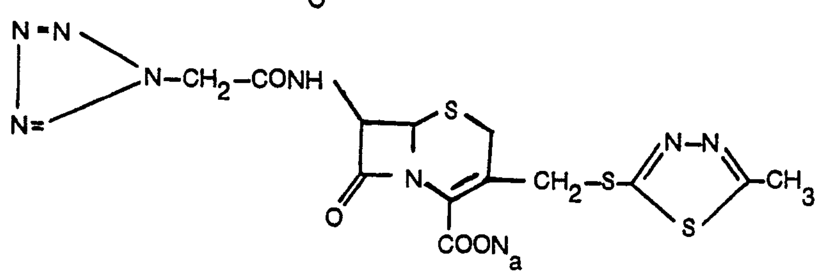

The antibiotic activities shown in Tables I—1 and I—9 above were determined in the following manner.

The minimum inhibitory concentrations of the compounds of formula (VI-b) in Tables I—1 to I—8 and the known compounds in Table I—9 on the various microorganisms shown in the above tables were measured by an agar dilution assay method using a brain heart infusion agar medium "Difco" (a product of Difco Laboratories Inc.).

A sample of the test compound was dissolved in sterile distilled water and diluted to prepare a two-fold dilution series of the compound solution. In a Petri dish of 9 cm in diameter, 1 ml of the compound solution was added to 9 ml of a molten sterile hear infusion agar medium (Difco Laboratories Inc.) at about 60°C to prepare an agar plate containing the compound. An overnight culture of each test organism indicated in the above table, which was prepared by stationary cultivation in trypto-soy broth (Eiken Chemi. Co., Ltd.) at 35°C for 18 to 20 hours, was diluted with the trypto-soy broth to give approximately $10^8$ cells/ml and the diluted inoculum was inoculated in the agar plate. After cultivating the plate at 35°C for 20 hours, the growth of the organism was observed.

The minimum concentration of the compound at which no growth of the microorganism was noted was determined, and defined as the minimum inhibitory concentration of the test compound.

Typical species of the compounds of formula (VI-b) were each administered subcutaneously to five mice in a dose of 2 mg. After 5 minutes from the administration, a small amount of blood was periodically drawn from the eyes of the mice into a heparinized capillary tube, and centrifuged. The concentration of the compound in the supernatant liquid (plasma) was determined by a bioassay method using *Comamonas terrigena* B—996. The results as an average on the five mice are given in Table II.

TABLE II

Test for concentration in blood

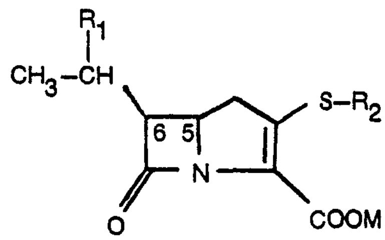

| Compound of above formula | | | Plasma of mouse | | |
|---|---|---|---|---|---|
| $R_1$ | $R_2$ | M | Dose/ (mg/ mouse) | Maximum concen- tration ($\mu$g/ml) | Half period (min.) **** |
| H | 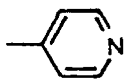 | H | 2 | 70.7 | 16.0 |
| $-CH_3$ | 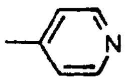 | H | 2 | 30.7 | 15.2 |
| $-OH$ | 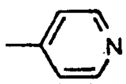 | H* | 2 | 103.4 | 18.0 |
| $-OH$ | 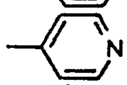 | H** | 2 | 29.0 | 13.5 |
| H | 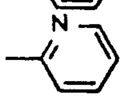 | H | 2 | 48.4 | 29.0 |
| H | 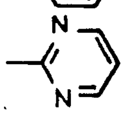 | H | 2 | 23.6 | 22.0 |
| $-CH_3$ | 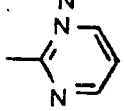 | H | 2 | 21.2 | 18.2 |
| $-OH$ | 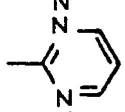 | H* | 2 | 54.0 | 25.0 |
| $-OH$ | 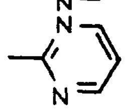 | H** | 2 | 23.3 | 19.0 |
| H | 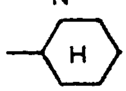 | Na | 2 | 8.24 | 7.2 |
| H | 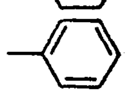 | Na | 2 | 11.3 | 25.2 |
| H | $-CH_2CH_2N(CH_3)_2$ | H | 2 | 36.2 | 6.0 |
| $-CH_3$ | $-CH_2CH_2N(CH_3)_2$ | H | 2 | 22.0 | 6.1 |
| H | $-CH_2CH_2OH$ | Na | 2 | 36.9 | 6.0 |
| H | $-CH_2CH_2NHCOCH_2$ 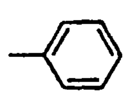 | Na | 2 | 2.8 | 6.0 |

TABLE II (Continued)

| Compound of above formula | | Plasma of mouse | | |
|---|---|---|---|---|
| | | Dose (mg/mouse) | Maximum concentration ($\mu$g/ml) | Half period (min.) **** |
| Known compounds | PS–5.Na*** | 2 | 40.0 | 7.0 |
| | PS–6.Na*** | 2 | 23.8 | 5.0 |
| | PS–3A.Na*** | 2 | 52.0 | 12.0 |
| | PS–3B.Na*** | 2 | 26.3 | 5.0 |

*: 5,6-trans isomer
**: 5,6-cis isomer
***: Sodium salt
****: The period that elapsed until the concentration was reduced to half.

The compounds of formula (VI-b) tested were well absorbed, and gave considerably high maximum concentrations. In particular, compounds of formula (VI-b) in which $R_2$ is an aromatic heterocyclic group have a superior maximum concentration. Compounds of formula (VI-b) in which the S side-chain at the 3-position has a phenyl group (i.e., $R_2$=phenyl) have a long half period and thus maintained a high blood level over a long period of time.

The *in vivo* activities of the compounds of formula (VI-b) were determined by using mice (five per group, male DDY mice, Shizuoka) which had been intraperitoneally infected with *Staphylococcus aureus* Smith strain in an amount of $5 \times 10^5$ cells per mouse. Two hours after the infection, an injection containing a sodium salt of each of the compounds of formula (VI-b) was subcutaneously administered to the mice. The results of the infection treating test ($CD_{50}$) are shown in Table III.

Infection treating test

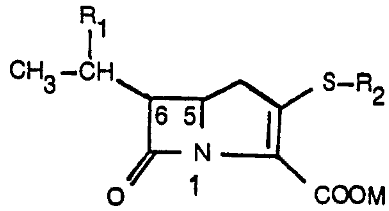

| Compound of above formula | | | |
|---|---|---|---|
| $R_1$ | $R_2$ | M | $CD_{50}$ (mg/kg) |
| H | 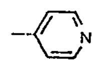 | H | 0.063 |
| $-CH_3$ | 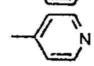 | H | 0.5 |
| $-OH$ | 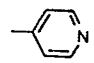 | H* | 0.125 |
| $-OH$ | 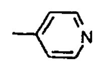 | H** | 0.125 |
| H | 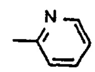 | H | 0.125 |
| H | 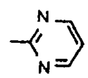 | H | 0.25 |
| $-CH_3$ | 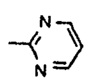 | H | 1.0 |
| $-OH$ | 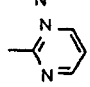 | H* | 0.25 |
| $-OH$ | 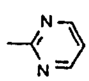 | H** | 0.25 |
| H | $-CH_2CH_2OH$ | Na | 1.0 |
| H | $-CH_2CH_2N(CH_3)_2$ | H | 0.25 |
| $-CH_3$ | $-CH_2CH_2N(CH_3)_2$ | H | 2.0 |
| Known compounds | PS-5.Na | | 1.0 |
| | PS-6.Na | | 8.0 |
| | PS-3A.Na | | 4.0 |
| | PS-EB.Na | | 2.0 |

*: 5,6-trans isomer

**: 5,6-cis isomer

The data of the *in vitro* and *in vivo* activites given above show the compounds of formula (VI-b) and their salts to have high antimicrobial activites and/or stability both *in vitro* and *in vivo.* They can be effectively used as an active ingredient of antimicrobial agents for the prevention, treatment and/or medication of infectious. diseases caused by Gram-positive and Gram-negative bacteria not only in humans but also in other animals such as mammals, poultry, and fish.

The compounds of formula (VI-b) or the salts thereof can be administered orally, topically or parenterally (intravenously, intramuscularly, intraperitioneally, etc.). As is usual, these compounds can be formulated into various dosage forms according to the route of administration.

The compounds of formula (I) provided by this invention show excellent antimicrobial activites in *in vitro* tests in horse serum as shown in Table IV below.

TABLE IV

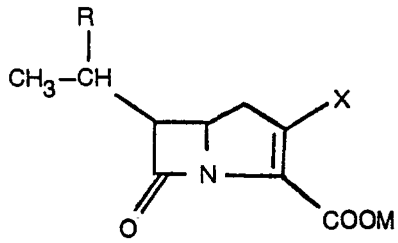

| Compound of above formula | | | Inhibitory zone diameter(*) (mm) |
|---|---|---|---|
| R | X | M | |
| H | Br | $CH_3$ | 29.7 |
| H | Br | $CH_2$ 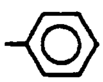 | 30.2 |
| H | Br | $CH_2$ 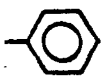 $-NO_2$ | 28.6 |
| $CH_3$ | Br | $CH_2$ 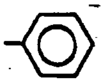 $-NO_2$ | 26.8 |
| OH | Br | $CH_2$ 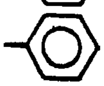 $-NO_2$ | 31.2 |
| H | I | $CH_3$ | 29.6 |
| H | I | $CH_2$ 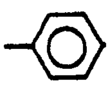 | 30.0 |
| OH | I | $CH_3$ | 31.0 |

(*): A nutrient broth (a product of Kyokuto Co.) containing 10% horse serum (a product of Nippon Biotest Co., Ltd.); test microorganism S. aureus; assay method by disc diffusion.

The following Examples illustrate the present invention more specifically. It should be understood however that the invention is in no way limited by these Examples.

Example 1

Production of benzyl 3-bromo-6-ethyl-7-oxo-1-azabicyclo[3.2.0]hept-2-ene-2-carboxylate:—

A solution of 28 mg (0.149 M) of N-bromo-succinimide in 5 ml of methylene chloride was added dropwise with stirring under ice cooling to a solution of 50 mg (0.124 mM) of benzyl 3-(2-acetamido-ethylsulfinyl)-6-ethyl-7-oxo-1-azabicyclo[3.2.0]hept-2-ene-2-carboxylate (abbreviated the sulfoxide of PS—5.benzyl ester) in 15 ml of methylene chloride. The mixture was stirred at 15 to 20°C for 1 hour. The reaction mixture was adsorbed onto a slica gel column (2.7 x 12 cm, 35 g, Kieselgel 60, 70—230 mesh, a product of E. Merck Co.) previously treated with benzene-acetone (20:1 v/v), and developed and eluted with the same mixed solvent as above into 15 g-fractions. Each of the fractions was distilled to remove the solvent, and its ultraviolet absorption spectrum was measured in tetrahydrofuran (THF). Fractions which showed a maximum absorption at 290 nm were collected, and distilled to remove the solvent. There was obtained 2.3 mg of the captioned compound.

UV $\lambda_{max}^{THF}$ nm: 291.

IR $\nu_{max}^{CCl_4}$ $cm^{-1}$: 1795 (β-lactam C=O), 1735 (ester CO).

NMR ($C_6D_6$) δ ppm: 0.65 (3H, t, J=8Hz, —$CH_2CH_2$), 1.05—1.45 (2H, m, —$CH_2CH_3$), 2.20 (2H, d, J=10Hz, C—$4H_2$), 2.37 (1H, dt, J=6Hz, J=3Hz, C—6H), 3.04 (1H, dt, J=10Hz, J=3Hz, C—5H), 5.12 (2H, s, $CH_4$Ar).

Mass m/e: 351, 349 ($M^+$), 281, 279 ($M^+$—EtCH=C—O).

Example 2

Production of p-nitrobenzyl 3-bromo-6-ethyl-7-oxo-1-azabicyclo[3.2.0]hept-2-ene-2-carboxylate:—

The same procedure as in Example 1 was performed using 50 mg (0.11 mM) of p-nitrobenzyl 3-(2-acetamidoethylsulfinyl)-6-ethyl-7-oxo-1-azabicyclo[3.2.0]hept-2-ene-2-carboxylate (abbreviated the sulfoxide of PS—5.p-nitrobenzyl ester) and 22 mg (0.12 mM) of N-bromosuccinimide. There was obtained 2.1 mg of the captioned compound.

UV $\lambda_{max}^{THF}$ nm: 290.

IR $\nu_{max}^{CCl_4}$ $cm^{-1}$: 1790 (β-lactam C=O), 1735 (ester C=O).

NMR ($C_6D_6$) δ ppm: 0.63 (3H, t, J=7Hz, $CH_2CH_3$), 1.10—1.50 (2H, m, $CH_2CH_3$), 2.21 (2H, d, J=9Hz, C—4Hz), 2.36 (1H, dt, J=6Hz, J=3Hz, C—6H), 3.04 (1H, dt, J=9Hz, J=3Hz, C—5H), 4.66 (1H, d, J=14Hz, CHHAr), 4.92 (1H, d, J=14Hz, CHHAr), 6.96 (2H, d, J=9Hz, ArH), 7.70 (2H, d, J=9Hz, ArH).

Mass m/e: 396, 394 ($M^+$), 326, 324 ($M^+$—Et—CH=C=O).

Example 3

Production of methyl 3-bromo-6-ethyl-7-oxo-1-azabicyclo[3.2.0]hept-2-ene-2-carboxylate:—

42.3 mg (0.129 mM) of methyl 3-(2-acetamidoethylsulfinyl)-6-ethyl-7-oxo-1-azabicyclo[3.2.0]-hept-2-ene-2-carboxylate (abbreviated the sulfoxide of PS—5. methyl ester) was dissolved in 8.0 ml of dry methylene chloride. With stirring under ice cooling, a solution of 25.2 mg (0.141 mM) of N-bromosuccinimide in 3.0 ml of dry methylene chloride was added. The reaction temperature was raised to 20°C, and the mixture was kept stirred for 2 hours. After the reaction, the reaction mixture was passed through a silica gel column (18.0 x 31.5 cm; 40 g; the same as the silica gel column described in Example 1) previously wetted with benzene-acetone (20:1), and developed with the same solvent as above to give 5 g-fractions. These fractions were examined by UV spectra, and the final product eluted in the 23rd to 31st fractions was obtained in an amount of 3.2 mg.

UV $\lambda_{max}^{THF}$ nm: 289.5.

IR $\nu_{max}^{THF}$ $cm^{-1}$: 1790 (β-lactam CO), 1730 (ester CO).

NMR ($C_6D_6$) δ ppm: 0.66 (3H, t, J=7.0Hz, —$CH_2CH_3$), 1.00—1.50 (2H, m, —$CH_2CH_3$), 2.20 (2H, d, J=9Hz, C—$4H_2$), 2.37 (1H, dt, J=7Hz, J=3Hz, C—6H), 3.06 (1H, dt, J=9Hz, J=3Hz, C—5H), 3.41 (3H, s, —$OCH_3$).

FE-Mass m/e: 275, 273 ($M^+$).

Example 4

Production of p-nitrobenzyl 3-bromo-6-(1-hydroxyethyl)-7-oxo-1-azabicyclo[3.2.0]hept-2-ene-2-carboxylate:

(A) 50 mg (0.120 mM) of p-nitrobenzyl 3-(2-acetamidoethylsulfinyl)-6-(1-hydroxyethyl)-7-oxo-1-azabicyclo[3.2.0]hept-2-ene-2-carboxylate (abbreviated the sulfoxide of PS—5.p-nitrobenzyl ester) and 27 mg (0.120 mM) of N-bromosuccinimide were reacted and treated in the same way as in Example 1. There was obtained 1.9 mg of p-nitrobenzyl (5R, 6R-S, 8S)3-bromo-6-(1-hydroxyethyl)-7-oxo-1-azabicyclo[3.2.0]]hept-2-ene-2-carboxylate.

UV $\lambda_{max}^{THF}$ nm: 290.

IR $\nu_{max}^{THF}$ $cm^{-1}$: 3460 (—OH), 1790 (β-lactam CO), 1735 (ester CO).

Mass m/e: 412, 410 ($M^+$).

(B) N-acetyl-thienamycin p-nitrobenzylester S-oxide obtained by the method described in U.S. Patent No. 4,123,547 was treated by the method described in (A) above to afford p-nitrobenzyl (5R, 6S, 8R)3-bromo-6-(1-hydroxyethyl)-7-oxo-1-azabicyclo[3.2.0]hept-2-ene-2-carboxylate.

Example 5

Production of benzyl 3-bromo-6-isopropyl-7-oxo-1-azabicyclo[3.2.0]hept-2-ene-2-carboxylate:—

50 mg (0.120 mM) of benzyl 3-(2-acetamidoethylsulfinyl)-6-isopropyl-7-oxo-1-azabicyclo-[3.2.0]hept-2-ene-2-carboxylate (abbreviated the sulfoxide of PS—6.benzyl ester) and 27 mg (0.152 mM) of N-bromo-succinimide were reacted and treated in the same way as in Example 1 to afford 2.3 mg of the captioned compound.

UV $\lambda_{max}^{THF}$ nm: 291.
IR $\nu_{max}^{CCl_4}$ cm$^{-1}$: 1795 ($\beta$-lactam CO), 1735 (ester CO).
NMR ($C_6D_6$) $\delta$ ppm: 0.63 (3H, d, J=7.0Hz,

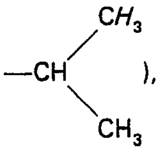

), 0.68 (3H, d, J=7.0Hz,

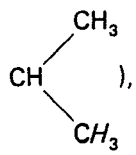

), 1.30—1.95 (1H, m,

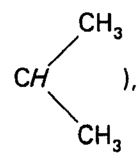

), 2.22 (2H, d, J=10.0Hz, C—4$H_2$), 2.37 (1H, dd, J=3.0Hz, J=9.0Hz, C—6H), 3.05 (1H, dt, J=3.0Hz, J=10.0Hz, C—5*H*), 5.13 (2H, s, C$H_2$Ar).
Mass m/e: 365, 363 ($M^+$), 281, 279

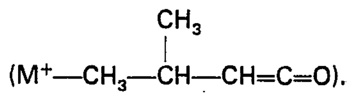

Example 6

Production of methyl 3-iodo-6-ethyl-7-oxo-1-azabicyclo[3.2.0]hept-2-ene-2-carboxylate:

50 mg (0.15 mM) of the sulfoxide of PS—5.methyl ester was dissolved in 30 ml of methylene chloride, and at room temperature 68 mg (0.30 mM) of N-iodosuccinimide was added. They were reacted for 2 hours under reflux. The reaction mixture was cooled to room temperature and subjected to a silica gel column (2.7 × 15 cm; the same as the silica gel described in Example 1). Benzene (100 ml) was caused to flow through it, and the column was developed and eluted with benzene-acetone (40:1 v/v) to obtain 15 g-fractions. Fractions which showed a maximum absorption at 290 nm in their ultraviolet absorption spectra were collected, and the solvent was distilled off to afford 2.3 mg of the captioned compound.

UV $\lambda_{max}^{THF}$ nm: 290.
IR $\nu_{max}^{THF}$ cm$^{-1}$: 1790 ($\beta$-lactam), 1730 (ester CO).
NMR ($C_6D_6$) $\delta$ ppm: 0.65 (3H, t, J=7Hz, $CH_2CH_3$). 1.10—1.50 (2H, m, $CH_2CH_3$), 2.30 (2H, d, J=9Hz, C—4$H_2$), 2.37 (1H, dt, J=6Hz, J=3Hz, C—6H), 3.04 (1H, dt, J=9Hz, J=3Hz, C—5H), 3.42 (3H, s, —OC$H_3$).
FD-Mass m/e: 321($M^+$).

Example 7

Production of benzyl 3-iodo-6-ethyl-7-oxo-1-azabicyclo[3.2.0]hept-2-ene-2-carboxylate:—

50 mg (0.12 mM) of the sulfoxide of PS—5.benzyl ester was dissolved in 28 mg (0.24 mM) of N-iodosuccinimide at room temperature, and then they were treated at 50°C for 90 minutes. The reaction mixture was chromatographed in the same way as in Example 6 to give 2.0 mg of the captioned compound.

UV $\lambda_{max}^{THF}$ nm: 290.
IR $\nu_{max}^{THF}$ cm$^{-1}$: 1785 ($\beta$-lactam), 1725 (ester CO).
NMR ($C_6D_6$) $\delta$ ppm: 0.63 (3H, t, J=7Hz, —$CH_2CH_3$), 1.15—1.50 (2H, m, $CH_2CH_3$), 2.30 (2H, d, J=9Hz, C—4$H_2$), 2.38 (1H, dt, J=6Hz, J=3Hz, C—6*H*), 3.04 (1H, dt, J=9Hz, J=3Hz, C—5*H*), 5.11 (2H, s, C$H_2$Ar).
FD-Mass m/e: 397 ($M^+$), 327 (M—EtHC=C=O).

Example 8

Production of benzyl 3-iodo-6-(1-hydroxyethyl)-7-oxo-1-azabicyclo[3.2.0]hept-2-ene-2-carboxylate:—

50 mg (0.108 mM) of the sulfoxide of PS—3.benzyl ester was dissolved in 30 ml of methylene chloride, and 29 mg (0.129 mM) of N-iodosuccinimide was added at room temperature. They were reacted, and the reaction product purified, in the same way as in Example 6 to afford 1.9 mg of the captioned compound.

UV $\lambda_{max}^{THF}$ nm: 291.
IR $\nu$ (THF) cm$^{-1}$: 1785 ($\beta$-lactam), 1720 (ester).
FD-Mass m/e: 413 ($M^+$).

Example 9

Production of benzyl 3-n-butylthio-6-ethy-7-oxo-1-azabicyclo[3.2.0]hept-2-ene-2-carboxylate:—

5.0 mg (0.014 mM) of benzyl 3-bromo-6-ethyl-7-oxo-1-azabicyclo[3.2.0]hept-2-ene-2-carboxylate obtained in Example 1 was dissolved in 3.0 ml of dimethylformamide (DMF), and the solution was cooled to −50°C. A solution of 2.3 mg (0.21 mM) of n-butyl mercaptan sodium salt in 1.0 ml of DMF was added to the resulting solution. They were reacted at −50°C for 10 minutes. The reaction mixture was poured into 20 ml of benzene, and washed with 0.1M phosphate buffer (20 ml × 3) at pH 6.8. The benzene layers were dried over anhydrous sodium sulfate, and the solvent was distilled off under reduced pressure. The residue was dissolved in a small amount of benzene, and passed through a silica gel column (4 g, 0.4 × 20 cm, the same as the silica gel described in Example 1) previously wetted with benzene, and developed with benzeneacetone (80:1). The eluates were then passed through a column of Biobeads S—X3 (100 g, 1.2 × 88.0 cm, a product of Bio-Rad Co.) wetted with benzene, to afford 4.1 mg of the captioned compound.

UV $\lambda_{max}^{THF}$ nm: 318.
IR $\nu_{max}^{CHCl_3}$ cm$^{-1}$: 1765 (β-lactam CO), 1695 (ester CO).
NMR (CDCl$_3$)δ ppm: 0.92 (3H, t, J=7.0Hz, —CH$_2$*CH$_3$*), 1.06 (3H, t, J=7.0Hz, C—9H), 1.20—2.00 (6H, m), 2.70—3.40 (5H, m), 3.94 (1H, dt, J=3.0Hz, J=9.0Hz, C—5H), 5.21 (2H, d, J=14.0Hz, C*H*HAr), 5.50 (2H, d, J=14.0Hz, CH*H*Ar), 7.35 (5H, s).
Mass m/e: 369 (M$^+$), 299 (M$^+$—CH$_3$CH$_2$CH=C=O).

Example 10

Production of *p*-nitrobenzyl 3-n-butylthio-6-ethyl-7-oxo-1-azabicyclo[3.2.0]hept-2-ene-2-carboxylate:—

6.0 mg (0.015 mM) of *p*-nitrobenzyl 3-bromo-6-ethyl-7-oxo-1-azabicyclo[3.2.0]hept-2-ene-2-carboxylate obtained in Example 2 and 2.4 mg (0.021 mM) of n-butyl mercaptan sodium salt were reacted, and the reaction product purified, in the same way as in Example 9 to afford 5.2 mg of the captioned compound.

UV $\lambda_{max}^{THF}$ nm: 322.
IR $\nu_{max}^{CHCl_3}$ cm$^{-1}$: 1765 (β-lactam CO), 1695 (ester CO).
NMR (CDCl$_3$)δ ppm: 0.80—1.18 (6H, m, 2X—CH$_2$CH$_3$), 1.20—2.00 (6H, m, 3X—*CH$_2$*—), 2.70—3.30 (5H, m, 2X—*CH$_2$*—, —CH—), 3.94 (1H, dt, J=9Hz, J=3Hz, C—5H), 5.19 (1H, d, J=14Hz, —*C*HHAr), 5.49 (1H, d, J=14Hz, —CH*H*Ar), 7.62 (2H, d, J=9Hz, ArH), 8.18 (2H, d, J=9Hz, ArH).
Mass m/e: 404 (M$^+$), 334 (M$^+$—Et—CH=C=O).

Example 11

Production of benzyl 3-n-butylthio-6-(1-hydroxyethyl)-7-oxo-1-azabicyclo[3.2.0]hept-2-ene-2-carboxylate:—

5.0 mg (0.012 mM) of benzyl 3-iodo-6-(1-hydroxyethyl)-7-oxo-1-azabicyclo[3.2.0]hept-2-ene-2-carboxylate obtained in Example 8 and 2.2 mg (0.020 mM) of n-butyl mercaptan sodium salt were reacted, and the reaction product purified, in the same way as in Example 9 to afford 3.6 mg of the captioned compound.

UV $\lambda_{max}^{THF}$ nm: 318.
IR $\nu_{max}^{CHCl_3}$ cm$^{-1}$: 1765 (β-lactam CO), 1695 (ester CO).
NMR (CDCl$_3$)δ ppm: 1.00 (3H, t, J=7.0Hz), 1.20—2.20 (7H, m), 2.70—3.40 (5H, m), 4.00—4.55 (2H, m), 5.22 (2H, d J=14.0Hz, C*H*HAr), 5.51 (2H, d, J=14.0Hz), 7.38 (5H, s).
Mass m/e: 385 (M$^+$).

Example 12

Production of benzyl 3-n-butylthio-6-isopropyl-7-oxo-1-azabicyclo[3.2.0]hept-2-ene-2-carboxylate:—

10 mg (0.024 mM) of benzyl 3-bromo-6-isopropyl-7-oxo-azabicyclo[3.2.0]hept-2-ene-2-carboxylate obtained in Example 5 and 4.4 mg (0.04 mM) of n-butyl mercaptan sodium salt were reacted, and the reaction product purified, in the same way as in Example 9 to afford 8.0 mg of the captioned compound.

UV $\lambda_{max}^{THF}$ nm: 318.
IR $\nu_{max}^{CHCl_3}$ cm$^{-1}$: 1768 (β-lactam-CO), 1695 (ester CO).
NMR (CDCl$_3$)δ ppm: 0.80—1.15 (9H, m), 1.20—2.20 (5H, m), 2.62—3.50 (5H, m), 3.94 (1H, dt, J=3.0Hz, J=9.0Hz), 5.35 (2H, ABq J=14.0Hz), 7.35 (5H, s).

Example 13

Production of *p*-nitrobenzyl 3-(4-pyridylthio)-6-ethyl-7-oxo-1-azabicyclo[3.2.0]hept-2-ene-carboxylate:—

6.0 mg (0.015 mM) of *p*-nitrobenzyl 3-bromo-6-ethyl-7-oxo-1-azabicyclo[3.2.0]hept-2-ene-2-carboxylate obtained in Example 2 was dissolved in 3.0 ml of DMF, and the solution was cooled to

−50°C. A solution of 3.0 mg (0.023 mM) of 4-pyridine thiol sodium salt in 1.0 ml of DMF was added to the resulting solution. The mixture was reacted at −50°C for 10 minutes. The reaction mixture was poured into 20 ml of benzene, and washed with 0.1M phosphate buffer (20 ml × 3) at pH 6.8. The benzene layer was dried over anhydrous sodium sulfate, and the solvent was distilled off under reduced pressure. The residue was dissolved in a small amount of benzene, and passed through a silica gel column (0.4 × 20 cm, 4 g) previously wetted with benzene, and developed with benzene-acetone (5:1 v/v). The eluates were then passed through a column of Biobeads S—X3 (100 g, 1.2 × 88.0 cm, a product of Bio-Rad Co.) wetted with benzene to afford 3.2 mg of the captioned compound (yield 49.6%).

$[\alpha]_D^{22}$: +18.4° (C 1.00, THF).
UV $\lambda_{max}^{THF}$ nm ($\varepsilon$): 322 7900), 267 (8700).
IR $\nu_{max}^{CHCl_3}$ cm$^{-1}$: 1785 (β-lactam CO), 1720 (ester CO).
NMR ($CDCl_3$)δ ppm: 1.10 (3H, t, J=7.5Hz, $CH_2CH_3$), 1.64—2.04 (2H, m, $CH_2CH_3$), 2.60—3.24 (3H, m, C—$4H_2$, C—6*H*), 3.92 (1H, dt, J=9Hz, J=3Hz, C—5*H*), 5.30 (1H, d, J=14Hz, ArC*H*H), 5.58 (1H, d, J=14Hz, ArCH*H*), 7.40 (2H, dd, J=7Hz, J=3Hz, PyH), 7.68 (2H, d, J=9Hz, ArH), 8.41 (2H, d, J=9Hz, ArH), 8.60 (2H, dd, J=7Hz, J=3Hz, PyH).
Mass (m/e): 425 ($M^+$), 355 ($M^+$—EtCH=C=O).

## Example 14

Production of *p*-nitrobenzyl 3-(4-pyridylthio)-6-(1-hydroxyethyl)-7-oxo-1-azabicyclo[3.2.0]hept-2-ene-2-carboxylate:—

50 mg (0.123 mM) of *p*-nitrobenzyl 3-bromo-6-(1-hydroxyethyl)-7-oxo-1-azabicyclo[3.2.0]hept-2-ene-2-carboxylate obtained in Example 4 was dissolved in 20 ml of DMF, and the solution was cooled to −50°C. A solution of 20 mg (0.15 mM) of sodium salt of 4-pyridinethiol in 5.0 ml of DMF was added to the resulting solution. The mixutre was reacted at −50°C for 30 minutes. Then, the reaction mixture was poured into 100 ml of ethyl acetate, and washed with 0.1M phosphate buffer (80 ml × 3) at oH 6.8. The ethyl acetate layer was dried over anhydrous sodium sulfate, and the solvent was distilled off to afford a yellow oily product. When this product was subjected to a column (0.5 × 40.0 cm) filled with 8 g of silica gel using benzene, 5,6-trans and 5,6-cis isomers of the product were obtained from fractions eluted with the benzene-acetone (10:1, v/v). The solvent was distilled off under reduced pressure, and each of these isomers were dissolved in a small amount of benzene, and passed through a column of Bioheads S—X3 (100 g, 1.2 × 88.0 cm, a product of Bio-Rad Co.) wetted with benzene to afford 10.2 mg of a 5,6-trans isomer of the captioned compound and 8.4 mg of a 5,6-cis isomer of the captioned compound (yield 34.7%).

*5,6-trans isomer*
UV $\lambda_{max}^{THF}$ nm ($\varepsilon$): 322 (8500), 267 (9200).
IR $\nu_{max}^{CHCl_3}$ cm$^{-1}$: 1785 (β-lactam) CO, 1720 (ester).
NMR ($CDCl_3$)δ ppm: 1.35 (3H, d, J=7.0Hz, CH—$CH_3$), 2.60—3.50 (3H, m, C—$4H_2$, C—6*H*), 3.80—4.35 (2H, m, C—5H, HOC*H*—$CH_3$), 5.28 (1H, d, J=14.0Hz, ArC*H*H), 5.54 (1H, d, J=14.0Hz, ArCH*H*), 7.39 (2H, dd, J=6.0Hz, J=2.0Hz, Py*H*), 7.68 (2H, d, J=9.0Hz, Ar*H*), 8.24 (2H, d, J=9.0Hz, Ar*H*), 8.60 (2H, dd, J=6.0Hz, J=2.0Hz, Py*H*).

*5,6-cis isomer*
UV $\lambda_{max}^{THF}$ nm ($\varepsilon$): 322 (8300, 267 (9100).
IR $\nu_{max}^{CHCl_3}$ cm$^{-1}$: 1785 (β-lactam CO), 1720 (ester).
NMR ($CDCl_3$)δ ppm: 1.37 (3H, d, J=7.0Hz, CH$CH_3$), 2.60—4.40 (5H, m, C—$4H_2$, C—5*H*, C—6*H*, HOC*H*$CH_3$), 5.28 (1H, d, J=14.0Hz, ArCHH), 5.56 (1H, d, J=14.0Hz, ArCH*H*), 7.38 (2H, dd, J=6.0Hz, J=2.0Hz, PyH), 7.66 (2H, d, J=9.0Hz, Ar*H*), 8.24 (2H, d, J=9.0Hz, Ar*H*), 8.62 (2H dd, J=6.0Hz, J=2.0Hz, Py*H*).

## Example 15

Production of benzyl 3-(4-pyridylthio)-6-ethyl-7-oxo-1-azabicyclo[3.2.0]hept-2-ene-2-carboxylate:—

5.0 mg (0.014 mM) of benzyl 3-bromo-6-ethyl-7-oxo-1-azabicyclo[3.2.0]hept-2-ene-2-carboxylate was dissolved in 3.0 ml of dimethylformamide (DMF), and the solution was cooled to −50°C. A solution of 2.8 mg (0.021 mM) of sodium salt of 4-pyridinethiol in 1.0 ml of DMF was added to the resulting solution. The mixture was reacted at −50°C for 10 minutes. The reaction mixture was then poured into 20 ml of benzene, and washed with 0.1M phosphate buffer (200 ml × 3) at pH 6.8. The benzene layer was dried over anhydrous sodium sulfate, and the solvent was distilled off under reduced pressure. The residue was dissolved in a small amount of benzene, and passed through a column of silica gel (4 g; 0.4 × 20 cm), and eluted with benzene-acetone 5:1 v/v). The eluates were then passed through a column of Biobeads S—X3 (100 g; 1.2 × 88.0 cm; a product of Bio-Rad Co.) wetted with benzene to aford 2.6 mg of the captioned compound (yield 48.0%).

UV $\lambda_{max}^{THF}$ nm($\varepsilon$): 322 (8100).
IR $\nu_{max}^{CHCl_3}$ $cm^{-1}$: 1785 ($\beta$-lactam CO), 1720 (ester CO).
NMR ($CDCl_3$)$\delta$ ppm: 1.07 (3H, t, J=7.5Hz, $CH_2CH_3$), 1.60—2.00 (2H, m, $CH_2CH_3$), 2.50—3.20 (3H, m, C—4$H_2$, C—6*H*), 3.88 (1H, dt, J=9Hz, J=3Hz, C—5*H*), 5.36 (1H, d, J=12Hz, ArC*H*H), 5.49 (1H, d, J=12Hz, ArCH*H*), 7.30—7.44 (2H, m, PyH), 8.50—8.70 (2H, m, PyH).
Mass (m/e): 380 ($M^+$) 310 ($M^+$—EtCH=C=O).

Example 16

Production of *p*-nitrobenzyl 3-(4-pyridylthio)-6-isopropyl-7-oxo-1-azabicyclo[3.2.0]hept-2-ene-2-carboxylate:—

10 ml (0.024 mM) of *p*-nitrobenzyl 3-bromo-6-isopropyl-7-oxo-1-azabicyclo[3.2.0]hept-2-ene-2-carboxylate obtained by the similar method as described in Example 5 was dissolved in 6.0 ml of DMF, and the solution was cooled to −50°C. A solution of 4.8 mg (0.036 mM) of sodium salt of 4-pyridinethiol in 2.0 ml of DMF was added to the resulting solution. The mixture was reacted at −50°C for 10 minutes. The reaction mixture as poured into 50 ml of benzene, and washed with 0.1M phosphate buffer (30 ml ×3) at pH 6.8. The benzene layer was dried over anhydous sodium sulfate, and the solvent was distilled under reduced pressure. The residue was dissolved in a small amount of benzene, and passed through a column of silica gel (4 g, 0.4 ×20 cm) wetted previously with benzene, and developed with benzene-acetone (5:1 v/v). The eluates were then passed through a column of Biobeads S—X3 (100 g, 1.2 × 88.0 cm, a product of Bio-Rad Company) wetted with benzene to afford 5.5 mg of the captioned compound yield 51.2%).

UV $\lambda_{max}^{THF}$ nm($\varepsilon$): 322 (8000), 267 (8800).
IR $\nu_{max}^{CHCl_3}$ $cm^{-1}$: 1785 ($\beta$-lactam) CO), 1720 (ester CO).
NMR ($CDCl_3$)$\delta$ ppm: 1.00 (3H, d, J=7.0Hz,

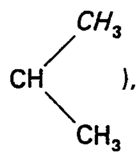 ), 1.03 (3H, d, J=7.0Hz, 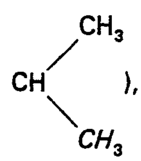 ), 1.85—2.30 (1H, m, 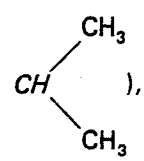 ),

2.60—3.30 (3H, m, C—4$H_2$, C—6*H*), 3.95 (1H, dt, J=9.0Hz, J=3.0Hz, C—5*H*), 5.28 (1H, d, J=14.0 Hz, ArC*H*·H), 5.58 (1H, d, J=14.0Hz, ArCH*H*), 7.41 (2H, dd, J=6.0Hz, J=20Hz, Py*H*), 7.68 (2H, d, J=9.0Hz, ArH), 8.27 (2H, d, J=9.0Hz, ArH), 8.65 (2H, dd, J=6.0Hz, J=2.0Hz, PyH).

The compounds shown in the following Examples 17 to 47 were obtained by method similar to those shown in Examples 9 to 16.

Example 17

*p*-Nitrobenzyl 3-(2pyridylthio)-6-ethyl-7-oxo-1-azabicyclo[3.2.0]hept-2-ene-2-carboxylate:—

$[\alpha]_D^{22}$ + 16.2° (C 1.00, THF).
IR $\nu_{max}^{CHCl_3}$ $cm^{-1}$: 1780 (CO of $\beta$-lactam), 1710 (CO of ester).
UV $\lambda_{max}^{THF}$ nm($\varepsilon$): 325 (16900), 269 (12100).
NMR ($CDCl_3$)$\delta$ ppm: 1.00 (3H, t, J=7Hz, $CH_2CH_3$), 1.60—2.10 (2H, m, $CH_2CH_3$, 2.92 (1H, dd, J=18Hz, J=9Hz, C—4H), 3.22 (1H, dd, J=18Hz, J=9Hz, C—4H), 3.07 (1H, dt, J=7Hz, J=3Hz, C—6H), 3.92 (1H, dt, J=9Hz, J=3Hz, C—5H), 5.26 (1H, dd, J=14Hz, ArC*H*H), 5.53 (1H, dd, J=14Hz, ArCH*H*), 7.40—7.80 (3H, m, PyH), 7,63 (2H, d, J=8Hz, ArH), 8.20 (2H, d, J=8Hz, ArH), 8.58 (1H, dd, J=5Hz, J=2Hz, PyH).
Mass (m/e): 425 ($M^+$), 355 ($M^+$—EtCH=C=O.

Example 18

*p*-Nitrobenzyl 3-2-pyridylthio)-6-isopropyl-7-oxo-1-azabicyclo[3.2.0]hept-2-ene-2-carboxylate:—

UV $\lambda_{max}^{THF}$ nm($\varepsilon$): 325 (16500), 269 (11500).
IR $\nu_{max}^{CHCl_3}$ $cm^{-1}$: 1780 (CO of $\beta$-lactam), 1708 (CO of ester).
NMR ($CDCl_3$)$\delta$ ppm: 1.01 (3H, d, J=7.0Hz,

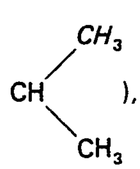 ), 1.04 (3H, d, J=7.0Hz, 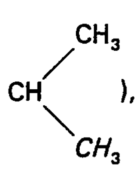 ), 1.88—2.25 (1H, m, 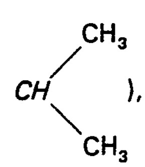 ),

2.70—3.40 (3H, m, C—4H, C—6H), 3.94 (1H, dt, J=9Hz, J=3.0Hz, C—5H), 5.27 (1H, d, J=14.0Hz,

ArC*H*H), 5.56 (1H, d, J=14.0Hz, ArCH*H*), 7.38—7.80 (3H, m, PyH), 7.62 (2H, d, J=8.0Hz, ArH), 8.19 (2H, d, J=8.0Hz, ArH), 8.59 (1H, dd, J=5.0Hz, J=2.0Hz, PyH).

Example 19

*p*-Nitrobenzyl 3-(2-pyrimidinylthio)-6-ethyl-7-oxo-1-azabicyclo[3.2.0]hept-2-ene-2-carboxylate:—

$[\alpha]_D^{22}$: +38.4° (c 9.47, THF).
UV $\lambda_{max}^{THF}$ nm(ε): 322 (9800), 268 (10300).
IR $\nu_{max}^{CHCl_3}$ $cm^{-1}$: 1780 (CO of β-lactam), 1715 (CO of ester).
NMR $(CDCl_3)\delta$ ppm: 1.08 (3H, t, J=7Hz, $CH_2CH_3$), 1.68—2.00 (2H, m, $CH_2CH_3$), 3.00—4.10 (4H, m, C—4H, C—5H, C—6H), 5.28 (1H, d, J=14Hz, ArC*H*H), 5.52 (1H, d, J=14Hz, ArCH*H*), 7.04 (1H, t, J=6Hz, C—5'H), 7.62 (2H, d, J=9Hz, ArH), 8.20 (2H, d, J=9Hz, ArH), 8.56 (2H, d, J=6Hz, C—4'H, C—6'H).
Mass (m/e): 426 ($M^+$), 356 ($M^+$—EtCH=C=O).

Example 20

Benzyl 3-(2-pyrimidinylthio)-6-ethyl-7-oxo-1-azabicyclo[3.2.0]hept-2-ene-2-carboxylate:—

UV $\lambda_{max}^{THF}$ nm(ε): 321 (9900).
IR $\nu_{max}^{CHCl_3}$ $cm^{-1}$: 1780 (CO of β-lactam), 1715 (CO of ester).
NMR $(CDCl_3)\delta$ ppm: 1.04 (3H, t, J=7Hz, $CH_2CH_3$), 1.63—1.95 (2H, m, $CH_2CH_3$), 3.00—4.10 (4H, m, C—4H, C—5H, C—6H), 5.34 (1H, d, J=13.5Hz, ArC*H*H), 5.40 (1H, d, J=13.5Hz, ArCH*H*), 7.10—8.00 (8H, m. PyH, ArH), 8.50—8.64 (1H, distorted d, PyH).
Mass (m/e): 381 ($M^+$), 311 ($M^+$—EtCH=C=O).

Example 21

*p*-Nitrobenzyl 3-(2-pyrimidinylthio)-6-isopropyl-7-oxo-1-azabicyclo[3.2.0]hept 2-2-ene-carboxylate:—

UV $\lambda_{max}^{THF}$ nm(ε): 322 (10100), 267 (10500).
IR $\nu_{max}^{CHCl_3}$ $cm^{-1}$: 1780 (CO of β-lactam), 1715 (CO of ester).
NMR $(CDCl_3)\delta$ ppm: 1.02 (3H, d, J=7.0Hz,

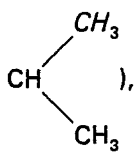 ), 1.05 (3H, d, J=7.0Hz, 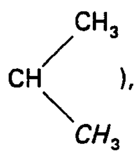 ), 1.90—2.28 (1H, m, 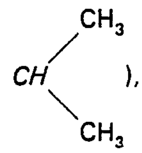 ),

2.90—4.12 (4H, m, C—4H, C—5H, C—6H), 5.27 (1H, d, J=14Hz, ArC*H*H), 5,53 (1H, d, J=14Hz, ArCH*H*), 7.03 (1H, t, J=6.0Hz, C—5'H), 7.60 (2H, d, J=9.0Hz, ArH), 8.20 (2H, d, J=9.0Hz, ArH), 8.54 (2H, d, J=6.0Hz, C—4'H, C—6'H).

Example 22

*p*-Nitrobenzyl 5,6-trans-3-(2-pyrimidinylthio)-6-(1-hydroxyethyl)-7-oxo-1-azabicyclo[3.2.0]hept-2-ene-2-carboxylate:—

UV $\lambda_{max}^{THF}$ nm(ε): 322 (10200), 268 (10700).
IR $\nu_{max}^{CHCl_3}$ $cm^{-1}$: 1780 (CO of β-lactam), 1715 (CO of ester).
NMR $(CDCl_3)\delta$ ppm: 1.37 (3H, d, J=7.0Hz, $CHCH_3$), 2.80—4.40 (5H, m, C—4H, C—5H, C—6H, HOC*H*$CH_3$), 5.29 (1H, d, J=14.0Hz, ArCH*H*), 5.50 (1H, d, J=14.0Hz, ArCH*H*), 7.08 (1H, t, J=6.0Hz, C—5'H),, 7.64 (2H, d, J=9.0Hz, ArH), 8.20 (2H, d, J=9.0Hz, ArH), 8.55 (2H, d, J=6.0Hz, C—4'H, C—6'H).

Example 23

*p*-Nitrobenzyl, 5,6-cis-3-(2-pyrimidinylthio)-6-(1-hydroxyethyl)-7-oxo-1-azabicyclo[3.2.0]hept-2-ene-2-carboxylate:—

UV $\lambda_{max}^{THF}$ nm(ε): 322 (10800), 268 (11200)
IR $\nu_{max}^{CHCl_3}$ $cm^{-1}$: 1780 (CO of β-lactam), 1710 (CO of ester).
NMR $(CDCl_3)\delta$ ppm: 1.38 (3H, d, J=7.0Hz, CH—$CH_3$), 2.70—4.45 (5H, m, C—4H, C—5H, C—6H, HOC*H*$CH_3$), 5.25 (1H, d, J=14.0Hz, ArCH*H*), 5.50 (1H, d, J=14.0Hz, ArCH*H*), 7.06 (1H, t, J=6.0Hz, C—5'H), 7.62 (2H, d, J=9.0Hz, ArH), 8.18 (2H, d, J=9.0Hz, ArH), 8.52 (2H, d, J=6.0Hz, C—4'H, C—6'H).

Example 24

*p*-Nitrobenzyl 3-(phenylthio)-6-ethyl-7-oxo-1-azabicyclo[3.2.0]hept-2-ene-2-carboxylate:—

$[\alpha]_D^{22}$: +3.0° (c 1.00, THF).

UV $\lambda_{max}^{THF}$ nm(ε): 320 (11000), 271 (9100).

IR $\nu_{max}^{CHCl_3}$ $cm^{-1}$: 1780 (CO of β-lactam), 1710 (CO of ester).

NMR ($CDCl_3$)δ ppm: 1.00 (3H, t, J=7.5Hz, $CH_2CH_3$), 1.6—1.90 (2H, m, $CH_2CH_3$), 2.66 (2H, d, J=9Hz, C—4H), 3.02 (1H, dt, J=9Hz, J=3Hz, C—6H), 3.82 (1H, dt, J=9Hz, J=3Hz, C—5H), 5.24, 5.54 (2H, each d, J=14Hz, ArC*H*H). 7.28—7.60 (5H, m, ArH), 7.68 (2H, d, J=9Hz, ArH), 8.24 (2H, d, J=9Hz, ArH).

Mass (m/e): 424 ($M^+$), 354 ($M^+$—EtCH=C=O).

Example 25

*p*-Nitrobenzyl 3-(*p*-nitrophenylthio)-6-ethyl-7-oxo-1-azabicyclo[3.2.0]hept-2-ene-2-carboxylate:—

$[a]_D^{22}$: +32.2° (c 1.00, THF).

UV $\lambda_{max}^{THF}$ nm(ε): 266 (17800), 310sh (12400).

IR $\nu_{max}^{CHCl_3}$ $cm^{-1}$: 1772 (CO of β-lactam), 1700 (CO of ester).

NMR ($CDCl_3$) ppm: 1.02 (3H, t, J=7.5Hz, $CH_2CH_3$), 1.60—2.00 (2H, m, $CH_2CH_3$), 2.78 (2H, d, J=9.0Hz, C—4H), 3.08 (1H, m, C—6H), 3.92 (1H, dt, J=9Hz, J=3Hz, C—5H), 5.24 (1H, d, J=14Hz, ArC*H*H), 5.50 (1H, d, J=14Hz, ArCH*H*), 7.64 (4H, d, J=9Hz, ArH), 8.18 (4H, d, J=9Hz, ArH).

Mass (m/e): 469 ($M^+$), 399 ($M^+$—Et—CH=C=O).

Example 26

*p*-Nitrobenzyl 3-phenylthio-6-isopropyl-7-oxo-1-azabicyclo[3.2.0]hept-2-ene-2-carboxylate (PS—6.*p*-nitrobenzyl ester):—

UV $\lambda_{max}^{THF}$ nm(ε): 320 (11500), 270 (9200).

IR $\nu_{max}^{CHCl_3}$ $cm^{-1}$: 1780 (CO of β-lactam), 1710 (CO of ester).

NMR ($CDCl_3$) δ ppm: 0.97 (3H, d, J=7.0Hz,

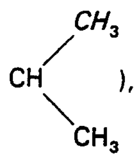 ), 1.00 (3H, d, J=7.0Hz, 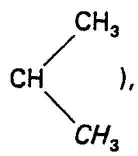 ), 1.85—2.25 (1H, m, 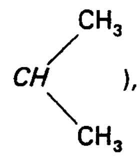 ),

2.65 (2H, d, J=9.0Hz, C—4H), 3.04 (1H, dd, J=3.0Hz, J=9.0Hz, C—6H), 3.83 (1H, dt, J=9.0Hz, J=3.0Hz, C—5H), 5.25 (1H, d, J=14.0Hz, ArC*H*H), 5.55 (1H, d, J=14.0Hz, ArCH*H*), 7.25—7.72 (5H, m, Ar*H*), 7.67 (2H, d, J=9.0Hz, ArH), 8.25 (2H, d, J=9.0Hz, ArH).

Example 27

*p*-Nitrobenzyl 5,6-trans-3-phenylthio-6-(1-hydroxyethyl)-7-oxo-1-azabicyclo[3.2.0]hept-2-ene-2-carboxylate:—

UV $\lambda_{max}^{THF}$ nm(ε): 320 (11800), 270 (9800).

IR $\nu_{max}^{CHCl_3}$ $cm^{-1}$: 170 (CO of β-lactam), 1700 (CO of ester).

NMR ($CDCl_3$) δ ppm: 1.31 (3H, d, J=7.0Hz, —CHOH—$CH_3$), 2.68 (2H, d, J=9.0Hz, C—4H), 3.18 (1H, dd, J=6.0Hz, J=3.0Hz, C—6H), 4.02 (1H, dt, J=9.5Hz, J=3.0Hz, C—5H), 4.12 (1H, m, —*C*HOH—$CH_3$), 5.27 (1H, d, J=14.0Hz, —C*H*H—Ar), 5.56 (1H, d, J=14.0Hz —CH*H*—Ar), 7.10—7.60 (5H, m, Ar*H*), 7.67 (2H, d, J=9.0Hz, ArH), 8.24 (2H, d, J=9.0Hz, ArH).

Example 28

*p*-Nitrobenzyl 5,6-cis-3-phenylthio-6-(1-hydroxyethyl)-7-oxo-1-azabicyclo[3.2.0]hept-2-ene-2-carboxylate:—

UV $\lambda_{max}^{THF}$ nm(ε): 320 (11500), 270 (9500).

IR $\nu_{max}^{CHCl_3}$ $cm^{-1}$: 1775 (CO of β-lactam), 1710 (CO of ester).

NMR ($CDCl_3$) δ ppm: 1.36 (3H, d, J=7.0Hz, —CHOH—$CH_3$), 2.55 (1H, dd, J=19.0Hz, J=9.5Hz, C—4H), 3.13 (1H, dd, J=19.0Hz, J=9.5Hz, C—4H), 3.52 (1H, dd, J=9.0Hz, J=5.5Hz, C—6H), 3.85—4.45 (2H, m, C—5H, HOC*H*$CH_3$), 5.28 (1H, d, J=14.0Hz, —C*H*H—Ar), 5.56 (1H, d, J=14.0Hz, —CH*H*—Ar), 7.32—7.80 (7H, m, ArH), 8.22 (2H, d, J=9.0Hz, ArH).

Example 29

*p*-Nitrobenzyl 3-cyclohexylthio-6-ethyl-7-oxo-1-azabicyclo[3.2.0]hept-2-ene-2-carboxylate:—

Rf: 0.43 (benzene:acetone = 20:1).

$[a]_D^{22}$: +42.6° (c 1.00, THF).

UV $\lambda_{max}^{THF}$ nm(ε): 325 (15600), 270 (12800).

IR $\nu_{max}^{CHCl_3}$ $cm^{-1}$: 1770 (CO of β-lactam), 1695 (CO of ester).

NMR ($CDCl_3$) δ ppm: 1.08 (3H, t, J=7.5Hz, $CH_2$*$CH_3$*), 1.2—2.1 (12H, m, *$CH_2$*$CH_3$, cyclohexyl $CH_2$), 2.8—3.2 (4H, m, C—4H, C—6H, SCH), 3.94 (1H, dt, J=9.0Hz, J=3.0Hz, C—4H), 5.18 (1H, d, J=14Hz, ArC*H*H), 5.50 (1H, d, J=14Hz, ArCH*H*), 7.62 (2H, d, J=9.0Hz, ArH), 8.18 (2H, d, J=9.0Hz, ArH).

Mass (m/e): 430 ($M^+$), 360 ($M^+$—EtCH=C=O).

## Example 30

*p*-Nitrobenzyl 3-cyclohexylthio-6-isopropyl-7-oxo-1-azabicyclo[3.2.0]hept-2-ene-2-carboxylate:—

UV $\lambda_{max}^{THF}$ nm (ε): 325 (15000), 270 (12100).

IR $\nu_{max}^{CHCl_3}$ $cm^{-1}$: 1770 (CO of β-lactam), 1695 (ester).

NMR ($CDCl_3$) δ ppm: 1.02 (3H, d, J=7.0Hz, 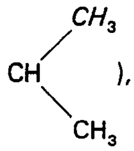 ), 1.05 (3H, d, J=7.0Hz, 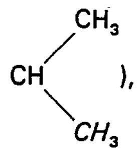 ), 1.18—2.30 (11H, m, 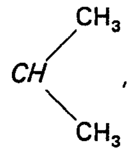 , cyclohexyl $CH_2$), 2.78—3.30 (4H, m, C—4H, C—6H, SCH), 3.96 (1H, dt, J=9.0Hz, J=3.0Hz, C—4H), 5.20 (1H, d, J=14.0Hz, ArC*H*H), 5.48 (1H, d, J=14.0Hz, ArCH*H*), 7.63 (2H, d, J=9.0Hz, ArH), 8.20 (2H, d, J=9.0Hz, ArH).

## Example 31

*p*-Nitrobenzyl 5,6-trans-3-cyclohexylthio-6-(2-hydroxyethyl)-7-oxo-1-azabicyclo[3.2.0]hept-2-ene-2-carboxylate:—

UV $\lambda_{max}^{THF}$ nm(ε): 325 (14500), 270 (11600).

IR $\nu_{max}^{CHCl_3}$ $cm^{-1}$: 1770 (β-lactam), 1695 (ester).

NMR ($CDCl_3$) δ ppm: 1.20—2.00 (13H, m, CH—*$CH_3$*, cyclohexyl $CH_2$), 2.75—3.70 (4H, m, SCH, C—4H, C—6H), 3.80—4.38 (2H, m, C—5H, HOC*H*$CH_3$), 5.20 (1H, d, J=14.0Hz, ArC*H*H), 5.50 (1H, d, J=14.0Hz, ArCH*H*), 7.64 (2H, d, J=9.0Hz, ArH), 8.18 (2H, d, J=9.0Hz, ArH).

## Example 32

2p-Nitrobenzyl 5,6-cis-3-cyclohexylthio-6-(1-hydroxyethyl)-7-oxo-1-azabicyclo[3.2.0]hept-2-ene-2-carboxylate:—

UV $\lambda_{max}^{THF}$ nm(ε): 325 (14700), 270 (11700).

IR $\nu_{max}^{CHCl_3}$ $cm^{-1}$: 1770 (CO of β-lactam), 1695 (CO of ester).

NMR ($CDCl_3$) δ ppm: 1.20—2.00 (13H, m, CH—*$CH_3$*, cyclohexyl $CH_2$), 2.70—4.45 (6H, m, C—4H, C—5H, C—6H, SCH, HOC*H*$CH_3$), 5.20 (1H, d, J=14.0Hz, ArC*H*H), 5.46 (1H, d, J=14.0Hz, ArCH*H*), 7.60 (2H, d, J=9.0Hz, ArH), 8.17 (2H, d, J=9.0Hz, ArH).

## Example 33

*p*-Nitrobenzyl 5,6-trans-3-*n*-butylthio-6-(1-hydroxyethyl)-7-oxo-1-azabicyclo[3.2.0]hept-2-ene-2-carboxylate:—

UV $\lambda_{max}^{THF}$ nm(ε): 322 (11000), 270 (9800).

IR $\nu_{max}^{CHCl_3}$ $cm^{-1}$: 1770 (CO of β-lactam), 1698 (CO of ester).

NMR ($CDCl_3$) δ ppm: 0.93 (3H, t, J=7.0Hz, $CH_2CH_2CH_2$*$CH_3$*), 1.20—2.00 (7H, m, 2 × $CH_2$, CH—*$CH_3$*), 2.70—3.60 (5H, m, C—4H, $SCH_2$, C—6H), 3.80—4.40 (2H, m, C—5H, HOC*H*$CH_3$), 5.20 (1H, d, J=14.0Hz, ArC*H*H), 5.50 (1H, d, J=14.0Hz, ArCH*H*), 7.63 (2H, d, J=9.0Hz, ArH), 8.20 (2H, d, J=9.0Hz, ArH).

## Example 34

*p*-Nitrobenzyl 5,6-cis-3-*n*-butylthio-6-(1-hydroxethyl)-7-oxo-1-azabicyclo[3.2.0]hept-2-ene-2-carboxylate:—

UV $\lambda_{max}^{THF}$ nm(ε): 322 (11800), 270 (10500).

IR $\nu_{max}^{CHCl_3}$ $cm^{-1}$: 1770 (CO of β-lactam), 1695 (CO of ester).

NMR ($CDCl_3$) δ ppm: 0.92 (3H, t, J=7.0Hz, $CH_2CH_2CH_2$*$CH_3$*), 1.20—2.00 (7H, m, 2 × $CH_2$, CH*$CH_3$*), 2.60—3.65 (5H, m, C—4H, C—6H, $SCH_2$), 3.80—4.40 (2H, m, C—5H, HOC*H*$CH_3$), 5.18 (1H, d, J=14.0Hz, ArC*H*H), 5.48 (1H, d, J=14.0Hz, ArCH*H*), 7.65 (2H, d, J=9.0Hz ArH), 8.20 (2H, d, J=9.0Hz, ArH).

## Example 35

*p*-Nitrobenzyl 3-(2-hydroxyethylthio)-6-ethyl-7-oxo-1-azabicyclo[3.2.0]hept-2-ene-2-carboxylate:—

IR $\nu_{max}^{CHCl_3}$ $cm^{-1}$: 3450 (OH), 1790 (CO of β-lactam), 1700 (CO of ester).

UV $\lambda_{max}^{THF}$ nm(ε): 320 (10400), 270 (10600).

$[a]_D^{22}$: +33.2° (c 1.0, THF).

NMR ($CDCl_3$) δ ppm: 1.04 (3H, t, J=7.5Hz, $CH_2$*$CH_3$*), 1.75—2.05 (2H, m, *$CH_2$*$CH_3$), 2.85—3.30 (5H, m, 2X—*$CH_2$*, CH), 3.70—4.10 (3H, m, C—5H, —*$CH_2$*OH), 5.18 (1H, d, J=14Hz, Ar*CH*H), 5.49 (1H, d, J=14Hz, ArCH*H*), 7.80 (2H, d, J=9.0Hz, ArH), 8.16 (2H, d, J=9.0Hz, ArH).

Mass (m/e): 392 ($M^+$), 322 ($M^+$—EtCH=C=O), 304 ($M^+$—EtCH=C=O—$H_2O$).

Example 36

*p*-Nitrobenzyl 3-(2-hydroxyethylthio)-6-isopropyl-7-oxo-1-azabicyclo[3.2.0]hept-2-ene-2-carboxylate:— boxylate:—

UV $\lambda_{max}^{THF}$ nm(ε): 320 (10500), 270 (10800).

IR $\nu_{max}^{CHCl_3}$ $cm^{-1}$: 1780 (CO of β-lactam), 1705 (CO of ester).

NMR ($CDCl_3$) δ ppm: 1.02 (3H, d, J=7.0Hz,

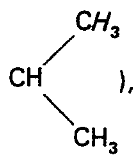
), 1.05 (3H, d, J=7.0Hz, 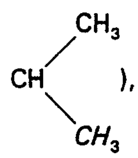
), 1.90—2.25 (1H, m, 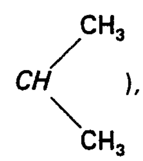
),

2.80—3.30 (5H, m, C—4H), S—$CH_2$, C—6*H*), 3.65—4.10 (3H, m, C—5H, $CH_2$OH), 5.20 (1H, d, J=14.0Hz, Ar*CH*H), 5.50 (1H, d, J=14.0Hz, ArCH*H*), 7.78 (2H, d, J=9.0Hz, ArH), 8.14 (2H, d, J=9.0Hz, ArH).

Example 37

*p*-Nitrobenzyl 5,6-trans-3-(2-hydroxyethylthio)-6-(1-hydroxyethyl)-7-oxo-1-azabicyclo[3.2.0]-hept-2-ene-2-carboxylate:—

UV $\lambda_{max}^{THF}$ nm(ε): 320 (11000), 270 (11200).

IR $\nu_{max}^{CHCl_3}$ $cm^{-1}$: 1780 (CO of β-lactam), 1705 (CO of ester).

NMR ($CDCl_3$) δ ppm: 1.38 (3H, d, J=7.0Hz, CH—*$CH_3$*), 2.80—4.45 (8H, m, C—4H, C—5H, C—6H, 2 × $CH_2$, HO—CH—$CH_3$), 5.18 (1H, d, J=14.0Hz, Ar*CH*H), 5.45 (1H, d, J=14.0Hz, ArCH*H*), 7.58 (2H, d, J=9.0Hz, ArH), 8.15 (2H, d, J=9.0Hz, ArH).

Example 38

*p*-Nitrobenzyl 5,6-cis-3-(2-hydroxyethylthio)-6-(1-hydroxyethyl)-7-oxo-1-azabicyclo[3.2.0]hept-2-ene-2-carboxylate:—

UV $\lambda_{max}^{THF}$ nm(ε): 320 (11700), 270 (11800).

IR $\nu_{max}^{CHCl_3}$ $cm^{-1}$: 1778 (CO of β-lactam), 1705 (CO of ester).

NMR ($CDCl_3$) δ ppm: 1.40 (3H, d, J=7.0Hz, CH—$CH_3$), 2.70—4.45 (9H, m, C—4H, C—5H, C—6H), 2 × $CH_2$, HO*CH*$CH_3$), 5.20 (1H, d, J=14.0Hz, ArCH*H*), 5.48 (1H, d, J=14.0Hz, ArCH*H*), 7.60 (2H, d, J=9.0Hz, ArH), 8.18 (2H, d, J=9.0Hz, ArH).

Example 39

*p*-Nitrobenzyl 3-(2-ethoxyethylthio)-6-ethyl-7-oxo-1-azabicyclo[3.2.0]hept-5-ene-2-carboxylate:—

IR $\nu_{max}^{CHCl_3}$ $cm^{-1}$: 1775 (CO of β-lactam), 1700 (CO of ester).

UV $\lambda_{max}^{THF}$ nm(ε): 320 (14100), 270 (12400).

$[\alpha]_D^{22}$: +45.7° (c 1.0, THF).

NMR ($CDCl_3$) δ ppm: 1.06 (3H, t, J=7.0Hz, $CH_2$*$CH_3$*), 1.18 (3H, t, J=7.0Hz, $CH_2$*$CH_3$*), 1.70—2.00 (2H, m, *$CH_2$*$CH_3$), 2.90—3.70 (9H, m, C—6H, C—4H, 3X—$CH_2$), 3.91 (1H, dt, J=9Hz, J=3Hz, C—5H), 5.18 (1H, d, J=13Hz, Ar*CH*H), 5.48 (1H, d, J=13Hz, ArCH*H*), 7.60 (2H, d, J=9Hz, ArH), 8.15 (2H, d, J=9Hz, ArH).

Mass (m/e): 420 ($M^+$), 350 ($M^+$—EtCH=C=O), 304 ($M^+$—EtCH=C=EtOH).

Example 40

*p*-Nitrobenzyl 3-(2-ethoxyethylthio)-6-isopropyl-7-oxo-1-azabicyclo[3.2.0]hept-2-ene-2-carboxylate:—

UV $\lambda_{max}^{THF}$ nm(ε): 320 (13600), 269 (12000).

IR $\nu_{max}^{CHCl_3}$ $cm^{-1}$: 1775 (CO of β-lactam), 1700 (CO of ester).

NMR ($CDCl_3$) δ ppm: 0.90—1.25 (9H, m,

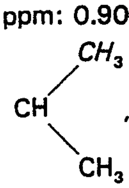
, $CH_2$*$CH_3$*), 1.90—2.30 (1H, m, 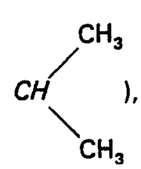
),

2.80—3.75 (9H, m, C—4H, C—6H, $CH_2$ × 3), 3.95 (1H, dt, J=9.0Hz, J=3.0Hz, C—5H), 5.20 (1H, d, J=14Hz, ArCH*H*), 5.48 (1H, d, J=14.0Hz, ArCH*H*), 7.62 (2H, d, J=9.0 Hz, ArH), 8.18 (2H, d, J=9.0Hz, ArH).

Example 41

*p*-Nitrobenzyl 5,6-trans-3-(2-ethoxyethylthio)-6-(1-hydroxethyl)-7-oxo-1-azabicyclo[3.2.0]hept-2-ene-2-carboxylate:—

UV $\lambda_{max}^{THF}$ nm($\varepsilon$): 320 (13500), 270 (11800).

IR $\nu_{max}^{CHCl_3}$ $cm^{-1}$: 1775 (CO of $\beta$-lactam), 1700 (CO of ester).

NMR ($CDCl_3$) $\delta$ ppm: 1.18 (3H, t, J=7.0Hz, $CH_2CH_3$), 1.38 (3H, d, J=7.0Hz, $CHCH_3$), 2.80—4.40 (11H, m, C—4H, C—5H, C—6H, HO*CH*$CH_3$, 3 × $CH_2$), 5.18 (1H, d, J=14.0Hz, Ar*CH*H), 5.50 (1H, d, J=14.0Hz, ArCH*H*), 7.64 (2H, d, J=9.0Hz, ArH), 8.20 (2H, d, J=9.0Hz, ArH).

Example 42

p-Nitrobenzyl 5,6-cis-3-(2-ethoxyethylthio)-6-(1-hydroxyethyl)-7-oxo-1-azabicyclo[3.2.0]hept-2-ene-2-carboxylate:—

UV $\lambda_{max}^{THF}$ nm($\varepsilon$): 320 (13000), 270 (11200).

IR $\nu_{max}^{CHCl_3}$ $cm^{-1}$: 1775 ($\beta$-lactam), 1705 (ester).

NMR ($CDCl_3$) $\delta$ ppm: 1.18 (3H, t, J=7.0Hz, $CH_2CH_3$), 1.38 (3H, d, J=7.0Hz, $CHCH_3$), 2.60—4.45 (11H, m, C—4H, C—5H, C—6H, 3 × $CH_2$, HO*CH*$CH_3$), 5.18 (1H, d, J=14.0Hz, ArCH*H*), 5.49 (1H, d, J=14.0Hz, ArCH*H*), 7.60 (2H, d, J=9.0Hz, ArH), 8.18 (2H, d, J=9.0Hz, ArH).

Example 43

*p*-Nitrobenzyl 3-methylthio-6-ethyl-7-oxo-1-azabicyclo[3.2.0]hept-2-ene-2-carboxylate:—

IR $\nu_{max}^{CHCl_3}$ $cm^{-1}$: 1778 (CO of $\beta$-lactam), 1702 (CO of ester), 1525, 1350 ($NO_2$).

Example 44

*p*-Nitrobenzyl 3-(2-acetoxyethylthio)-6-ethyl-7-oxo-1-azabicyclo[3.2.0]hept-2-ene-2-carboxylate:—

UV $\lambda_{max}^{THF}$ nm($\varepsilon$): 317 (9600), 268 (9800).

IR $\nu_{max}^{CHCl_3}$ $cm^{-1}$: 1780 (CO of $\beta$-lactam), 1740 (O—*CO*Me), 1700 (CO of ester).

NMR ($CDCl_3$) $\delta$ ppm: 1.08 (3H, t, J=7Hz, $CH_2CH_3$), 1.70—2.20 (4H, m, $CH_2CH_3$), 2.00 (3H, s, $COCH_3$), 2.80—3.50 (5H, m, 2X—$CH_2$CH), 3.98 (1H, dt, J=9.5Hz, J=3Hz, C—5H), 4.20 (2H, t, J=7.5Hz, $CH_2OCO$—), 5.20 (1H, d, J=14Hz, Ar*CH*H), 5.50 (1H, d, J=14Hz, ArCH*H*), 7.59 (2H, d, J=9.0Hz, ArH), 8.15 (2H, d, J=9.0Hz, ArH).

Example 45

*p*-Nitrobenzyl 3(2-acetoxyethylthio)-6-isopropyl-7-oxo-1-azabicyclo[3.2.0]hept-2-ene-2-carboxylate:—

UV $\lambda_{max}^{THF}$ nm($\varepsilon$): 317 (9800), 268 (10200).

IR $\nu_{max}^{CHCl_3}$ $cm^{-1}$: 1780 (CO of $\beta$-lactam), 1740 (OAc), 1700 (CO of ester).

NMR ($CDCl_3$) $\delta$ ppm: 1.03 (3H, d, J=7.0Hz,

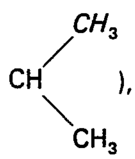 ), 1.06 (3H, d, J=7.0Hz, 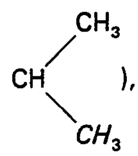 ), 1.90—2.30 (1H, m, 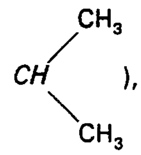 ),

2.07 (3H, S, $COCH_3$), 2.70—3.50 (5H, m, $SCH_2$, C—4H, C—6H), 4.00 (1H, dt, J=9.5Hz, J=3.0Hz, C—5H), 4.20 (2H, t, J=7.5Hz, $CH_2OCO$), 5.18 (1H, d, J=14.0Hz, Ar*CH*H), 5.48 (1H, d, J=14.0Hz, ArCH*H*), 7.58 (2H, d, J=9.0Hz, ArH), 8.14 (2H, d, J=9.0Hz, ArH).

Example 46

*p*-Nitrobenzyl 5,6-trans-3-(2-acetoxyethylthio)-6-(1-hydroxyethyl)-7-oxo-1-azabicyclo[3.2.0]-hept-2-ene-2-carboxylate:—

UV $\lambda_{max}^{THF}$ nm($\varepsilon$): 317 (10200), 268 (10800).

IR $\nu_{max}^{CHCl_3}$ $cm^{-1}$: 17800 (CO of $\beta$-lactam), 1740 (OAc), 1700 (CO of ester).

NMR ($CDCl_3$) $\delta$ ppm: 1.37 (3H, d, J=7.0Hz, $CHCH_3$), 2.05 (3H, S, $COCH_3$), 2.80—4.45 (9H, m, C—4H, C—5H, C—6H, HOC*H*$CH_3$, 2 × $CH_2$), 5.20 (1H, d, J=14.0Hz, Ar*CH*H), 5.51 (1H, d, J=14.0Hz, ArCH*H*), 7.64 (2H, d, J=9.0Hz, ArH), 8.20 (2H, d, J=9.0Hz, ArH).

Example 47

*p*-Nitrobenzyl 5,6-cis-3-(2-acetoxyethylthio)-6-(1-hydroxyethyl)-7-oxo-1-azabicyclo[3.2.0]hept-2-ene-2-carboxylate:—

UV $\lambda_{max}^{THF}$ nm($\varepsilon$): 317 (11100), 268 (11500).

IR $\nu_{max}^{CHCl_3}$ $cm^{-1}$: 1780 (CO of $\beta$-lactam), 1740 (OAc), 1700 (CO of ester).

NMR ($CDCl_3$) $\delta$ ppm: 1.38 (3H, d, J=7.0Hz, $CHCH_3$), 2.06 (3H, S, $COCH_3$), 2.65—4.50 (9H, m, C—4H, C—5H, C—6H, 2 × $CH_2$, HO*CH*$CH_3$), 5.20 (1H, d, J=14.0Hz, ArCH*H*), 5.48 (1H, d, J=14.0Hz, ArCH*H*), 7.62 (2H, d, J—9.0Hz, ArH), 8.18 (2H, d, J=9.0Hz, ArH).

Example 48

Production of 3-(4-pyridylthio)-6-ethyl-7-oxo-1-azabicyclo[3.2.0]hept 2-ene-2-carboxylic acid:

54 mg of benzyl 3-(4-pyridylthio)-6-ethyl-7-oxo-1-azabicyclo[2.3.0]hept-2-ene-2-carboxylate obtained in Example 15 was dissolved in a mixture of 2 ml of dioxane and 2 ml of a 0.1M phosphate buffer (pH 7.0). Then, 54 mg of platinum oxide was added, and the reaction was performed at room temperature for 4 hours in a Paar reducing apparatus (hydrogen pressure 4 kg/cm$^2$). The catalyst was removed by filtration. The filtrate and the wash liquid were combined and concentrated under reduced pressure to about 2/3 of the original amount. To the resulting solution was added 2.5 g of sodium chloride, and water was added to make the total amount 50 ml. The solution was charged on a column of Diaion HP—20AG (1.1 x 20 cm), and eluted with 300 ml in total of an aqueous solution of acetone having a concentration gradually varying from 0 to 30% by a concentration gradient method. The eluate was fractionated into 5 g fractions. The fractions were screened by high-velocity liquid chromatography, and fractions having an ultraviolet absorption at $\lambda_{max}$ 303 nm were collected, and lyophilized to afford 20.7 mg (yield 56.8%) of the captioned compound.

UV $\lambda_{max}^{H_2O*}$ nm($\varepsilon$): 303 (8500).

*0.1M phosphate buffer (pH 7.0)

NMR ($D_2O$) $\delta$ ppm: 1.00 (3H, t, J=7Hz, $CH_2CH_3$), 1.64—2.00 (2H, m, $CH_2CH_3$), 2.92 (2H, d, J=9Hz, C—4H), 3.34 (1H, dt, J=7.5Hz, J=3Hz, C—6H), 4.06 (1H, dt, J=9Hz, J=3Hz, C—5H), 7.50 (2H, dd, Jx6Hz, J=2Hz, PyH), 8.30—8.60 (2H, m, PyH).

By the same reductive de-esterification method as in Example 48, the corresponding de-esterification products shown in Examples 49 to 51 were obtained from the ester compounds obtained in Examples 14 and 16.

Example 49

3-(4-Pyridylthio)-6-isopropyl-7-oxo-1-azabicyclo[3.2.0]hept-2-ene-2-carboxylic acid:—

UV $\lambda_{max}^{H_2O}$ nm($\varepsilon$): 303 (8200).

*0.1M phosphate buffer (pH 7.0)

NMR ($D_2O$) $\delta$ ppm: 1.00 (3H, d, J=7.0Hz,

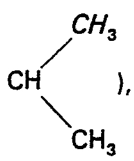 ), 1.03 (3H, d, J=7.0Hz, 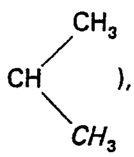 ), 1.90—2.25 (1H, m, 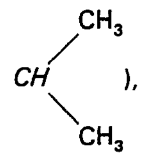 ),

2.70—3.50 (3H, m, C—4H, C—6H), 4.08 (1H, dt, J=9.0Hz, J=3.0Hz, C—5H), 7.51 (2H, dd, J=6.0Hz, J=2.5Hz, PyH), 8.46 (2H, m, PyH).

Example 50

5,6-trans-3-(4-Pyridylthio)-6-(1-hydroxyethyl)-7-oxo-1-azabicyclo[3.2.0]hept-2-ene-2-carboxylic acid:—

UV $\lambda_{max}^{H_2O*}$ nm($\varepsilon$): 303 (8800).

*0.1M phosphate buffer (pH 7.0)

NMR ($D_2O$) $\delta$ ppm: 1.36 (3H, J=7.0Hz, $CHCH_3$), 2.75—3.65 (3H, m, C—4H, C—6H), 3.80—4.40 (2H, m, C—5H, $HOCHCH_3$), 7.50 (2H, dd, J=6.0Hz, J=2.5Hz, PyH), 8.48 (2H, m, PyH).

Example 51

5,6-cis-3-(4-Pyridylthio)-6-(1-hydroxethyl)-7-oxo-1-azabicyclo[3.2.0]hept-2-ene-2-carboxylic acid:—

UV $\lambda_{max}^{H_2O*}$ nm($\varepsilon$): 303 (8100).

*0.1M phosphate buffer (pH 7.0)

NMR ($D_2O$) $\delta$ ppm: 1.38 (3H, d, J=7.0Hz, $CHCH_3$), 2.60—3.80 (3H, m, C—4H, C—6H), 3.90—4.45 (2H, m, C—5H, $HOCHCH_3$), 7.52 (2H, dd, J=6.0Hz, J=2.0Hz, PyH), 8.46 (2H, m, PyH).

Example 52

Production of 3-(2-pyrimidinylthio)-6-ethyl-7-oxo-1-azabicyclo[3.2.0]hept-2-ene-2-carboxylic acid:—

63.4 mg of benzyl 3-(2-pyrimidinylthio)-6-ethyl-7-oxo-1-azabicyclo[3.2.0]hept-2-ene-2-carboxylate obtained in Example 20 was dissolved in a mixture of 3.2 ml of dioxane and 1.2 ml of a 0.1M phosphate buffer (pH 6.86). Then, 70 mg of platinum oxide was added, and the reaction was performed at room temperature for 4 hours in a Paar reducing apparatus (hydrogen pressure 4 kg/cm$^2$). The catalyst was removed by filtration. The filtrate and the wash liquid were combined, and after adjusting the pH of the mixture to 7.2, concentrated under reduced pressure to about 2/3 of the initial amount. Sodium chloride (2 g) was added to the concentrated solution, and water was added to adjust the total amount to 70 ml. The resulting solution was applied to a column of Diaion HP—20AG (1.1 x 20 cm),

and eluted with 300 ml in total of an aqueous solution of acetone with the concentration of acetone varying gradually from 0 to 50% (v/v) by a concentration gradient method. The fractions were screened by high-speed liquid chromatography, and fractions having an ultraviolet absorption at $\lambda_{max}$ 300 nm were collected and lyophilized to afford 16.8 mg of the desired product (yield 39%).

UV $\lambda_{max}^{H_2O*}$ nm($\varepsilon$): 297 (6400).
*0.1M phosphate buffer (pH 7.0)
NMR ($D_2$) $\delta$ ppm: 1.02 (3H, t, J=7Hz, $CH_2CH_3$), 1.68—2.04 (2H, m, $CH_2CH_3$), 2.84—3.52 (3H, m, C—4H, C6H), 4.14 (1H, dt, J=9Hz, J=3Hz, C—5H), 7.35 (1H, t, J=6Hz, C—5′H), 8.64 (2H, d, J=6Hz, C—4′H, C—6′H).

As described in detail in Examples 48 and 52, other carboxylic acid esters obtained by this invention (for example, the compounds obtained in Examples 10, 17, 24, 29 and 21 to 23) can also be de-esterified by treating them in the same way as in Examples 48 or 52. The following Examples illustrate typical examples of the resulting compounds.

Example 53

3-(2-Pyrimidinylthio)-6-isopropyl-7-oxo-1-azabicyclo[3.2.0]hept-2-ene-2-carboxylic acid:—

UV $\lambda_{max}^{H_2O*}$ nm($\varepsilon$): 297 (6600).
*0.1M phosphate buffer (pH 7.0)
NMR ($D_2O$) $\delta$ ppm: 1.01 (3H, d, J=7.0Hz,

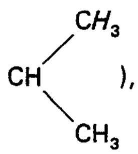 ), 1.04 (3H, d, J=7.0Hz, 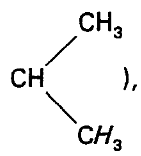 ), 1.90—2.30 (1H, m, 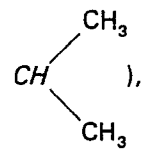 ),

2.80—3.75 (3H, m, C—4H, C—6H), 4.15 (1H, dt, J=9.0Hz, J=3.0Hz, C—5H), 7.38 (1H, t, J=6.0Hz, C—5′H), 8.63 (2H, d, J=6.0Hz, C—4′H, C—6′H).

Example 54

5,6-trans-3-(2-Pyrimidinylthio)-6-(1-hydroxyethyl)-7-oxo-1-azabicyclo[3.2.0]hept-2-ene-2-carboxylic acid:—

UV $\lambda_{max}^{H_2O*}$ nm($\varepsilon$): 296 (6500).
*0.1M phosphate buffer (pH 7.0)
NMR ($D_2O$) $\delta$ ppm: 1.38 (3H, d, J=7.0Hz, $CHCH_3$), 2.80—3.80 (3H, m, C—4H, C—6H), 3.95—4.50 (2H, m, C—5H, $HOCHCH_3$), 7.37 (1H, t, J=6.0Hz, C—5′H), 8.65 (2H, d, J=6.0Hz, C—4′H, C—6′H).

Example 55

5,6-cis-3-(2-Pyrimidinylthio)-6-(1-hydroxyethyl)-7-oxo-1-azabicyclo[3.2.0]hept-2-ene-2-carboxylic acid:—

UV $\lambda_{max}^{H_2O*}$nm ($\varepsilon$): 2.97 (6800).
*0.1M phosphate buffer (pH 7.0)
NMR ($D_2O$) $\delta$ ppm: 1.38 (3H, J=7.0Hz, $CHCH_3$), 2.60—3.85 (3H, m, C—4H, C—6H), 3.95—4.50 (2H, m, C—5H, $HOCHCH_3$), 7.36 (1H, t, J=6.0Hz, C—5′H), 8.63 (2H, d, J=6.0Hz, C—4′H, C—6′H).

Example 56

3-(2-Pyridylthio)-6-ethyl-7-oxo-1-azabicyclo[3.2.0]hept-2-ene-2-carboxylic acid:—

UV $\lambda_{max}^{H_2O}$ nm($\varepsilon$): 305 (12900).
NMR ($CDCl_3$) $\delta$ ppm: 0.99 (3H, t, J=7.5Hz, $CH_2CH_3$), 1.80 (2H, dq, J=7.5Hz, $CH_2CH_3$), 2.82 (2H, d, J=9.0Hz, C—4H), 3.26 (1H, dt, J=7.5Hz, J=3Hz, (C—6H), 3.98 (1H, dt, J=9.0Hz, J=3Hz, C—5H), .41, 7.62, 7.83 and 8.45 (1H, each m, ArH).

Example 57

Sodium 3-(phenylthio)-6-ethyl-7-oxo-1-azabicyclo[3.2.0]hept-2-ene-2-carboxylate:—

UV $\lambda_{max}^{H_2O*}$ nm($\varepsilon$): 303 (10600).
*0.1M phosphate buffer

NMR ($D_2O$) δ ppm: 0.96 (3H, t, J=7Hz, $CH_2CH_3$), 1.60—1.90 (2H, m, $CH_2CH_3$), 2.40—2.90 (2H, m, C—4H), 3.15 (1H, dt, J=8Hz, J=3Hz, C—6H), 3.86 (1H, dt, J=9Hz, J=3Hz, C—5H), 7.35—7.65 (5H, m, ArH).

Example 58

Sodium 3-cyclohexylthio-6-ethyl-7-oxo-1-azabicyclo[3.2.0]hept-2-ene-2-carboxylate:—

UV $\lambda_{max}^{H_2O*}$ nm(ε): 305 (6600).
*0.1M phosphate buffer (pH 7.0)
NMR ($D_2O$) δ ppm: 1.04 (3H, t, J=7.5Hz, $CH_2CH_3$), 1.2—2.2 (12H, m, $CH_2CH_3$, cyclohexyl $CH_2$), 3.0—3.4 (4H, m, C—4H, C—6H, S—CH), 4.02 (1H, dt, J=9Hz, J=3Hz, C—5H).

Example 59

Sodium 3-*n*-butylthio-6-ethyl-7-oxo-1-azabicyclo[3.2.0]hept-2-ene-2-carboxylate:—

UV $\lambda_{max}^{H_2O*}$ nm(ε): 302 (7550)
*0.1M phosphate buffer (pH 7.0)
IR $\nu_{max}^{KBr}$ $cm^{-1}$: 1752 (β-lactam), 1600 (carboxylate).
NMR ($D_2O$) δ ppm: 0.93 (3H, t, —$CH_2CH_3$), 1.04 (3H, t, —$CH_2CH_3$), 1.20—2.00 (6H, m, 2X—$CH_2$), 2.50—3.50 (5H, m, —CH—, 2X—$CH_2$—), 4.02 (1H, dt, J=9.0Hz, J=3.0Hz, C—5H).

The following Referential Examples illustrate the fermentative production of sodium salts of antibiotic PS—3, i.e. 3-(2-acetamidoethylthio)-6-(1-hydroxyethyl)-7-oxo-1-azabicyclo[3.2.0]hept-2-ene-2-carboxylic acid, and antibiotic PS—5, i.e. 3-(2-acetamidoethylthio)-6-ethyl-7-oxo-1-azabicyclo[3.2.0]hept-2-ene-2-carboxylic acid, and antibiotic PS—6, i.e. 3-(2-acetamidoethylthio)-6-isopropyl-7-oxo-1-azabicyclo[3.2.0]hept-2-ene-2-carboxylic acid, the esters of these antibiotics, and the sulfoxides of these esters, which were used as starting materials in Examples 1 to 8 given hereinbefore.

Referential Example 1

Production of antibiotic PS—3, sodium salt:—

A platinum loopful of *Streptomyces* sp. A 271 inoculated in a slant of the following composition (ISP—2) was inoculated in each of 500 ml. Erlenmeyer flasks each containing 100 ml of a culture medium (SE—4) and cultivated at 28°C for 2 days to make a seed culture. The seed culture was added in an amount of 2 ml to 200 Erlenmeyer flasks (500 ml) each containing 100 ml of a culture medium of ABG—1, and cultivated at 28°C for 4 days by a rotary shaker (200 rpm; amplitude 7 cm).

The culture broth in each flask was poured into 5 liters of crushed ice, and 600 g of High Flow Supercell (Johns-Manvile) was added. The cells were separated by filtration to obtain about 20 liters of a filtrate. The subsequent operation on columns was performed at a low temperature of less than 10°C.

The resulting filtrate was passed through a column (7.2 x 80 cm) filled with 3 liters of Diaion PA 306 (a product of Mitsubishi Chemical Co., Ltd.) to cause adsorption of active portions, and then the column was eluted with 20 liters of a 5% (w/v) aqueous solution of sodium chloride. The eluates were passed through a column (4.8 x 10 cm) filled with Diaion HP—20 (a product of Mitsubishi Chemical Co., Ltd.), and adsorbed to a column (6 x 70 cm) filled with 2 liters of Amberlite XAD—4 (a product of Rohm & Haas Co.). The column was eluted with a 30% (w/v) aqueous solution of acetone. The active portions were collected and concentrated and adsorbed to a column (1.5 x 30 cm) of QAE-Sephadex (A—25) (a product of Pharmacia Fine Chemicals, Co.) equilibrated with phosphate buffer (M/199, pH 8.4). The column was eluted with 2 liters of phosphate buffer (M/100, pH 8.4) having a sodium chloride concentration varying gradually from 0 to 0.4M by a concentration gradient method to form 17 g-fractions. Active fractions Nos. 15 to 25 detected by antimicrobial activity against *Comamonas terrigena* were collected, and sodium chloride was added to a concentration of 3% (w/v). Then, these fractions were adsorbed to a column (1.2 x 30 cm) of Diaion HP—20AG (200 to 400 mesh), and the column was eluted with 400 ml of an aqueous solution of acetone having a concentration linearly increasing from 0 to 5% (v/v) by a concentration gradient method. This operation resulted in nearly pure PS—3. The active fractions Nos. 5 to 14 detected in the above manner were collected, and lyophilized to afford 560 mg of the sodium salt of PS—3 as a light yellow dry crude powder.

Composition of a slant medium (ISP—2)

| | | |
|---|---|---|
| Glucose | (0.4% (w/v) | |
| Malt extract | 1.0 | " |
| Yeast extract | 0.4 | " |
| Agar | 1.5 | " |

pH: 7.0 before sterilization

Composition of seed culture (SE—4)

| | |
|---|---|
| Beef extract (Difco Laboratories) | 0.3% (w/v) |
| Bacto-tryptone (Difco Laboratories) | 0.5 „ |
| Glucose | 0.1 „ |
| Soluble starch | 2.4 „ |
| Yeast extract | 0.5 „ |
| Calcium carbonate | 0.4 „ |
| Defatted soybean meal | 0.5 „ |

pH: 7.5 before steriliation

Composition of a production medium (ABG—1)

| | | |
|---|---|---|
| Glycerol | 1.5% | (w/v) |
| Cotton seed residue | 1.5 | „ |
| Sodium chloride | 0.8 | „ |
| L-asparagine | 0.2 | „ |
| $CoCl_2.6H_2O$ | 0.00018 | „ |

pH: 7.4 before sterilization

The resulting powder was dissolved in 1 ml of a phosphate buffer (M/100, pH 8.4), and charged onto a column (1.1 x 90 cm) of Sephadex G—10 (a product of Pharmacia Fine Chemicals, Co.), and developed with the same buffer. The active fractions were collected by measuring their ultraviolet absorptions ($\lambda$300 nm). These fractions were adsorbed to a column (1.1 x 20 cm) of QAE Sephadex A—25 equilibrated with phosphate buffer (M/100, pH 8.4), and the column was eluted with 200 ml of phosphate buffer (M/100, pH 8.4) having a sodium chloride concentration linearly varying from 0 to 0.1M by a concentration gradient method. The ultraviolet absorption ($\lambda$300 nm) of each of the fractions was measured, and the active fractions were collected. Sodium chloride was added to the active fractions so that the concentration of sodium chloride reached 3% (w/v). The mixture was adsorbed to a column (1.1 x 20 cm) of Diaion HP—20AG (200—400 mesh), and eluted with water. The eluates were lyophilized to afford 5 mg of the sodium salt of PS—3 as a white dry powder. The physical and chemical properties of the product were as follows:

1) Color
Colorless

2) Solubility
Readily soluble in water, and substantially insoluble in acetone.

3) Paper chromatography
The antibiotic PS—3 (sodium salt) shows the following Rf values when developed with the following solvents using Toyo Filter Paper No. 50 (a product of Toyo Roshi Kaisha Ltd.) by a descending method.

| | |
|---|---|
| Acetonitrile/tris/EDTA (*1): | Rf=0.19 |
| Ethanol/water (7/3): | Rf=0.59 |

(*1): A mixed solvent consisting of 120 ml of acetonitrile, 30 ml of a 1/10M tris(hydroxymethyl)-aminomethane/HCl buffer (pH 7.5), and 1 ml of a 1/10M aqueous solution of sodium ethylenediaminetetraacetate).

4) Thin-layer chromatography (TLC)

The antibiotic PS—3 (sodium salt) shows the following Rf values when developed with the following solvents by thin-layer chromatography using Chromagram Sheet 13254 Cellulose (No. 6065) (a trademark for a product of Eastman Kodak Co.).

| | |
|---|---|
| n-Butanol/ethanol/water (4/1/5): (upper layer) | Rf=0.30 |
| n-Butanol/isopropanol/water (7/7/6): | Rf=0.42 |
| Acetonitrile/water (8/2): | Rf=0.29 |

5) High voltage paper electrophoresis

The antibiotic PS—3 (sodium salt) shows the following behavior in a buffer solution having the following composition when it is subjected to electrophoresis on Toyo Filter Paper No. 50 (Toyo Roshi Kaisha Ltd.) by using a high voltage paper electrophoresis apparatus (Savant Instrument Inc., High Voltage Power Supply HV 3000 A, Electrophoresis Chamber FP 18A).

The antibiotic is seen to migrate through a distance of at least 5 mm, usually 10 to 40 mm, toward the anode when an electric current is passed for 30 minutes on a potential gradient of 42 V/cm in a buffer (pH 8.6) composed of 3000 ml of water, 3.3 g of barbital and 25.5 g of sodium barbital.

6) Ultraviolet absorption spectrum

The ultraviolet absorption of a solution of 90 micrograms of the antibiotic PS—3 sodium salt in 3 ml of water (pH 7.0) is measured by using a double beam spectrophotometer (Hitachi Model 200—20). The characteristic UV absorption values are as follows:

$\lambda_{min}^{H_2O}$ = about 242.5 nm
$\lambda_{max}^{H_2O}$ = about 298.0 nm

An aqueous solution of hydroxylamine having a pH of 7.5 is added to a solution of the above substance in deionized water so that the concentration of the antibiotic PS—3 to about 20 $\mu$g/ml and the concentration of hydroxylamine to 10 mM. When the mixed solution is allowed to stand at 22°C for 30 minutes, about 93% of its absorbance at 300.0 nm disappears.

7) Infrafred absorption spectrum

Characteristic absorption maxima of the antibiotic PS—3 measured by a KBr method by means of an infrared spectrophotometer (Hitachi Model 260—30) are as follows:

(1) About 1770 $cm^{-1}$ (—CO— of the $\beta$-lactam ring)
(2) About 1640 $cm^{-1}$ (—CO— of amide)
(3) About 1590 $cm^{-1}$ ($—COO^{\ominus}$)
(4) About 1555 $cm^{-1}$ (—CO—NH— of amide)

8) Proton NMR spectrum

The antibiotic PS—3A shows the following characteristic signals in a 100 MHz proton NMR spectrum (internal reference: $d_4$-sodium 3-trimethylsilylpropionate) measured in heavy water ($D_2O$) by using Nippon Denshi JNM PS—100.

(1) A doublet centered at about 1.32 ppm
(J=about 6.5Hz, $\mathit{CH}_3$—CH—)
　　　　　　　　　　　|
(2) A sharp singlet at about 2.00 ppm ($\mathit{CH}_3$—CO—)
(3) A multiplet at about 2.75—3.55 ppm
　　　　　　　　　|
(—$\mathit{CH}_2$—, —CHOH—*CH*—, —S—$\mathit{CH}_2$—$\mathit{CH}_2$—NH—)
(4) A multiplet at about 4.01—4.28 ppm
　　　　　　　|
(—*CH*—, $CH_3$—*CH*OH)
　|
　N

9) Color reactions

Ehrlich reagent reaction: positive
Iodine-chloroplatinic acid reaction: positive
Ninhydrin reaction: negative

Referential Example 2

Production of PS—5 sodium salt and PS—6 sodium salt:—

A seed culture of *Streptomyces* sp. A271 (ATCC 31358) in a flask was prepared. One hundred milliliters of the seed culture was innoculated in two 3-liter jar fermentors containing 15 liters of the SE—4 medium, and cultivated at 28°C for 24 hours with stirring at 200 rpm at an air flow rate of 7.5 liters/minute. Thirty liters of the resulting culture broth was inoculated in a 1-kiloliter stainless steel fermentation tank containing 700 liters of an AGB—42 medium (described hereinbelow), and cultivated at 28°C for 72 hours with stirring at 170 rpm at a flow rate of 300 liters/minute. The supernatant liquid resulting from centrifugation of the culture broth had an antibiotic potency of 1040 CCU/ml.

To the culture broth was added 3% (W/V) of Topco Perlite as a filtration aid (a product of Toko Perlite Kogyo K.K.), and it was filtered by a filter press, and washed with water to obtain 600 liters of a filtrate (total antibiotic activity 5.09 x $10^8$ CCU).

The operations subsequent to this were performed at 6°C.

The filtrate was first passed through a column packed with 10 liters of an ion exchange resin (Diaion PA—306, a product of Mitsubishi Chemical Industries, Ltd.) to decolorize it (the total antibiotic activity in the effluent was 3.70 x $10^8$ CCU; yield 73%).

The effluent was adsorbed to a column packed with 20 liters of Diaion HP—20 (a product of Mitsubishi Chemical Industries, Ltd.), and eluted by a gradient method with 60 liters in total of an aqueous solution of acetone with the concentration of acetone increasing linearly from 0 to 80%. The eluate was fractionated into 2-liter fractions. Each of the fractions was analyzed by descending paper chromatography [developed with a mixed solvent consisting of 20 ml of acetonitrile, 30 ml of a 1/10M tria(hydroxymethyl)aminomethane-HCl buffer having a pH of 7.5 and a 1/10M aqueous solution of sodium ethylenediaminetetraacetate having a pH of 7.5] and bioautography, and two active portions, one consisting of fractions Nos. 4 to 8 and the other consisting of fractions Nos. 9 and 10, were collected. The first portion mainly contained antibiotic PS—5 (the total antibiotic activity 2.54 x $10^8$ CCU; yield 49%), and the second portion was a mixture of the antibiotic PS—6 and antibiotic PS—5 (the total antibiotic activity 3.13 x $10^7$ CCU; yield 6.1%). The content of the antibiotic PS—6 in the mixture was about one-tenth of the total antibiotic activity. The PS—5 sodium salt was separated and purified from the first portion, and PS—6 was separated and purified from the second portion, in the following manner.

(1) Purification of PS—5 sodium salt

The first portion was adsorbed to a column packed with 5 liters of Diaion PA306S (a product of Mitsubishi Chemical Industries, Ltd.) and washed with 2 liters of water. The column was eluted by a gradient method with 20 liters in total of an aqueous solution of sodium chloride with the concentration of sodium chloride increasing linearly from 0 to 3%. The eluate was fractionated into 500 ml fractions. Active fractions Nos. 23 to 45 were collected to obtain 10.5 liters of an active eluate (1.98 x $10^8$ CCU; yield 39%). The active eluate was adsorbed to a column packed with 4 liters of Diaion HP—20, and eluted by a gradient method with 10 liters in total of an aqueous solution of acetone with the concentration of acetone increasing linearly from 0 to 25%. The eluate was fractionated into 100 ml fractions. Active fractions Nos. 59 to 73 were collected to obtain 1.5 liters of an active eluate (1.58 x $10^8$ CCU; yield 31%). The active eluate was treated under reduced pressure to remove acetone, and adsorbed to a column (3.4 cm in diameter and 60 cm in length) packed with 500 ml of QAE-Sephadex (a product of Pharmacia Fire Chemicals Co.). The column was eluted by a gradient method with 5 liters of an aqueous solution of sodium chloride with the concentration of sodium chloride increasing linearly from 0 to 2%, and the eluate was fractionated into 20 ml fractions. Active fractions Nos. 88 to 133 were collected to obtain 900 ml of an active eluate (1.31 x $10^8$ CCU; yield 26%).

The pH of the active eluate was carefully adjusted to 8.3 with 1% NaOH, and then it was adsorbed to a column (3.4 cm in diameter and 65 cm in length) packed with 600 ml of Diaion HP—20AG (a product of Mitsubishi Chemical Industries, Ltd.). The column was eluted by a gradient method with 3 liters of an aqueous solution of acetone with the concentration of acetone increasing linearly from 0 to 10%, and the eluate was fractionated into 20 ml fractions. Active fractions Nos. 56 to 71 were collected to obtain 320 ml of an active eluate (1.19 x $10^8$ CCU; yield 23%). The active eluate was lyophilized to afford 12.4 g of a yellow powder (8400 CCU/mg).

The yellow powder was dissolved in 50 ml of a 0.01M sodium phosphate buffer (pH 8.0), and applied to a column (5.6 cm in diameter and 90 cm in length) packed with 2 liters of Sephadex G—10 (Pharmacia Fine Chemicals Co.). The column was eluted with a 0.01M sodium phosphate buffer (pH 8.0), and the eluate was fractionated into 10 ml fractions. Active fractions Nos. 85 to 121 were collected to obtain 360 ml of an active eluate (9.3 x $10^7$ CCU; yield 18%). The active eluate was adsorbed to a column (3.0 cm x 43 cm) packed with 300 ml of QAE-Sephadex A—25 (Pharmacia Fine Chemicals Co.), and the column was eluted by a gradient method with 2 liters in total of an aqueous solution of sodium chloride with the concentration of sodium chloride increasing linearly from 0 to 1.5%. The eluate was fractionated into 20 ml fractions. Active fractions Nos. 42 to 63 were collected to obtain 440 ml of an active eluate (8.8 x $10^7$ CCU; yield 17%).

The pH of the active eluate was carefully adjusted to 8.3 with 1% NaOH, and it was adsorbed to a column (3.0 cm in diameter and 44 cm in length) packed with 300 ml of Diaion HP—20AG (a product of Mitsubishi Chemical Industries, Ltd.). The column was eluted by a gradient method with 2 liters in total of an aqueous solution of acetone with the concentration of acetone increasing linearly from 0 to 10%, and the eluate was fractionated into 10 ml fractions. Active fractions Nos. 54 to 86 were collected, and lyophilized to afford 3.38 g of colorless powder (25,000 CCU/mg).

(2) Purification of PS—6 sodium salt

The second portion was adsorbed to a column (3.4 x 60 cm) packed with Diaion PA—306 (a product of Mitsubishi Chemical Industries, Ltd.), and eluted by a gradient method with 3 liters in total of an aqueous solution of a sodium chloride with the concentration of sodium chloride increasing linearly from 0 to 4.5%. The eluate was fractionated into 50 g fractions. These fractions were analyzed by bioassay, and active fractions were collected (total antibiotic activity $2.62 \times 10^7$ CCU; yield 5.1%). The active fractions were adsorbed to a column (2.0 cm in diameter and 25 cm in length) packed with Diaion HP—20, and eluted with a 10% aqueous solution of acetone. The eluate was fractionated into 30 ml fractions. Active fractions Nos. 8 to 15 were collected to obtain 240 ml of an active eluate ($1.83 \times 10^7$ CCU; yield 3.6%). The active eluate was concentrated under reduced pressure to remove acetone. The residue was adsorbed to a column (2.4 cm in diameter and 30 cm in length) of QAE-Sephadex (a product of Pharmacia Fine Chemicals Co.) which had been equilibrated with a M/100 phosphate buffer (pH 8.0). The column was eluted by a gradient method with a M/100 phosphate buffer by linearly increasing its sodium chloride concentration from 0 to 0.4M, and the eluate was fractionated into 10 g fractions. Each of the fractions was diluted to 100 times, and analyzed by ultraviolet absorption spectroscopy and bioassay. Thus, active fractions Nos. 25 to 43 were collected to obtain 190 ml of an active eluate ($1.58 \times 10^6$ CCU; yield 3.1%).

To remove the antibiotic PS—5 completely, the active eluate was adsorbed to a column (1.2 cm in diameter and 40 cm in length) packed with Diaion HP—20AG (a product of Mitsubishi Chemical Industries, Ltd.), and eluted by a gradient method with 300 ml in total of an aqueous solution of methanol with the concentration of methanol increasing linearly from 10 to 75%. The eluate was fractionated into 4 g fractions. Each of the fractions was analyzed by descending paper chromatography and bioautography, and two active portions, one consisting of fractions Nos. 26 to 33 containing the antibiotic PS—5 and the other consisting of fractions Nos. 35 to 45 containing the antibiotic PS—6, were collected. The second portion containing the antibiotic PS—6 ($5.8 \times 10^6$ CCU; yield 1.1%) was concentrated to remove methanol, and then lyophilized. To purify the resulting powder further, it was dissolved in 1 ml of a M/100 phosphate buffer (pH 8.0). The solution was applied to a column (5.6 cm in diameter and 80 cm in length) packed with Sephadex G—10 (a product of Pharmacia Fine Chemicals Co.), and eluted with the same phosphate buffer. The eluate was fractionated into 10 g-fractions. Active fractions Nos. 85 to 92 were collected to obtain 80 ml of an active eluate ($5.08 \times 10^5$ CCU; yield 1.1%). The active eluate was adjusted to pH 8.3 with an aqueous sodium hydroxide solution, and adsorbed to a column (2.0 cm in diameter and 30 cm in length) packed with Diaion HP—20 (Mitsubishi Chemical Industries, Ltd.). The column was eluted with a 10% aqueous solution of acetone. The eluate was lyophilized to obtain 205 mg of a white powder.

Formulation of a production medium (AGB—42)

| | | |
|---|---|---|
| Maltose | 5.0% | (W/V) |
| Soluble starch | 1.0 | " |
| Glycerin | 0.3 | " |
| Dry yeast | 3.0 | " |
| Sodium chloride | 0.5 | " |
| Dipotassium phosphate | 0.05 | " |
| Magnesium sulfate ($MgSO_4.7H_2O$) | 0.05 | " |
| Calcium carbonate ($CaCO_3$) | 0.3 | " |
| Cobalt chloride ($CoCl_2.6H_2O$) | 0.00013 | " |

pH: 7.0 before sterilization

Referential Example 3

Production of *p*-nitrobenzyl 3-(2-acetamidoethylthio)-6-(1-hydroxyethyl)-7-oxo-1-azabicyclo-[3.2.0]hept-2-ene-2-carboxylate (PS—3·*p*-nitrobenzyl ester:—

Triethylamine (245 microliters) was added to a solution of 88 mg of PS—3 sodium salt (purity 64%) in 10 ml of dry dimethylformamide, and while the mixture was stirred under ice cooling in a stream of nitrogen, a solution of 346 mg of p-nitrobenzyl bromide in 1 ml of dry dimethylformamide was added. After stirring the mixture at room temperature for 3 hours, the reaction mixture was poured into 30 ml of ethyl acetate, and washed three times with 15 ml of a phosphate buffer (0.1M; pH 6.8). The solvent layer was dried over anhydrous sodium sulfate, and distilled off under reduced pressure at less than 30°C. The residue was washed three times with 5 ml of methylene chloride to afford 54.0 mg of a white powder. The methylene chloride layer was concentrated to 5 ml, then adsorbed to a silica gel column (4 g, 1.0 x 10.5 cm, Kieselgel 60, 70—230 mesh, a product of E. Merck Co.), and then developed with benzene-acetone (1:1) to obtain a fraction with a single fraction. Distillation under reduced pressure afforded 12.2 mg of a white powder.

UV $\lambda_{max}^{THF}$ nm($\varepsilon$): 321 (12800), 270 (10500).

IR $\nu_{max}^{KBr}$ cm$^{-1}$: 3500—3250 (OH, NH), 1770 (CO of $\beta$-lactam), 1700 (CO of ester), 1655 (CO of amide).

NMR ($CDCl_3$) $\delta$ ppm: 1.37 (3H, d, J=6.5Hz, —CHOH—$CH_3$), 1.96 (3H, S, —$COCH_3$), 2.50—3.95 (7H, m, C6—*H*, C4—$H_2$, —S—$CH_2$—$CH_2$—NH—), 3.95—4.50 (2H, m, —*CH*OH—$CH_3$, C5—*H*), 5.21 (1H, d, J=14.0Hz, —*CH*H—Ar), 5.45 (1H, d, J=14.0Hz, —CH*H*—Ar), 5.80 (1H, br. NH), 7.59 (2H, d, J=8.4Hz, —$CH_2$ 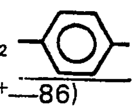 $NO_2$, 8.16 (2H, d, J=8.4Hz, —$CH_2$ 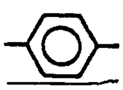 $NO_2$).

Mass(m/e): 363 ($M^+$—86)

Referential Example 4

Production of *p*-nitrobenzyl 3-(2-acetamidoethylthio)-6-ethyl-7-oxo-1-azabicyclo[3.2.0]hept-2-ene-2-carboxylate (PS—5·*p*-nitrobenzyl ester):—

PS—5 sodium salt (275 mg) was dissolved in 20 ml of dry dimethylformamide, and with stirring at room temperature, 197 microliters of triethylamine was added. Ten minutes later, a solution of 179 mg of *p*-nitrobenzyl bromide in 5 ml of dry dimethylformamide was added at 5°C with stirring, and the mixture was stirred at this temperature for 3 hours. After the reaction, the reaction mixture was poured into 150 ml of benzene, and washed three times with 100 ml of a phosphate buffer (0.1M; pH 6.8). The product was dried over anhydrous sodium sulfate, and the solvent was distilled off under reduced pressure. The residue was chromatographed on a column of silica gel (30 g, 1.8 x 30 cm) using a benzene-acetone (3:1) as an eluent. The eluates were distilled to remove the solvent and thereby to obtain yellow crystals. Recrystallization of the yellow crystals from benzene afforded 240 mg of yellow needles having a melting point of 163 to 165°C.

$[\alpha]_D^{21}$ + 70.7° (c 1.00, $CHCl_3$).

UV $\lambda_{max}^{CHCl_3}$ nm($\varepsilon$): 322 (12800), 269 (12000).

IR $\nu_{max}^{CHCl_3}$ cm$^{-1}$: 3460 (*NH*CO), 1780 (CO of $\beta$-lactam), 1710 (CO of ester), 1675 (NH*CO*), 1530 ($NO_2$), 1350 ($NO_2$).

NMR ($CDCl_3$) $\delta$ ppm: 1.04 (3H, t, J=7Hz, $CH_2CH_3$), 1.84 (2H, m, $CH_2CH_3$), 1.96 (3H, s, $NHCOCH_3$), 2.80—3.60 (7H, m, 3X$CH_2$, CH), 3.98 (1H, dt, J=9.0Hz, J=3Hz, C—5H), 5.19 (1H, d, J=14Hz, Ar*CH*·H), 5.49 (1H, d, J=14Hz, ArCH·*H*), 5.95 (1H, br s, *NH*), 7.61 (2H, d, J=9Hz, ArH), 8.18 (2H, d, J=9Hz, ArH).

Mass (m/e): 433 ($M^+$), 363 ($M^+$—EtCH=C=0).

*Anal.* Calcd. for $C_{20}H_{23}O_6N_3S$:
C, 55.43: H, 5.35: N, 9.70
Found: C, 55.29: H, 5.24: N, 9.40

The compounds shown in the following Referential Examples 5 to 9 were obtained by the same method as in Referential Example 4.

Referential Example 5

PS—5·benzyl ester

$[\alpha]_D^{21}$ + 19.97° (c 0.33, $CHCl_3$).

UV $\lambda_{max}^{MeOH}$ nm($\varepsilon$): 318 (11000).

IR $\nu_{max}^{CHCl_3}$ cm$^{-1}$: 3430 (CO*NH*), 1770 (CO of $\beta$-lactam), 1690 (CO of ester), 1665 (*CO*NH).

NMR ($CDCl_3$) $\delta$ ppm: 1.04 (3H, t, J=7Hz, $CH_2CH_3$), 1.82 (2H, m, $CH_2CH_3$), 1.96 (3H, s, $COCH_3$),

2.80—3.60 (7H, m, 3X$CH_2$, CH), 3.94 (1H, dt, J=9.0Hz, J=3Hz, C—5, *H*), 5.21 (1H, d, J=12Hz, Ar*CH*·H), 5.38 (1H, d, J=12Hz, ArCH·*H*), 5.90—6.10 (1H, br s, N*H*), 7.34 (5H, distorted, s, ArH).

Mass (m/e): 388 ($M^+$).

Referential Example 6
PS—5·benzhydryl ester

$[\alpha]_D^{21}$ + 2.55° (C = 0.33, $CHCl_3$).

UV $\lambda_{max}^{MeOH}$ nm(ε): 320 (11200).

IR $\nu_{max}^{CHCl_3}$ $cm^{-1}$: 3450 (—CO*NH*—), 1775 (CO of β-lactam), 1675 (CO of ester).

NMR ($CDCl_3$) δ ppm: 1.06 (3H, t, J=8Hz, $CH_3CH_2$—), 1.94 (3H, s, $CH_3$CONH—), 3.94 (1H, dt, J=9Hz, J=3Hz, C—5H), 5.92 (1H, br s, NH), 6.90 (1H, s, —*CH*$Ar_2$), 7.32 (10H, s, —CH$Ar_2$).

Mass (m/e): 464 ($M^+$), 394 ($M^+$—EtCH=C=O).

Referential Example 7
PS—5·methyl ester

$[\alpha]_D^{21}$ + 72.23° (C = 0.36, $CHCl_3$).

UV $\lambda_{max}^{MeOH}$ nm(ε): 315 (10300).

IR $\nu_{max}^{CHCl_3}$ $cm^{-1}$: 3450 (—CO*NH*—), 1775 (CO of β-lactam), 1675 (CO of ester).

NMR ($CDCl_3$) δ ppm: 1.06 (3H, t, J=8Hz, —CH—$CH_3$), 1.70—2.06 (2H, m, $CH_2$—$CH_3$), 1.99 (3H, s, —NHCO$CH_3$), 2.90—3.56 (7H, m, 3X—$CH_2$—, —CH—), 3.83 (3H, s, —$CO_2CH_3$), 3.96 (1H, dt, J=8Hz, J=2Hz, C—5*H*), 6.20 (1H, br s, —*NH*).

Mass (m/e): 312 ($M^+$), 242 ($M^+$—EtCH=C=O).

Referential Example 8
*p*-Nitro-benzyl 3-(2-acetamidoethylthio)-6-isopropyl-7-oxo-1-azabicyclo[3.2.0]hept-2-ene-2-carboxylate (PS—6·p-nitrobenzyl ester):

UV $\lambda_{max}^{THF}$ nm(ε): 322 (ε=12500), 269 (11800)

IR $\nu_{max}^{CHCl_3}$ $cm^{-1}$: 1780 (CO of β-lactam), 1708 (CO of ester).

NMR ($CDCl_3$) δ ppm: 1.02 (3H, d, J=7.0Hz,

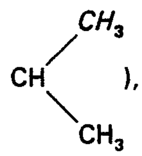), 1.05 (3H, d, J=7.0Hz, 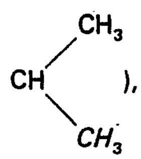), 1.90—2.25 (1H, m, 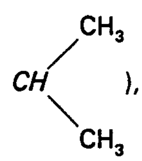),

1.98 (3H, S, $COCH_3$), 2.80—3.60 (7H, m, C—4H, C—6H, 2 x $CH_2$), 4.02 (1H, dt, J=9.0Hz, J=3.0Hz, C—5H), 5.20 (1H, d, J=14.0Hz, Ar*CH*H), 5.48 (1H, d, J=14.0Hz, ArCH*H*), 6.00 (1H, m, NH), 7.62 (2H, d, J=9.0Hz, ArH), 8.18 (2H, d, J=9.0Hz, ArH).

Referential Example 9
PS—6·benzyl ester

UV $\lambda_{max}^{MeOH}$: 318 nm.

IR $\nu_{max}^{CHCl_3}$ $cm^{-1}$: 3400 (N*H*—CO), 1765 (CO of β-lactam), 1665 (NH*CO*).

NMR ($CDCl_3$) δ ppm: 1.07 (6H, t, J=8Hz, —CH·($CH_3$)$_2$), 2.00 (3H, s, —NHCO$CH_3$), 4.05 (1H, dt, C—5H), 5.31 (1H, d, J=12Hz, —*CH*, HAr), 5.47 (1H, d, J=12Hz, —CH, *H*Ar), 6.07 (1H, br s, NH—), 7.46 (5H, s, Ar—H).

Mass (m/e): 402 ($M^+$), 318 ($M^+$—i·Pr·CH=C=O).

Referential Example 10

Production of *p*-nitrobenzyl 3-(2-acetamidoethylsulfinyl)-6-(1-hydroxyethyl)-7-oxo-1-azabicyclo-[3.2.0]hept-2-ene-carboxylate (the sulfoxide of PS—3·p-nitrobenzyl ester):—

PS—3·p-nitrobenzyl ester (10.1 mg) was dissolved in 20 ml of dry tetrahydrofuran, and the solution was cooled to −25°C. With stirring, a solution of 5.2 mg of m-chloroperbenzoic acid in 0.2 ml of dry dichloromethane was added dropwise in a stream of nitrogen. The mixture was stirred at this temperature for 1 hour. The reaction mixture was diluted with 40 ml of ethyl acetate, washed with a phosphate buffer (0.1M; pH 6.8) and then with a saturated aqueous solution of sodium bicarbonate, and dried over anhydrous sodium sulfate. The dried product was adsorbed to a silica gel column (5 g, 1.1 x 15 cm, the same as the silica gel described hereinabove), and eluted with acetone to afford 2.6 mg of the captioned compound.

UV $\lambda_{max}^{THF}$ nm(ε): 320 (sh, 3600), 270 (9500).

IR $\nu_{max}^{CHCl_3}$ $cm^{-1}$: 3450—3250 (—OH, NH), 1785 (CO of β-lactam), 1715 (CO of ester), 1670 (CO of amide).

NMR ($CDCl_3$) δ ppm: 1.41 (3H, d, J=6.0Hz, —CHOH—$CH_3$), 1.97 (3H, s, —CO—$CH_3$), 2.85—3.95 (7H, m, C—6H, C—4$H_2$, S—$CH_2$—$CH_2$NH), 4.00—4.55 (2H, m, C—5*H*, C—7H), 5.25 (1H, d, J=14Hz, C*H*H—Ar), 5.42 (1H, d, J=14Hz, CH*H*—Ar), 6.10—6.50 (1H, br, —NH—), 7.58 (2H, d, J=9.0Hz, —ArH), 8.19 (2H, d, J=9.0Hz, —ArH).

Referential Example 11

Production of the sulfoxide of PS—5·*p*-nitrobenzyl ester:—

PS—5 *p*-nitrobenzyl ester (240 mg) obtained by the method of Referential Example 4 was dissolved in 25 ml of dry methylene chloride, and cooled to −30°C. A solution of 104.9 g of m-chloroperbenzoic acid in 12 ml of dry methylene chloride was added to the resulting solution with stirring and reacted at this temperature for 40 minutes. After the reaction, the reaction mixture was poured into a solution of 0.09 ml of triethylamine in 100 ml of ethyl acetate, which had previously been prepared and cooled with ice. The mixture was stirred for 5 minutes with ice cooling, and 100 ml of ethyl acetate was added. The mixture was transferred to a separating funnel, and washed four times with an ice-cooled phosphate buffer (0.1M; pH 6.8). The solvent layer was dried over anhydrous sodium sulfate, and then benzene was added. The mixture was concentrated under reduced pressure to a volume of 1 ml. The concentrate was passed through a column of 18 g of silica gel (42 ml, 2.0 x 13.4 cm, the same silica gel as described hereinabove) wetted with benzene-acetone (1:1), and developed with about 50 ml of the same solvent. The resulting eluate contained 14.1 mg of the starting material. The eluate was further developed with about 60 ml of benzene-acetone (1:2) and then with 70 ml of benzene-acetone (1:3) to afford 211 mg (89%) of the captioned compound. In a silica gel TLC plate, this compound showed an Rf value of 0.36 with benzene-acetone (1:2).

$[\alpha]_D^{21}$ + 18.0° (c 1.00. $CHCl_3$).

UV $\lambda_{max}^{CHCl_3}$ nm(ε): 267 (13100), 310 (7800).

IR $\nu_{max}^{CHCl_3}$ $cm^{-1}$: 3460 (*NH*CO), 1785 (CO of β-lactam), 1720 (CO of ester), 1680 (NH*CO*).

NMR ($CDCl_3$) δ ppm: 1.08 (3H, t, J=7.0Hz, $CH_2$*$CH_3$*), 1.84 (2H, m, *$CH_2$*$CH_3$), 2.00 (3H, s, NHCO*$CH_3$*), 2.90—3.92 (7H, m, 3X$CH_2$, CH), 3.92—4.23 (1H, m, CH), 5.22 (1H, d, J=14.0Hz, ArC*H*H), 5.48 (1H, d, J=14.0Hz, ArCH*H*), 6.40 (1H, br s, N*H*), 7.53—7.72 (2H, d, J=9.0Hz, Ar*H*), 8.12—8.28 (2H, d, J=9.0Hz, ArH).

Mass (m/e): 449 ($M^+$) (FD Mass).

The compounds shown in Referential Examples 12 to 16 below were obtained in a similar manner to Referential Example 11 using the corresponding compounds obtained in Referential Examples 5 to 9.

Referential Example 12

PS—5·benzyl ester, sulfoxide

$[\alpha]_D^{21}$ − 43.96 (c 1.00, MeOH).

UV $\lambda_{max}^{MeOH}$ nm(ε): 307 (4400).

IR $\nu_{max}^{CHCl_3}$ $cm^{-1}$: 3400 (N*H*CO), 1790 (CO of β-lactam), 1710 (CO of ester), 1670 (NH*CO*).

NMR ($CDCl_3$) δ ppm: 1.04 (3H, t, J=7.0Hz, $CH_2$*$CH_3$*), 1.86 (2H, m, *$CH_2$*$CH_3$), 1.94 (3H, s, CO*$CH_3$*), 2.90—3.84 (7H, m, 3X$CH_2$, C*H*), 4.12 (1H, m, C—5*H*), 5.26 (2H, s, Ar$CH_2$), 6.40 (1H, m, NH), 7.36 (5H, s, Ar*H*).

Mass (m/e): 404 ($M^+$).

Referential Example 13

Sulfoxide of PS—5·benzhydryl ester

$[\alpha]_D^{21}$ − 64.04° (*c* 0.28, $CHCl_3$).

UV $\lambda_{max}^{MeOH}$ nm (ε): 308 (5600).

IR $\nu_{max}^{CHCl_3}$ $cm^{-1}$: 3450 (*NH*CO—), 1790 (CO of β-lactam), 1710 (CO of ester), 1670 (NH*CO*).

NMR ($CDCl_3$) δ ppm: 1.08 (3H, t, J=8Hz, —$CH_2$—*$CH_3$*), 1.92 (3H, s, —NHCO*$CH_3$*), 4.08 (1H, dt, J=9Hz, J=3Hz, C—5H), 6.34 (1H, br, N*H*), 6.87 (1H, s, —*CH*$Ar_2$), 7.32 (10H, s-like, —CH*$Ar_2$*).

Mass (m/e): 480 ($M^+$).

Referential Example 14

Sulfoxide of PS—5·methyl ester

$[\alpha]_D^{21}$ − 74.23° (c 0.25, $CHCl_3$).

UV $\lambda_{max}^{MeOH}$ nm(ε): 303 (5200).

IR $\nu_{max}^{CHCl_3}$ $cm^{-1}$: 3450 *NH*CO), 1790 (CO of β-lactam), 1720 (CO of ester), 1670 (NH*CO*).

NMR ($CDCl_3$) δ ppm: 1.07 (3H, t, J=8Hz, —$CH_2$—*$CH_3$*), 1.70—2.00 (2H, m, —*$CH_2$*—$CH_3$), 2.00

(3H, s, —NHCO$CH_3$), 2.90—3.90 (7H, m, 3X—$CH_2$—, —CH—), 3.89 (3H, s, —$CO_2CH_3$), 4.00—4.20 (1H, m, C—5H), 6.60 (1H, br s, —NH).

Mass (m/e): 328 ($M^+$) (FD Mass).

### Referential Example 15
### Sulfoxide of PS—6·p-nitrobenzyl ester

UV $\lambda_{max}^{CHCl_3}$ nm($\varepsilon$): 267 (13000), 310 (7800)

IR $\nu_{max}^{CHCl_3}$ cm$^{-1}$: 1792 (CO of $\beta$-lactam), 1720 (ester).

NMR ($CDCl_3$) $\delta$ ppm: 1.00 (3H, d, J=7.0Hz,

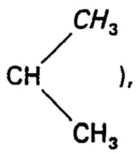 ), 1.03 (3H, d, J=7.0Hz, 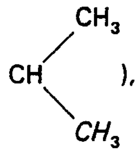 ), 1.90—2.20 (4H, m, 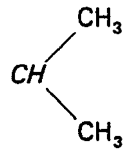

$COCH_3$), 2.90—4.25 (8H, m, C—4H, C—5H, C—6H, 2 x $CH_2$), 5.22 (1H, d, J=14.0Hz, ArC*H*H), 5.44 (1H, d, J=14.0Hz, ArCH*H*), 6.45 (1H, m, NH), 7.56 (2H, d, J=9.0Hz, ArH), 8.18 (2H, d, J=9.0Hz, ArH).

### Referential Example 16
### Sulfoxide of PS—6·benzyl ester

UV $\lambda_{max}^{MeOH}$: 307 nm.

IR $\nu_{max}^{CHCl_3}$ cm$^{-1}$: 3400 (—N*H*CO), 1780 (CO of $\beta$-lactam), 1710 (CO of ester), 1670 (NH*CO*).

NMR ($CDCl_3$) $\delta$ ppm: 1.07 (6H, t, J=7Hz, —CH($CH_3$)$_2$, 1.98 (3H, s, —NHCO$CH_3$), 4.12 (1H, m, C—5H), 5.29 (2H, s, —$CH_2$—Ar), 6.47 (1H, br, —N*H*), 7.37 (5H, s -like, ArH).

Mass (m/e): 418 ($M^+$) (FD Mass).

## Claims

1. A compound of the formula

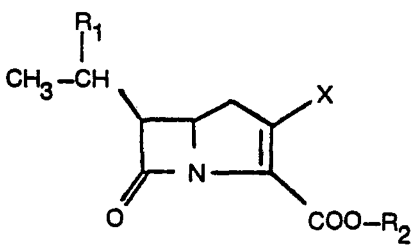

(I)

wherein $R_1$ represents a hydrogen atom, a hydroxyl group or a methyl group, $R_2$ represents a $C_1$—$C_6$ alkyl group or an aralkyl group having 7 to 20 carbon atoms which may be substituted by a halogen atom, a $C_1$—$C_6$ alkyl group, a $C_1$—$C_6$ alkoxy group, a halo $C_1$—$C_6$ alkyl group, a nitro group or a $C_1$—$C_6$ alkylsulfonyl group, and X represents a bromine or iodine atom with the proviso that *o*- and *p*- nitrobenzyl 3-bromo-6-(1-hydroxyethyl)-7-oxo-1-azabicyclo[3.2.0]hept-2-ene-2-carboxylate are excluded.

2. The compound of claim 1 wherein $R_1$ represents a hydrogen atom or a methyl group.

3. The compound of claim 1 wherein $R_2$ represents an alkyl group having 1 to 4 carbon atoms, or a methyl or ethyl group substituted by 1 to 3 phenyl groups which may be substituted by 1 to 3 substituents selected from halogen atoms, $C_1$—$C_6$ alkyl groups, $C_1$—$C_6$ alkoxy groups, $C_1$—$C_6$ haloalkyl groups, nitro groups and $C_1$—$C_6$ alkyl sulfonyl groups.

4. The compound of claim 1 wherein $R_2$ represents an alkyl group having 1 to 4 carbon atoms, a benzyl group, a benzyl group which may be substituted by a halogen atom, a $C_1$—$C_6$ alkyl group, a halo $C_1$—$C_6$ alkyl group, a nitro group or a $C_1$—$C_6$ alkylsulfonyl group, or a benzhydryl group.

5. The compound of claim 1 which is selected from benzyl 3-bromo-6-ethyl-7-oxo-1-azabicyclo[3.2.0]hept-2-ene-2-carboxylate, *p*-nitrobenzyl 3-bromo-6-ethyl-7-oxo-1-azabicyclo[3.2.0]-hept-2-ene-carboxylate, methyl 3-bromo-6-ethyl-7-oxo-1-azabicyclo[3.2.0]hept-2-ene-2-carboxylate, benzyl 3-bromo-6-isopropyl-7-oxo-1-azabicyclo[3.2.0]hept-2-ene-2-carboxylate, methyl 3-iodo-6-ethyl-7-oxo-1-azabicyclo[3.2.0]hept-2-ene-2-carboxylate, benzyl 3-iodo-6-ethyl-7-oxo-1-azabicyclo-[3.2.0]hept-2-ene-2-carboxylate and benzyl 3-iodo-6-(1-hydroxyethyl)-7-oxo-1-azabicyclo[3.2.0]hept-2-ene-2-carboxylate.

6. A process for producing a compound of the formula I′

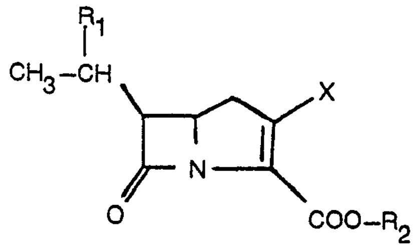

(I′)

wherein $R_1$ represents a hydrogen atom, a hydroxyl group or a methyl group, $R_2$ represents a $C_1$—$C_6$ alkyl group or an aralkyl group having 7 to 20 carbon atoms which may be substituted by a halogen atom, a $C_1$—$C_6$ alkyl group, a $C_1$—$C_6$ alkoxy group, a halo $C_1$—$C_6$ alkyl group, a nitro group or a $C_1$—$C_6$ alkylsulfonyl group, and X represents a bromine or iodine atom, which comprises reacting a compound of the formula II

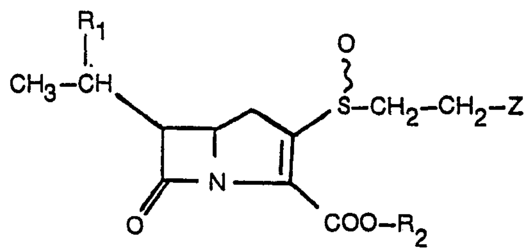

(II)

wherein $R_1$ and $R_2$ are as defined above, and Z represents an amino group or a blocked amino group, with a brominating or iodinating agent selected from N-bromimides, N-bromamides, N-iodimides, N-iodamides and alkali metal iodides.

7. A process for producing a compound of the following formula

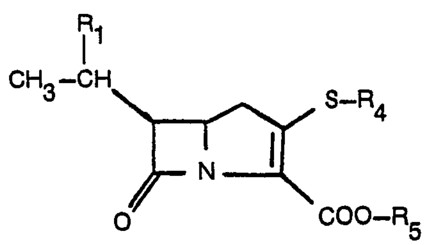

(VI)

wherein $R_1$ represents a hydrogen atom, a hydroxyl group or a methyl group, $R_4$ represents a $C_1$—$C_6$ alkyl group, a hydroxy $C_1$—$C_6$ alkyl group, a $C_1$—$C_6$ alkoxy-$C_1$—$C_6$ alkyl group, a di($C_1$—$C_6$ alkyl) amino-$C_1$—$C_6$ alkyl group, a $C_1$—$C_6$ alkanoyloxy-$C_1$—$C_6$ alkyl group, a phenylacetylamino-$C_1$—$C_6$ alkyl group, a cyclohexyl group, a phenyl group, a *p*-nitrophenyl group, or a 5- or 6-membered aromatic heterocyclic group having 1 or 2 nitrogen atoms, and $R_5$ represents a hydrogen atom or the group $R_2$, or the salts thereof, which comprises reacting a compound of the formula

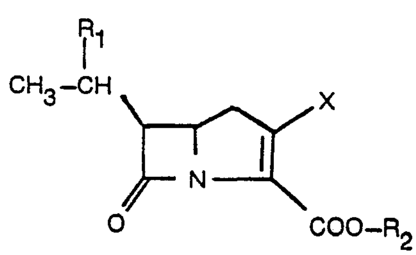

(I)

wherein $R_1$ and $R_2$ are as defined above, and X represents a bromine or iodine atom, with a thiol compound of the formula

$R_4$—SH (V)

wherein $R_4$ is as defined above, or its reactive derivative, and when producing the compound of formula (VI) in which $R_5$ is a hydrogen atom, saponifying or hydrogenolyzing the product, and optionally converting the resulting product into its salt.

**Revendications**

1. Les composés de formulé générale:

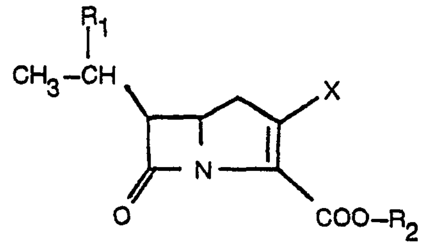

(I)

dans laquelle $R_2$ représente un atome d'hydrogène, un hydroxyle ou un méthyle,$R_2$ un alkyle en $C_1$—$C_6$ ou un aralkyle en $C_7$—$C_{20}$ pouvant être substitué par un atome d'halogène, un alkyle, un alcoxy ou un halogéno-alkyle en $C_1$—$C_6$, un groupe nitro ou un alkyl-sulfonyle en $C_1$—$C_6$, et X représente un atome de brome ou d'iode, à l'exclusion des 3-bromo-6-(1-hydroxy-éthyl)-7-oxo-1-azabicyclo[3.2.0]hept-2-ène-2-carboxylates de *o*- et *p*-nitrobenzyle.

2. Composé selon la revendication 1, dans lequel $R_1$ est un atome d'hydrogène ou un méthyle.

3. Composé selon la revendication 1, dans lequel $R_2$ est un alkyle en $C_1$—$C_4$ ou bien un méthyle ou un éthyle substitué par 1 à 3 phényles pouvant eux-même porter 1 à 3 substituants choisis parmi les halogènes, des alkyles, des alcoxy et des halogéno-alkyles en $C_1$—$C_6$, le groupe nitro et des alkyl-sulfonyles en $C_1$—$C_6$.

4. Composé selon la revendication 1 dans lequel $R_2$ est un alkyle en $C_1$—$C_4$, un benzyle éventuellement substitué par un atome d'halogène, un alkyle ou un halogéno-alkyle en $C_1$—$C_6$, un groupe nitro ou un alkyl-sulfonyle en $C_1$—$C_6$, ou bien un groupe benzhydryle.

5. Composé selon la revendication 1, choisi parmi le 3-bromo-6-éthyl-7-oxo-1-azabicyclo[3.2.0]hept-2-ène-2 carboxylate de benzyle, le 3-bromo-6-éthyl-7-oxo-1-azabicyclo[3.2.0]hept-2-ène-2-carboxylate de *p*-nitrobenzyle, le 3-bromo-6-éthyl-7-oxo-1-azabicyclo[3.2.0]hept-2-ène-2-carboxylate de méthyle, le 3-bromo-6-isopropyl-7-oxo-1-azabicyclo[3.2.0]hept-2-ène-2-carboxylate de benzyle, le 3-iodo-6-éthyl-7-oxo-1-azabicyclo[3.2.0]hept-2-ène-2-carboxylate de méthyle, le 3-iodo-6-éthyl-7-oxo-1-azabicyclo[3.2.0]hept-2-ène-2-carboxylate de benzyle et le 3-iodo-6-(1-hydroxyéthyl)-7-oxo-1-azabicyclo[3.2.0]hept-2-ène-2-carboxylate de benzyle.

6. Procédé de préparation de composés de formule générale I':

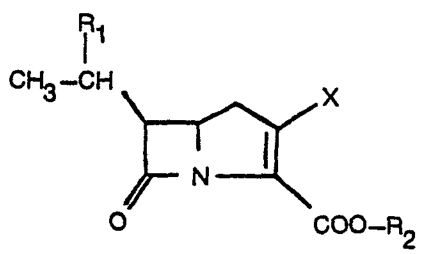

(I')

dans laquelle $R_1$ représente un atome d'hydrogène, un hydroxyle ou un méthyle, $R_2$ un alkyle en $C_1$—$C_6$ ou un aralkyle en $C_7$—$C_{20}$ pouvant être substitué par un atome d'halogène, un alkyle, un alcoxy ou un halogéno-alkyle en $C_1$—$C_6$, un groupe nitro ou un alkyl-sulfonyle en $C_1$—$C_6$, et X représente un atome de brome ou d'iode, procédé selon lequel on fait régair un composé de formule II:

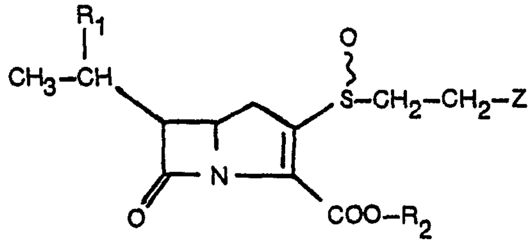

(II)

Z représentant un groupe amino éventuellement bloqué, avec un agent de bromation ou d'iodation choisi parmi des N-bromimides, des N-bromamides, des N-iodimides, des N-iodamides et des iodures de métaux alcalins.

7. Procédé de préparation de composés de formule:

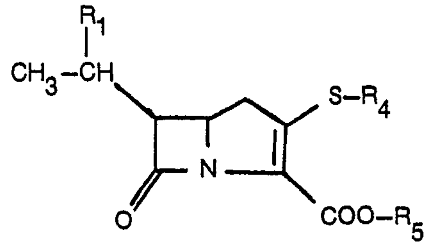

(VI)

dans laquelle $R_1$ représente un atome d'hydrogène, un hydroxyle ou un méthyle, $R_4$ un alkyle ou un hydroxyalkyle en $C_1$—$C_6$, un $C_1$—$C_6$ alcoxy-$C_1$—$C_6$ alkyle, un di ($C_1$—$C_6$ alkyl)amino-$C_1$—$C_6$ alkyle, un $C_1$—$C_6$ alcanoyloxy-$C_1$—$C_6$ alkyle, un phénylacétylamino-$C_1$—$C_6$ alkyle, un cyclohexyle, un phényle, un *p*-nitrophényle ou un radical hétérocyclique aromatique pentagonal ou hexagonal avec un ou deux atomes d'azote, et $R_5$ représente un atome d'hydrogène ou le groupe $R_2$, ainsi que des sels de ces composés, procédé selon lequel on fait réagir un composé de formule:

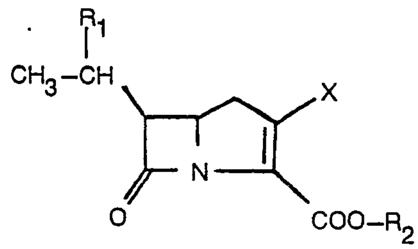

(I)

$R_1$ et $R_2$ ayant les mêmes significations et X représentant un atome de brome ou d'iode avec un thiol de formule:

$$R_4—SH \qquad (V)$$

ou un dérivé réactif de ce thiol, et si l'on veut obtenir un composé de formule (VI) dans lequel $R_5$ est l'hydrogène, on saponifie ou on hydrogènolyse le produit formé, et le cas échéant on transforme le produit résultant en un sel de celui-ci.

**Patentansprüche**

1. Verbindung der Formel

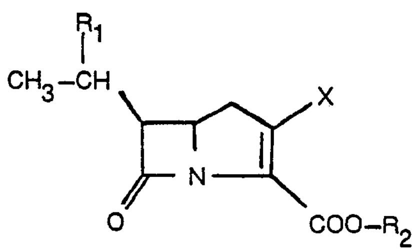

(I)

worin $R_1$ ein Wasserstoffatom, eine Hydroxylgruppe oder eine Methylgruppe bedeutet, $R_2$ eine $C_1$—$C_6$-Alkylgruppe oder eine Aralkylgruppe mit 7 bis 20 Kohlenstoffatomen bedeutet, die durch ein Halogenatom, eine $C_1$—$C_6$-Alkylgruppe, eine $C_1$—$C_6$-Alkoxygruppe, eine Halo-$C_1$—$C_6$-alkylgruppe, eine Nitrogruppe oder eine $C_1$—$C_6$-Alkylsulfonylgruppe substituiert sein kann, und X ein Brom- oder Jodatom bedeutet, mit der Maßgabe, daß o- und p-Nitrobenzyl-3-brom-6-(1-hydroxyäthyl)-7-oxo-1-azabicyclo[3.2.0]hept-2-en-2-carboxylat ausgeschlossen sind.

2. Verbindung nach Anspruch 1, dadurch gekennzeichnet, daß $R_1$ ein Wasserstoffatom oder eine Methylgruppe bedeutet.

3. Verbindung nach Anspruch 1, dadurch gekennzeichnet, daß $R_2$ eine Alkylgruppe mit 1 bis 4 Kohlenstoffatomen oder eine Methyl- oder Äthylgruppe bedeutet, die durch 1 bis 3 Phenylgruppen, welche durch 1 bis 3 Substituenten, ausgewählt unter Halogenatomen, $C_1$—$C_6$-Alkylgruppen, $C_1$—$C_6$-Alkoxygruppen, $C_1$—$C_6$-Haloalkylgruppen, Nitrogruppen und $C_1$—$C_6$-Alkylsulfonylgruppen, substituiert sein können, substituiert ist.

4. Verbindung nach Anspruch 1, dadurch gekennzeichnet, daß $R_2$ eine Alkylgruppe mit 1 bis 4 Kohlenstoffatomen, eine Benzylgruppe, eine Benzylgruppe, die durch ein Halogenatom, eine $C_1$—$C_6$-Alkylgruppe, eine Halo-$C_1$—$C_6$-alkylgruppe, eine Nitrogruppe oder eine $C_1$—$C_6$-Alkylsulfonylgruppe substituiert sein kann, oder eine Benzhydrylgruppe bedeutet.

5. Verbindung nach Anspruch 1, dadurch gekennzeichnt, daß sie ausgewählt wird unter Benzyl-3-brom-6-äthyl-7-oxo-1-azabicyclo[3.2.0]hept-2-en-2-carboxylat, p-Nitrobenzyl-3-brom-6-äthyl-7-oxo-1-azabicyclo[3,2,0]hept-2-en-2-carboxylat, Methyl-3-brom-6-äthyl-7-oxo-1-azabicyclo[3,2,0]hept-2-en-2-carboxylat, Benzyl-3-brom-6-isopropyl-7-oxo-1-azabicyclo[3,2,0]hept-2-en-2-carboxylat, Methyl-3-jod-6-äthyl-7-oxo-1-azabicyclo[3,2,0]hept-2-en-2-carboxylat, Benzyl-3-jod-6-äthyl-7-oxo-1-azabicyclo-[3,2,0]hept-2-en-2-carboxylat und Benzyl-3-jod-6-(1-hydroxyäthyl)-7-oxo-1-azabicyclo[3,2,0]hept-2-en-2-carboxylat.

6. Verfahren zur Herstellung einer Verbindung der Formel I'

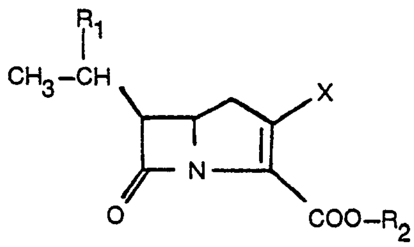

(I')

worin $R_1$ ein Wasserstoffatom, eine Hydroxylgruppe oder eine Methylgruppe bedeutet, $R_2$ eine $C_1$—$C_6$-Alkylgruppe oder eine Aralkylgruppe mit 7 bis 20 Kohlenstoffatomen, die durch ein Halogenatom, eine $C_1$—$C_6$-Alkylgruppe, eine $C_1$—$C_6$-Alkoxygruppe, eine Halo-$C_1$—$C_6$-alkylgruppe, eine Nitrogruppe oder eine $C_1$—$C_6$-Alkylsulfonylgruppe substituiert sein kann, bedeutet und X ein Brom- oder Jodatom bedeuter, dadurch gekennzeichnet, daß man eine Verbindung der Formel II

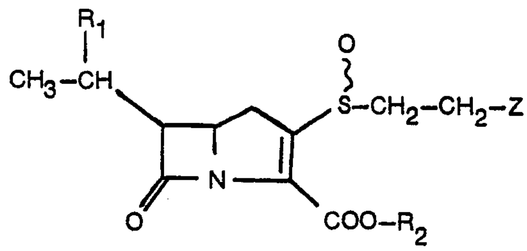

(II)

worin $R_1$ und $R_2$ die oben gegebenen Definitionen besitzen und Z eine Aminogruppe oder eine geschützte Aminogruppe bedeutet, mit einem Bromierungs- oder Jodierungsmittel, ausgewählt unter N-Bromimiden, N-Bromamiden, N-Jodimiden, N-Jodamiden und Alkalimetalljodiden, umsetzt.

7. Verfahren zur Herstellung einer Verbindung der folgenden Formel

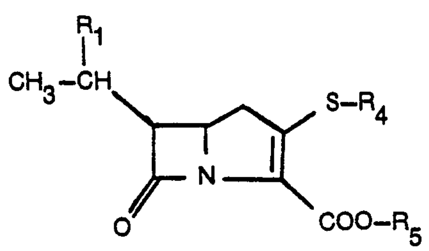

(VI)

worin $R_1$ ein Wasserstoffatom, eine Hydroxylgruppe oder eine Methylgruppe bedeutet, $R_4$ eine $C_1$—$C_6$-Alkylgruppe, eine Hydroxy-$C_1$—$C_6$-alkylgruppe, eine $C_1$—$C_6$-Alkoxy-$C_1$—$C_6$-alkylgruppe, eine Di($C_1$—$C_6$-alkyl)amino-$C_1$—$C_6$-alkylgruppe, eine $C_1$—$C_6$-Alkanoyloxy-$C_1$—$C_6$-alkylgruppe, eine Phenylacetylamino-$C_1$—$C_6$-alkylgruppe, eine Cyclohexylgruppe, eine Phenylgruppe, eine p-Nitrophenylgruppe oder eine 5- oder 6-gliedrige aromatische heterocyclische Gruppe 1 mit oder 2 Stickstoffatomen bedeutet und $R_5$ eine Wasserstoffatom oder die Gruppe $R_2$ bedeutet oder ihre Salze, dadurch gekennzeichnet, daß man eine Verbindung der Formel

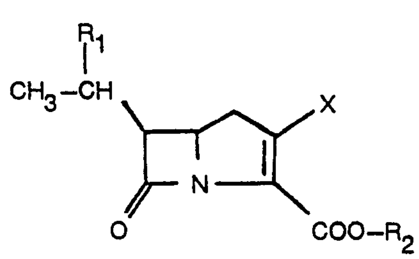

(I)

worin $R_1$ und $R_2$ die oben gegebene Definition besitzen und X ein Brom- oder Jodatom bedeutet, mit einer Thiolverbindung der Formel

$$R_4—SH \quad (V)$$

worin $R_4$ die oben gegebene Definition besitzt, oder ihrem reaktiven Derivat umsetzt und zur Herstellung der Verbindung der Formel (VI), worin $R_5$ ein Wasserstoffatom bedeutet, das Produkt verseift oder einer Hydrogenolyse unterwirft und gegebenenfalls das entstehende Produkt in sein Salz überführt.